Europäisches Patentamt

European Patent Office

Office européen des brevets

(19)

(11) **EP 0 348 348 B1**

(12) **EUROPÄISCHE PATENTSCHRIFT**

(45) Veröffentlichungstag und Bekanntmachung des Hinweises auf die Patenterteilung:
**09.08.2000  Patentblatt 2000/32**

(51) Int. Cl.$^7$: **A01N 65/00**, A01H 5/00, C12N 15/82

(21) Anmeldenummer: **89810447.6**

(22) Anmeldetag: **13.06.1989**

(54) **Verfahren zur Bekämpfung von Pflanzenschädlingen mit nicht-pflanzlichen Proteinase-Inhibitoren**

Process for controlling plant pests with the help of non-plant-derived proteinase-inhibitors

Procédé de lutte contre les parasites des plantes utilisant des inhibiteurs de protéinase non-dérivés de plantes

(84) Benannte Vertragsstaaten:
**AT BE CH DE ES FR GB GR IT LI LU NL SE**

(30) Priorität: **20.06.1988 US 208331**
**07.03.1989 US 320195**

(43) Veröffentlichungstag der Anmeldung:
**27.12.1989  Patentblatt 1989/52**

(73) Patentinhaber:
• **Novartis AG**
  **4058 Basel (CH)**
  Benannte Vertragsstaaten:
  **BE CH DE ES FR GB GR IT LI LU NL SE**
• **Novartis-Erfindungen Verwaltungsgesellschaft m.b.H.**
  **1235 Wien (AT)**
  Benannte Vertragsstaaten:
  **AT**

(72) Erfinder: **Fowler, Elizabeth, Dr.**
**Durham, N.C. 27707 (US)**

(56) Entgegenhaltungen:
EP-A- 0 257 472          EP-A- 0 270 355
EP-A- 0 270 356          EP-A- 0 272 144

• NATURE, Band 330, Nr. 6144, 12.-18. November 1987, Seiten 160-163, London, GB; V.A. HILDER et al.: "A novel mechanism of insect resistance engineered into tobacco"
• THE EMBO JOURNAL, Band 6, Nr. 2, Februar 1987, Seiten 303-306, IRL Press Ltd, Eynsham, Oxford, GB; J.J. SANCHEZ-SERRANO et al.: "Wound-induced expression of a potato proteinase inhibitor II gene in transgenic tobacco plants"
• BIOFUTUR, April 1988, Seiten 21-29; J. RAJNCHAPEL-MESSAI: "La stimulation des défenses des plantes"
• CHEMICAL ABSTRACTS, Band 106, 1987, Seite 378, Zusammenfassung Nr. 47300v, Columbus, Ohio, US; L.L. MURDOCK: "Primary gene products as plant defenses", & ENERGY RES. ABSTR. 1986, 11(4) ABSTR. No. 32794
• FEBS LETTERS, Band 216, Nr. 2, Juni 1987, Seiten 229-233, Elsevier Science Publishers B.V. (Biomedical Division); M. ABRAHAMSON et al.: "Molecular cloning and sequence analysis of cDNA coding for the precursor of the human cysteine proteinase inhibitor cystatin C"
• BIOTECH'89 - PROCEEDINGS OF THE CONFERENCE, London, Mai 1989, Seiten 169-174, Blenheim Online Publications, Pinner, Middx, GB; D. BOULTER et al.: "Genetically engineered insect resistant plants"
• UCLA SYMP. MOL. CELL. BIOL. NEW SER. 1983, Band 12, (PLANT MOLECULAR BIOLOGY), Seiten 223-233, Alan R. Liss, Inc., New York, US; D.E. FOARD et al.: "Engineering of crop plants with resistance to herbivores and pathogens: An approach using primary gene products"

• COMMONWEALTH AGRICULTURAL BUREAU, CAB 891127495; D. BOULTER et al.: "Agbiotech news and information engineering insect resistance into crop plants", & PESTICIDE SCIENCE, Band 26, Nr. 1, 1989, Seiten 103-105

**Beschreibung**

Die vorliegende Erfindung betrifft ein Verfahren zur Bekämpfung von Schädlingen auf Pflanzen. Insbesondere betrifft sie ein Verfahren zur Bekämpfung von Schädlingen auf Pflanzen, indem die Schädlinge nicht-pflanzlichen Proteinase-Inhibitoren ausgesetzt werden, die biologisch von den Pflanzen synthetisiert werden. Die Erfindung betrifft auch Pflanzen, die auf Grund ihres Gehalts an nicht-pflanzlichen Proteinase-Inhibitoren den Befall durch Schädlinge hemmen oder für Schädlinge toxisch sind.

A. Proteinasen und ihre Inhibitoren

Als Proteinase wird gewöhnlicherweise ein Enzym bezeichnet, welches Peptidbindungen hydrolysiert, wodurch Moleküle, die solche Bindungen enthalten, beispielsweise Proteine, zerstört werden. Proteinasen, die die Peptidbindung einer terminalen Aminosäure trennen, werden als "Exoproteinasen" bezeichnet. Enzyme, die eine nicht-terminale Peptidbindung hydrolysieren, werden als "Endoproteinasen" bezeichnet. Im Zusammenhang mit der vorliegenden Erfindung wird der Ausdruck jedoch auch als auf Stoffe anwendbar erachtet, die Peptidbindungen durch andere als Hydrolysemechanismen spalten.

Beide genannten Proteinaseklassen zeigen verschiedene Wirkungsweisen. Exoproteinasen können entweder am N-terminalen oder am C-terminalen Ende des Peptids schneiden. Aminopeptidasen wirken am N-terminalen Ende, Carboxypeptidasen greifen am C-terminalen Ende an.

Endopeptidasen sind im allgemeinen spezifischer in ihren Wirkungen. Durch Konvention werden diese Enzyme in vier Hauptklassen eingeteilt, die die Art der hydrolysierten Bindungen und/oder die Vertiefung, die den Ort der Hydrolyseaktivität umgibt, und/oder das Erfordernis eines charakteristischen Teils in der Proteinase widerspiegeln.

Serin-Endoproteinasen werden durch die Beteiligung einer Serin-Hydroxylgruppe des abzubauenden Proteins in der Hydrolysereaktion charakterisiert. Als Gruppe sind diese Proteinasen wahrscheinlich am besten charakterisiert. Sie kommen jedoch in mikrobiellen und tierischen Geweben offenbar weitaus häufiger vor als in pflanzlichen Geweben.

Thiolproteinasen, die auch als Sulfhydrylproteinasen bezeichnet werden, sind offenbar die häufigste Gruppe von Proteinasen in Pflanzengeweben. Sie sind durch die offensichtliche Beteiligung von Schwefel in irgendeiner Form an der Hydrolysereaktion gekennzeichnet. Eine freie Sulfhydrylgruppe am Cystein ist im aktiven Zentrum einer Reihe dieser Proteinasen identifiziert worden.

Die sauren Proteinasen, die auch als Carboxylproteinasen bekannt sind, sind im Pflanzen- und Tierreich weit verbreitet. Die aktiven Zentren enthalten offenbar zwei Asparaginsäureseitenketten, die sich ein gemeinsames Proton und eine Hydroxylgruppe von einem Tyrosinrest teilen.

Die Metallproteinasen sind im Rahmen der vorliegenden Erfindung generisch so definiert, dass sie solche Proteinasen umfassen, die nicht in eine der obigen drei Gruppen fallen und die wenigstens ein Metallion für ihre hydrolytischen Aktivitäten brauchen. Calcium, Zink und Eisen gehören zu den üblicherweise in solchen Proteasen gefundenen Metallen.

Proteinase-Inhibitoren sind Stoffe, die als Antagonisten wirken, indem sie die Aktivität von Proteinasen unterbinden oder einschränken. Eine grosse Zahl von Klassifizierungsschemata ist entwickelt worden, um die Aktivitäten bestimmter Inhibitoren mit den jeweiligen Zielproteinasen und/oder anderen Inhibitoren zu korrelieren. Die gebräuchlichste Klassifizierung korreliert die Aktivität des Inhibitors mit der der Proteinase. Deshalb sind die vier Hauptklassen von Proteinase-Inhibitoren Serin-Proteinase-Inhibitoren, Thiolproteinase-Inhibitoren, saure Proteinase-Inhibitoren und Metallproteinase-Inhibitoren.

Serin-Proteinase-Inhibitoren kommen natürlicherweise in verschiedenen Gewebearten von Pflanzen vor. Sie sind offenbar gegen eine sehr grosse Anzahl von Serin-Proteinasen, die sowohl von Insekten als auch von Mikroorganismen stammen können, wirksam. Es gibt Anhaltspunkte, dass die Zielproteinase durch die Bildung eines stabilen Komplexes zwischen Proteinase und Inhibitor inaktiviert wird, der unfähig ist, Peptide zu hydrolysieren.

Vom mechanistischen Standpunkt aus werden die Inhibitoren der sauren Proteinasen und Metallproteinasen weniger gut verstanden. Sie kommen überall im Pflanzenreich vor, obwohl sie offenbar weniger allgegenwärtig sind als Serin-Proteinase-Inhibitoren. Es wird angenommen, dass die Inhibitoren der Thiolproteinasen hauptsächlich durch einen Mechanismus wirken, durch den der charakteristische Schwefelrest blockiert wird.

Proteinase-Inhibitoren können auch aufgrund struktureller Merkmale klassifiziert werden. Eine grosse Zahl von Proteinase-Inhibitoren mit niedrigem Molekulargewicht ist bekannt, grösstenteils nichtnatürlichen, synthetischen Ursprungs und als Laborreagens geeignet. Eine Zahl von natürlich vorkommenden Inhibitoren mit niedrigem Molekulargewicht ist charakterisiert worden, die aus bakteriellen und pilzlichen Quellen stammen. Diese Gruppe schliesst Inhibitoren wie die Leupeptine, Antipaine und Pepstatine ein. Viele natürlich vorkommende Proteinase-Inhibitoren sind tatsächlich Proteine, die direkt durch Genexpression in der Zelle gebildet oder in einer Abfolge chemischer, enzymatisch kontrollierter Reaktionen synthetisiert werden, und zwar unter Kontrolle solcher Enzyme, die direkt durch Genexpression in der Zelle hergestellt werden.

B. Proteinase-Inhibitoren in Pflanzen

Pflanzliche Proteinase-Inhibitoren kommen häufig in pflanzlichen Geweben vor, offensichtlich als Teil eines biologischen Kontrollmechanismus, um die Pflanz vor dem Angriff einer Vielzahl von Schädlingen zu schützen, insbesondere vor Insekten oder Mikroorganismen. Es wird angenommen, dass die Pflanze als Antwort auf einen solchen Angriff einen oder mehrere Proteinase-Inhibitoren freisetzt, der die Proteinasen im Körper des Angreifers inaktiviert, wenn er vom Angreifer aufgenommen wird. Solche Inaktivierung stört sehr wahrscheinlich die Stoffwechselprozesse des Angreifers, besonders dessen Verdauungsprozesse, und verlangsamt oder unterbindet so dessen Metabolismus.

Es ist gezeigt worden, dass der Zusatz von Proteinase-Inhibitoren zur Nahrung bestimmter Insekten deren Wachstum hemmt. Gatehouse und Boulter (1983) haben beispielsweise gezeigt, dass der Trypsin-Inhibitor aus der Augenbohne *(Vigna unguiculata)* die Entwicklung der Larve von *Callosobruchus maculatus* (gefleckter Bohnenkäfer) hemmt; die Inhibitoren aus der Sojabohne *(Glycine max)* und aus der Limabohne *(Phaseolus lunatus)* sind jedoch viel weniger wirksam. Murdock et al. (1987) haben gezeigt, dass einige Insekten der Ordnung *Coleoptera* (Käfer) Wachstumsverzögerungen erleiden, wenn sie Inhibitoren der Thiolproteinasen ausgesetzt sind.

Diese Ergebnisse weisen darauf hin, dass natürlich vorkommende Proteinase-Inhibitoren geeignete Mittel darstellen, um Insekten oder mikrobielle Schädlinge auf Pflanzen zu bekämpfen. Der Einsatz von natürlich vorkommenden Inhibitoren im grossen Massstab leidet jedoch an einer Reihe von Nachteilen. Solche Verbindungen sind typischerweise Proteine, und bekanntermassen ist es kostspielig, diese in grossen Mengen zu isolieren (oder auf andere Art herzustellen), zu reinigen und zu formulieren. Da viele von ihnen in der Umwelt schnell abgebaut werden, sind sie nur für kurze Zeit aktiv, was wiederholte Applikationen erforderlich macht. Zusätzlich zum raschen Abbau sind viele Proteinase-Inhibitoren wasserlöslich und nach dem ersten Regensturm oder nach der ersten Bewässerung weggewaschen.

Noch nachteiliger ist möglicherweise die Unspezifität vieler Proteinase-Inhibitoren. Viele Inhibitoren zeigen eine Aktivität gegen sehr unterschiedliche Insekten und Mikroorganismen. Entsprechend kann der Einsatz eines einzelnen Inhibitors gegen einen einzelnen Schädling sehr wohl eine gewaltige, ungünstige Wirkung auf nützliche Insekten und/oder Mikroorganismen haben.

C. In vivo Synthese von Proteinase-Inhibitoren in Pflanzen

Als Folge der Entwicklung auf dem Gebiet der Molekularbiologie ist nun die Kontrolle genetischer Prozesse, die zur biologischen Herstellung von Proteinen in einer grossen Vielfalt biologischer Organismen führen, in vielen Fällen bereits Routine. Von besonderer Bedeutung in der Entwicklung der Gentechnologie bzw. der rekombinanten DNA-Technologie ist die Verpflanzung von Genen aus einem Organismus in einen anderen, der in seinen Eigenschaften ziemlich verschieden vom Ursprungsorganismus ist, um so den Empfängerorganismus mit einem Phänotypus auszustatten, der für ihn nicht charakteristisch ist.

Die Transformation von Pflanzen hat sich langsamer entwickelt als die Transformation anderer eukaryotischer Organismen. Erst in den letzten Jahren sind die Techniken, Pflanzengewebe zu transformieren und ausgewachsene fertile Pflanzen zu regenerieren, zahlreicher und verlässlicher geworden, sodass est nunmehr möglich geworden ist, Pflanzen so zu transformieren, dass sie einzelne Proteine exprimieren. Heute stehen für diesen Zweck ziemlich leistungsfähige Verfahren zur Verfügung.

In Anbetracht der Fortschritte der rekombinanten DNA-Technologie und der Nachteile, die mit dem Gebrauch der Proteinase-Inhibitoren in einer reinen oder in einer im wesentlichen reinen Form verbunden sind, wird es nun möglich, transgene Pflanzen zu entwickeln, die fähig sind, biologisch Proteinase-Inhibitoren zu synthetisieren, die die Pflanzen mit einem neuen bzw. zusätzlichen Verteidigungssystem versorgen, um sich vor dem Angriff durch Insekten und/oder Mikroorganismen selbst zu schützen, sodass von aussen kein Pestizid mehr appliziert werden muss.

Die Nachteile, die oben für die topikale Applikation von Proteinase-Inhibitoren aufgezeigt wurden, werden durch die Schaffung resistenter Pflanzen ganz oder zumindest weitgehend ausgeräumt. Der Inhibitor wird in reiner Form und ausreichender Menge in der Pflanze hergestellt, und braucht weder appliziert noch formuliert zu werden. Abbau in der Umwelt und Verluste sind deshalb minimal, wenn nicht sogar bedeutungslos. Die biologische Synthese eines bestimmten Proteinase-Inhibitors gestattet überdies eine gewisse Kontrolle über Beine Spezifität in der Zielpflanze.

Die Möglichkeit, Pflanzen auf genetischer Ebene mit der Fähigkeit auszustatten, einen fremden Proteinase-Inhibitor zu exprimieren, das heisst, einen Proteinase-Inhibitor, den sie normalerweise nicht exprimieren, ist kürzlich gezeigt worden. EP-A-332 576 ist ein Dokument unter Art. 54(3)EPÜ, in dem unter anderem ein verfahren zur Bekämpfung von Pflanzenschädlingen beschrieben wird, bei dem Eglin C-Mutanten in monokotylen oder dikotylen Pflanzen exprimiert wird. In einer Arbeit von Hilder et al., 1987 wurden Tabakpflanzen mit dem Gen transformiert, das den Trypsin-Inhibitor aus *Vigna unguiculata* (Augenbohne) kodiert, und auf diese Weise wurde dem Tabak Resistenz gegenüber *Heliothis virescens* verliehen.

Es ist ganz klar, dass diese Demonstration einerseits zwar gewisse Schlussfolgerungen zulässt, andererseits jedoch zu bedenken ist, dass Tabak nur ein Modellsystem darstellt, das problemlos transformiert werden kann und deshalb allgemein für Einführungsversuche zur Pflanzentransformation benutzt wird. Die vorliegende Erfindung ist jedoch auf die Transformation einer Vielfalt von Pflanzen mit Genen für eine Vielfalt von Proteinase-Inhibitoren gerichtet, nicht nur zum Zwecke von Modellstudien sondern vielmehr im Hinblick auf eine gewerbliche Anwendbarkeit.

Die Variationsbreite hinsichtlich der Pflanzen und der Inhibitoren im Rahmen der vorliegenden Erfindung ist riesig. Die Transformation von Tabak mit Genen, die Nicht-Trypsin-Proteinase-Inhibitoren kodieren, ist wegen ihres speziellen Modellcharakters von Interesse. Die Transformation einer Vielfalt von Mono- und Dikotyledonen mit weitgestreuter Brauchbarkeit und wesentlichem kommerziellen Nutzen stellt einen bedeutenden Fortschritt dar, dessen Nutzen für das ganze Gebiet der Landwirtschaft von grosser Tragweite ist. So hat die Bereitstellung von Pflanzen mit erhöhter Resistenz gegen Schädlinge signifikante Vorteile nicht nur für die Anbauer sondern auch die Verbraucher dieser Pflanzen, die als Nahrungs-, Futter-, Zier-, Faser-, Energie- und Arzneimittelquelle geeignet sind.

Zusammenfassung der Erfindung

Die vorliegende Erfindung betrifft ein Verfahren zur Bekämpfung von Pflanzenschädlingen, welches im wesentlichen darauf beruht, den Schädling einer pestizid wirksamen Menge eines nicht-pflanzlichen Proteinase-Inhibitors auszusetzen, wobei der Inhibitor infolge einer genetischen Manipulation in der Pflanze selbst biologisch synthetisiert wird, wobei die Pflanze eine Mono- oder Dikotyledone ist. Die Erfindung betrifft auch die transgenen Pflanzen, die Gene enthalten, die nicht-pflanzlichen Proteinase-Inhibitoren kodieren, oder Gene, die Protein-Vorläufer der gewünschten Proteinase-Inhibitoren kodieren, und vorzugsweise solche Pflanzen, die diese Gene auch exprimieren.

Den Schwerpunkt dieser Erfindung bilden transgene Pflanen, die als Phänotyp die Fähigkeit zur in vivo Synthese eines für diese Pflanzen fremden Proteinase-Inhibitors aufweisen, wobei der Inhibitor vorzugsweise in einer zur Bekämpfung eines bestimmten Schädlings ausreichenden Menge entsteht und auf diese Weise den Pflanzen eine neue oder verbesserte Resistenz gegenüber diesem Schädling verliehen wird.

Einen weiteren Gegenstand bildet das Verfahren zur Herstellung von neuen transgenen einkeimblättrigen Pflanzen (Monokotyledonen), die den oben genannten Phänotyp aufweisen.

Ein anderer weiterer Gegenstand ist die Entwicklung neuer transgener zweikeimblättriger Pflanzen (Dikotyledonen) mit besagtem Phänotyp.

Kurzbeschreibung der Abbildungen

Fig. 1: Konstruktion von pRK252/Tn903/BglII.
Fig. 2: Konstruktion von pCIB5.

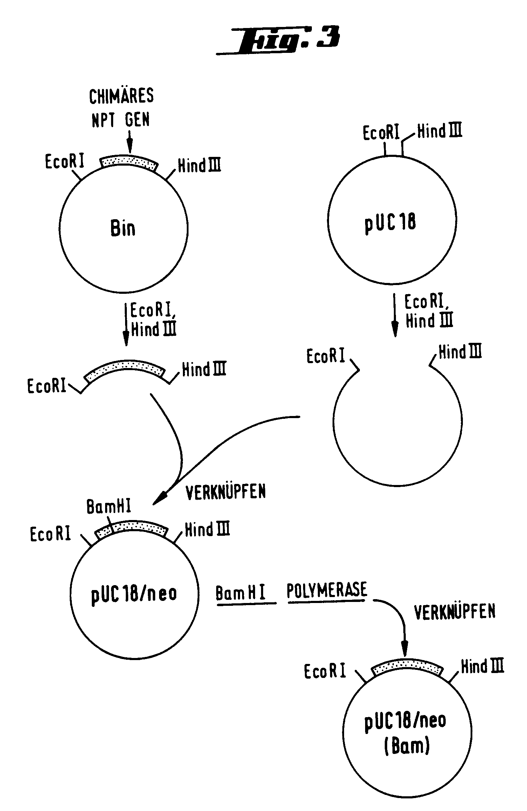

Fig. 3 und 4: Konstruktion von pCIB4.
Fig. 5: Konstruktion von pCIB2.
Fig. 6: Konstruktion von pCIB10, einem Plasmid mit breitem Wirtsspektrum, das die T-DNA-Begrenzungen und ein Gen zur Pflanzenselektion enthält.
Fig. 7: Konstruktion von pCIB710.
Fig. 8: Konstruktion von pCIB10/710.
Fig. 9: Sequenz eines Cystatin-Gens, das gemäss vorliegender Erfindung in eine transgene Pflanze inkorporiert wird
Fig. 10: Synthetische Genfragmente, die vorbereitet und anschliessend zum Cystatin-Gen von Fig. 9 zusammengefügt werden.
Fig. 11: Synthetische Genfragmente, die mit dem Sojabohnen Kunitz Trypsin-Inhibitor-Gen zusammengefügt werden.

Kurzbeschreibung der Tabellen A und B

Tabelle A enthält eine Auflistung von Pflanen, geordnet nach ihren Anwendungen. Sie ist aus Christie (1987) entnommen worden.

In Tabelle B werden unter anderem typische Vertreter transgener Pflanzen aufgelistet, die Gene für Proteinase-Inhibitoren oder für Vorläufer von Proteinase-Inhibitoren enthalten, die gemäss vorliegender Erfindung hergestellt werden, und nennt typische Vertreter von Insekten und anderen Schädlingen, gegen die diese Pflanzen resistent sind bzw. die durch diese Pflanzen unter Kontrolle gehalten werden können. Der Umfang der Erfindung, so wie sie hier beschrieben ist, ist in keiner Weise durch diese beispielhafte Auflistung begrenzt.

## AUSFUEHRLICHE BESCHREIBUNG DER ERFINDUNG

A. Allgemeine Aspekte der Erfindung

Ganz allgemein gesprochen ist die vorliegende Erfindung auf ein Verfahren zur Bekämpfung eines Pflanzenschädlings, der eine bestimmte Zielpflanze angreift, gerichtet, wobei das Verfahren im wesentlichen darauf beruht, diesen Schädling einer pestizid wirksamen Menge eines nicht-pflanzlichen Proteinase-Inhibitors in oder auf einer Pflanze auszusetzen, dadurch gekennzeichnet, dass

(a) besagter Inhibitor biologisch in der Pflanze als Ergebnis der Expression eines fremden Gens, das den Proteinase-Inhibitor kodiert, oder als Ergebnis der Expression eines oder mehrerer fremder Gene, die einen oder mehrere Vorläufer des Proteinase-Inhibitors kodieren, synthetisiert wird; wobei

(b) besagte Pflanze entweder

(i) eine Monokotyledone ist, typmässig ausgewählt aus der Gruppe bestehend aus Getreide, Gemüse- und Knollen-liefernden Pflanzen, Oelpflanzen, Zuckerpflanzen, Futter- und Rasengräsern, Fasern-und Holz-liefernden Pflanzen sowie Gewürz- und Duftpflanzen; oder

(ii) eine Dikotyledone ist, typmässig ausgewählt aus der Gruppe bestehend aus Getreide, Protein-liefernden Pflanzen, Obst-liefernden Pflanzen, Gemüse-und Knollen-liefernden Pflanen, Nüsse-liefernden Pflanzen, Oelpflanzen, Zuckerpflanzen, Futterleguminosen, Fasern- und Holz-liefernden Pflanzen sowie Gewürz- und Duftpflanzen.

Ein zweiter allgemeiner Aspekt der vorliegenden Erfindung richtet sich auf ein Verfahren zur Bekämpfung eines Pflanzenschädlings, der eine bestimmte Zielpflanze angreift, wobei dieses zweite Verfahren dadurch gekennzeichnet ist, den Schädling einer pestizid wirksamen Menge eines nicht-pflanzlichen Proteinase-Inhibitors in oder auf der Pflanze auszusetzen, dadurch gekennzeichnet, dass besagter Inhibitor:

(a) biologisch in der Pflanze als Ergebnis der Expression eines fremden Gens, das den Proteinase-Inhibitor kodiert, oder als Ergebnis der Expression eines oder mehrerer Gene, die einen oder mehrere Vorläufer des Proteinase-Inhibitors kodieren, synthetisiert wird; und

(b) aus der Gruppe von Nicht-Trypsin Proteinase-Inhibitoren, bestehend aus Inhibitoren von Thiolproteinasen, Metallproteinasen, sauren Proteinasen und Serin-Proteinasen auszuwählen ist.

Ein spezieller Aspekt der vorliegenden Erfindung richtet sich auf ein Verfahren zur Bekämpfung eines Pflanzenschädlings, der eine bestimmte Zielpflanze angreift, wobei das Verfahren im wesentlichen darauf beruht, den Schädling einer pestizid wirksamen Menge nicht-pflanzlichen eines Proteinase-Inhibitors in oder auf einer Pflanze auszusetzen, dadurch gekennzeichnet, dass besagter Inhibitor:

(a) biologisch in der Pflanze als Ergebnis der Expression eines fremden Gens, das den Proteinase-Inhibitor kodiert, oder als Ergebnis der Expression eines oder mehrerer fremder Gene, die einen oder mehrere Vorläufer des Proteinase-Inhibitors kodieren, synthetisiert wird; wobei

(b) der Inhibitor aus der Gruppe von Proteinase-Inhibitoren ausgewählt ist, die aus Inhibitoren von Thiolproteinasen, Metallproteinasen, sauren Proteinasen und Nicht-Trypsin-Serin-Proteinasen besteht.

Ein weiterer Aspekt der vorliegenden Erfindung betrifft eine transgene Pflanze, die ein fremdes Gen, welches fähig ist, einen nicht-pflanzlichen Proteinase-Inhibitor zu exprimieren, oder ein fremdes Gen oder eine fremde Gengruppe, die fähig ist, einen oder mehrere Vorläufer eines nicht-pflanzlichen Proteinase-Inhibitors zu exprimieren, enthält, dadurch gekennzeichnet, dass

(a) besagtes Gen oder besagte Gengruppe wenigstens einen kodierenden Abschnitt umfasst, der einen nicht-pflanzlichen Proteinase-Inhibitor oder einen oder mehrere Vorläufer eines nicht-pflanzlichen Proteinase-Inhibitors kodiert, wobei der Proteinase-Inhibitor aus der Gruppe bestehend aus Inhibitoren der Serin-Proteinasen, Thiolproteinasen, Metallproteinasen und sauren Proteinasen ausgewählt ist; wobei

(b) besagte Pflanze entweder

(i) eine Monokotyledone ist, typmässig ausgewählt aus der Gruppe bestehend aus Getreide, Gemüse- und Knollen-liefernden Pflanzen, Oelpflanzen, Zuckerpflanzen, Futter- und Rasengräsern, Fasern-und Holz-liefernden Pflanzen sowie Gewürz- und Duftpflanzen; oder

(ii) eine Dikotyledone ist, typmässig ausgewählt aus der Gruppe bestehend aus Getreide, Protein-liefernden Pflanzen, Obst-liefernden Pflanzen, Gemüse-und Knollen-liefernden Pflanzen, Nüsse-liefernden Pflanzen, Oelpflanzen, Zuckerpflanzen, Futterleguminosen, Fasern- und Holz-liefernden Pflanzen sowie Gewürz- und Duftpflanzen.

Ferner betrifft die vorliegende Erfindung auch eine transgene Pflanze, die ein fremdes Gen, welches fähig ist, einen nicht-pflanzlichen Proteinase-Inhibitor zu exprimieren, oder ein fremdes Gen oder eine fremde Gengruppe, die fähig ist, einen oder mehrere Vorläufer eines nicht-pflanzlichen Proteinase-Inhibitors zu exprimieren, enthält, dadurch gekennzeichnet, dass

(a) besagtes Gen oder besagte Gengruppe wenigstens einen kodierenden Abschnitt umfasst, der einen nicht-pflanzlichen Proteinase-Inhibitor oder einen oder mehrere Vorläufer eines nicht-pflanzlichen Proteinase-Inhibitors kodiert, wobei der Proteinase-Inhibitor aus der Gruppe bestehend aus Inhibitoren der Thiolproteinasen, Metallproteinasen, sauren Proteinasen und Nicht-Trypsin-Serin-Proteinasen ausgewählt ist; wobei
(b) besagte Pflanze entweder

(i) eine Monokotyledone ist, typmässig ausgewählt aus der Gruppe bestehend aus Getreide, Gemüse- und Knollen-liefernden Pflanzen, Oelpflanzen, Zuckerpflanzen, Futter- und Rasengräsern, Fasern- und Holz-liefernden Pflanzen sowie Gewürz- und Duftpflanzen; oder
(ii) eine Dikotyledone ist, typmässig ausgewählt aus der Gruppe bestehend aus Getreide, Protein-liefernden Pflanzen, Obst-liefernden Pflanzen, Gemüse- und Knollen-liefernden Pflanzen, Nüsse-liefernden Pflanzen, Oelpflanzen, Zuckerpflanzen, Futterleguminosen, Fasern- und Holz-liefernden Pflanzen, Drogen enthaltenden Pflanzen sowie Gewürz-und Duftpflanzen.

Ausserdem betrifft die vorliegende Erfindung transgene Pflanzen, die einen nicht-pflanzlichen Proteinase-Inhibitor exprimieren, der für das erwähnte Verfahren zur Bekämpfung eines Schädlings, der eine Zielpflanze angreift, geeignet ist.

### B. Definitionen

Um für ein klares und präzises sprachliches Verständnis der Beschreibung, der Ansprüche und des Schutzumfangs zu sorgen, werden nachfolgende Begriffe im Rahmen dieser Erfindung wie folgt definiert:

Biologische Synthese eines Proteinase-Inhibitors: Synthese eines Proteinase-Inhibitors in einer Wirtszelle. Dieser Ausdruck umfasst: (1) Prozesse, in denen ein aktiver Proteinase-Inhibitor als Protein infolge der Expression eines Gens, das diesen Proteinase-Inhibitor kodiert, produziert wird; (2) Prozesse, in denen ein Translationsprodukt posttranslational durch enzymatische Einwirkung verändert wird; und (3) Prozesse, in denen ein Protein oder mehrere Proteine als Ergebnis der Expression eines Gens oder mehrerer Gene produziert werden, wobei das Protein oder die Proteine an Reaktionen teilhaben, die einen oder mehrere Protein- oder Nichtprotein-Vorläufer in einen aktiven Proteinase-Inhibitor umformen. Dieser letztere Prozess umfasst auch die Fälle, bei denen in der Pflanze exogen oder endogen auftretende Stoffe in Folgereaktionen in aktive Proteinase-Inhibitoren umgewandelt werden.

Biologische Synthese als Ergebnis der Genexpression: Synthese eines Produkts in einer Wirtszelle, das direkt aus der Genexpression resultiert (d.h. als Produkt der Translation), oder Synthese eines Produkts, das indirekt aus der Genexpression (d.h. als Produkt chemischer Reaktionen, die von Translationsprodukten ausgeführt werden) resultiert.

Bekämpfung eines Schädlings: Töten eines Schädlings oder Hemmung seiner Aktivität, sodass der Wirt verschont bleibt.

Fremdes Gen oder fremde Gengruppe: Ein Gen oder eine Gengruppe, die von einem Wirt abstammen, der von dem, der letztlich den gewünschten Genotyp zeigt, verschieden ist; oder ein Gen oder eine Gengruppe, welche vom gleichen Wirt abstammen, mit der Massgabe, dass dieser Wirt in gewisser Hinsicht genetisch modifiziert vorliegt. Veränderung in dieser letzteren Situation schliesst spezifisch, ohne sich darauf zu beschränken, jeden Austausch in der DNA-Sequenz des Gens oder jedes Hinzufügen eines oder mehrerer kodierender Abschnitte, die einen zusätzlichen Phänotyp (beispielsweise einen Antibiotika-Marker) kodieren, ein.

Gen: Eine DNA-Sequenz, die alle Abschnitte enthält, die nötig sind, um die Translation eines Proteins durchzuführen. Die nötigen Abschnitte schliessen wenigstens eine Promotorsequenz, eine kodierende Sequenz und ein Terminationssignal ein. Diese Abschnitte können, aber müssen in keiner Weise von derselben Quelle abstammen; d.h. der Ausdruck "Gen", so wie er hier verwendet wird, umfasst DNA-Abschnitte, die von derselben oder von verschiedenen

Quellen abstammen.

<u>Nicht-Trypsin-Proteinase:</u> Eine Proteinase, deren Wirkungsweise sich wesentlich von der des Trypsins gemäss vereinbarter Klassifizierungsschemata unterscheidet oder die der Fachman als unterschiedlich ansieht. Veranschaulichende, nicht-limitierende Beispiele für diesen Ausdruck sind Proteinasen, die zwei oder mehrere Wirkungsweisen haben, von denen sich die bedeutendste wesentlich von der eines Trypsins unterscheidet, obwohl eine weniger bedeutende oder die am wenigsten bedeutende wie die eines Trypsins sein kann. Ein erfindungsgemässer Nicht-Trypsin-Proteinase-Inhibitor stört wenigstens die primäre Wirkungsweise, ungeachtet irgendeiner Störung der Trypsin-Wirkungsweise.

<u>Pestizid wirksame Menge:</u> Eine Menge, die ausreicht, einen Schädling zu bekämpfen.

<u>Pflanze:</u> Eine Pflanze im herkömmlichen Sinne, aber unter Einschluss von Pflanzengewebe, gleichgültig ob in einer Pflanze oder als gezüchtetes Gewebe, beispielsweise als Teil von Gewebekulturen in Nährmedien.

<u>Vorläufer eines Proteinase-Inhibitors:</u> Ein Stoff, gegebenenfalls aus Protein, der enzymatisch in der Wirtszelle in einen aktiven Proteinase-Inhibitor umgewandelt wird, oder der (entweder als Reaktionspartner oder als Enzym) an der Synthese eines Proteinase-Inhibitors teilnimmt. Dieser Ausdruck umfasst beispielsweise einzelne Verbindungen oder Gruppen von Verbindungen, die endogen oder exogen für eine Wirtszelle auftreten. Der Ausdruck umfasst darüberhinaus Enzyme, die an der Synthese eines Proteinase-Inhibitors aus zellulären Substraten teilhaben; in diesem Fall werden sowohl das Substrat als auch das Enzym als Proteinase-Inhibitor betrachtet.

<u>Proteinase:</u> Ein Stoff, entweder eine einzelne Verbindung oder eine Gruppe von gemeinsam wirkenden Verbindungen, der ein Protein inaktiviert. Obwohl der Ausdruck "Proteinase" hier austauschbar mit "Protease" ist, soll hier und im folgenden vorzugsweise der Ausdruck "Proteinase" gebraucht werden.

<u>Proteinase-Inhibitor:</u> Ein Stoff, entweder eine einzelne Verbindung oder eine Gruppe von gemeinsam wirkenden Verbindungen, der fähig ist, ein Protein zu inaktivieren.

<u>Transgene Pflanze:</u> Eine Pflanze, die wenigstens eine DNA-Sequenz enthält, welche ein Gen sein kann und sich von der entsprechenden DNA-Sequenz in der analogen Wildtyppflanze unterscheidet. Hier und im folgenden umfasst der Ausdruck jede Pflanze, in der irgendeine DNA relativ zur entsprechenden DNA der Wildtyppflanze verändert ist. Die veränderte DNA muss jedoch nicht zwangsweise der Wildtyppflanze einen neuen Phänotyp verleihen. Transgene Pflanzen in diesem Sinne umfassen demnach auch Pflanzen mit einer gegenüber dem Wildtyp erhöhten Zahl von Genkopien und Pflanzen mit modifizierten DNA-Sequenzen, die nicht notwendigerweise auf solche beschränkt sind, die Proteine kodieren.

<u>Funktionelle Aehnlichkeit von Inhibitoren:</u> Funktionell ähnlich sind Inhibitoren, wenn sie ohne signifikante Wirkungsverschlechterung gegeneinander ausgetauscht werden können, d.h. wenn sie eine gleichartige Wirkung aufweisen und ihre Wirkungshöhe in der gleichen Grössenordnung liegt.

<u>C. Transgene Pflanzen und Verfahren zur Schädlingsbekämpfung</u>

<u>(1) Transgene Pflanzen, die Proteinase-Inhibitoren aller vier Klassen enthalten, und die Verwendung dieser Inhibitoren</u>

Die vorliegende Erfindung bietet die Möglichkeit, eine Anzahl von transgenen ein- und zweikeimblättrigen Pflanzen vor Pflanzenschädlingen zu schützen. Dieser Schutz basiert auf nicht-pflanzlichen Proteinase-Inhibitoren, die repräsentativ für alle vier üblichen Klassen von solchen Inhibitoren sind und welche biologisch in diesen Pflanzen synthetisiert werden. Zu den Schädlingen, die auf diese Art bekämpft werden können, gehören vor allem Insekten, Milben, Pilze und Bakterien.

Zielpflanzen, die im Rahmen vorliegender Erfindung besonders interessant sind, sind insbesondere (a) Monokotyledonen, typmässig ausgewählt aus der Gruppe der Zierpflanzen und jener Pflanzen, die in Tabelle A als Getreide, Gemüse- und Knollen-liefernde Pflanzen, Oelpflanzen, Zuckerpflanzen, Futter- und Rasengräser, Fasern- und Holz-liefernde Pflanzen sowie Gewürz- und Duftpflanzen aufgelistet sind; und (b) Dikotyledonen, typmässig ausgewählt aus der Gruppe der Zierpflanzen und jener Pflanzen, die in Tabelle A als Getreide, Protein-liefernde Pflanzen, Obst-liefernde Pflanzen, Gemüse- und Knollen-liefernde Pflanzen, Nüsse-liefernde Pflanzen, Oelpflanzen, Zuckerpflanzen, Futterleguminosen, Fasern- und Holz-liefernde Pflanzen sowie Gewürz- und Duftpflanzen aufgelistet sind. Demgegenüber sind Dikotyledonen, die Drogen enthalten, unter diesem Aspekt der Erfindung nicht eingeschlossen.

Bevorzugte einkeimblättrige Pflanzentypen schliessen Getreide, Gemüse-und Knollen-liefernde Pflanzen, Zuckerpflanzen sowie Futter- und Rasengräser ein. Besonders bevorzugt sind Pflanzen der Gattungen *Avena* (Hafer), *Hordeum* (Gerste), *Oryza* (Reis), *Sorghum* (Hirse), *Triticum* (Weizen), *Dactylis* (Knäuelgras) und *Saccharum* (Zuckerrohr), sowie *Zea mays* (Mais).

Insbesondere bevorzugt sind Pflanzen der Gattung *Dactylis* und *Zea mays*, besonders *Zea mays*.

Bevorzugte zweikeimblättrige Pflanzentypen schliessen Obst-liefernde Pflanzen, Gemüse- und Knollen-liefernde Pflanzen, Oelpflanzen, Zuckerpflanzen, Futterleguminosen sowie Fasern- und Holz-liefernde Pflanzen ein. Besonders bevorzugt sind Pflanzen der Gattungen *Lycopersicon* (Tomate), *Solanum* (Kartoffel), *Pisum* (Erbse), *Beta* (Rübe), *Glycine* (Sojabohne), *Brassica* (Raps und Kohl) und *Gossypium* (Baumwolle).

Am bevorzugtesten sind Pflanzen der Gattungen *Lycopersicon, Solanum* und *Gossypium*. Eine andere Gruppe bevorzugter Pflanzen besteht aus Kartoffeln, Raps, Tomaten, Sojabohnen, Erbsen und Baumwolle.

Eine Hemmung kann beispielsweise bei transgenen Pflanzen beobachtet werden, die mindestens ein fremdes Gen oder eine fremde Gengruppe enthalten, das/die einen Inhibitor einer Serin-Proteinase kodiert. Bevorzugt sind Inhibitoren wenigstens einer Serin-Proteinase ausgewählt aus der Gruppe bestehend aus Thrombin, Plasmin, Elastase, Kallikrein, Subtilisin, Cathepsin G, Chymase, Acrosin, Plasminogen-Aktivator, Cl̄-Esterase, Enterokinase, Tryptase, Post-Prolin-schneidendem Enzym (Prolyl-Endoproteinase), ATP-abhängiger Protease, Thermitase, Mastzellen-Proteinase I und II, *Streptomyces griseus*-Proteinase A, *Staphylococcus aureus* V8-Proteinase, *Tenebrio* α-Proteinase, Urokinase, Blutgerinnungsfaktoren, Komplement-aktivierenden Faktoren und den Serin-Carboxypeptidasen sowie einer Proteinase, die eine wesentliche strukturelle und funktionelle Aehnlichkeit mit einer der vorgenannten hat.

Ausserdem kann der Inhibitor ein Trypsin- oder ein Chymotrypsin-Inhibitor sein.

Die Hemmung äussert sich auch als Ergebnis der in vivo Synthese von anderen Inhibitoren von Serin-Proteinasen, wenn einer oder mehrere solcher Inhibitoren ein Vertreter der Bowman-Birk Inhibitor-Familie, der Sojabohnen-Kunitz Inhibitor-Familie, der Rinderpankreas-Trypsin (Kunitz) Inhibitor-Familie, der Kazal-Trypsin Inhibitor-Familie, der *Streptomyces*-Subtilisin Inhibitor-Familie, der Kartoffel-Inhibitor I-Familie, der Kartoffel-Inhibitor II-Familie, der $\alpha_1$-Proteinase Inhibitor-Familie, der Hirudin-Familie, der Bdellin-Familie, der Inter-$\alpha_1$-Trypsin Inhibitor-Familie, der Serpin-Ueberfamilie, der Cl̄-Inhibitor-Familie, der *Ascaris* Inhibitor-Familie, der Leupeptine, der Antipaine, Elastinal und Chymostatin ist. Eingeschlossen sind Inhibitoren, die eine wesentliche strukturelle und funktionelle Aehnlichkeit mit einem der vorgenannten Inhibitoren haben.

Ein Besonders bevorzugten Serin-Proteinase-Inhibitore ist, $\alpha_1$-Antitrypsin (ein Beispiel für die $\alpha_1$-Proteinase-Inhibitor-Familie).

Eine bevorzugte Gruppe von Inhibitoren, die biologisch in einer transgenen Pflanze synthetisiert werden und bei der Schädlingsbekämpfung, besonders der Bekämpfung von Insekten, nützlich sind, besteht aus den Thiolproteinase-Inhibitoren. Bevorzugte Beispiele hierfür sind die Inhibitoren von Papain, Bromelain, Ficin, Calpain, Cathepsin B, Cathepsin C, Cathepsin L, Cathepsin H, Cathepsin S, Chymopapain, Clostripain, Asclepain, Prolyl-Endopeptidase, Pyroglutamyl-Peptidase, Dipeptyl-Proteinase I, Hefe-Proteinase B, *Streptococcus* Proteinase, *Staphylococcus* Thiolproteinase und Actinidin sowie von Proteinasen, die eine wesentliche strukturelle und funktionelle Aehnlichkeit mit einer der vorgenannten aufweisen.

Besonders bevorzugte Inhibitoren sind: Cystatin, Calpastatin, Bromelain-Inhibitor, Antipain, Leupeptin, Chymostatin und E64 oder ein Abkömmling davon sowie Inhibitoren, die eine wesentliche strukturelle und funktionelle Aehnlichkeit mit den zuvor genannten aufweisen. E64 ist der Trivialname für [N-(L-3-Transcarboxyoxiran-2-carbonyl)-L-leucyl]-amido(4-guanido)-butan. Bei Abkömmlingen von E64 ist im allgemeinen die Gruppe -NH-(CH$_2$)$_4$-NH-C(=NH)-NH$_2$ durch verschiedene Alkylgruppen oder durch Gruppen wie -NH-(CH$_2$)$_4$-NH$_2$, -NH-(CH$_2$)$_2$-CH$_3$, -NH-(CH$_2$)$_7$-NH$_2$ oder -O$_2$CCH=CH-CO-NHCH(i-propyl)-CO-NH-(CH$_2$)$_2$-CH-(CH$_3$)$_2$ ersetzt. Zu den bevorzugten Inhibitoren vom Cystatintyp gehören beispielsweise solche aus der Gruppe: Hühnereiweiss-Cystatin, Human-Cystatin A, Human-Cystatin B, Human-Cystatin C, Human-Cystatin S, Ratten-Cystatin α, Ratten-Cystatin β und Kininogen, beispielsweise L-Kininogen und H-Kininogen.

Ein besonders bevorzugter Thiolproteinase-Inhibitor ist Hühnereiweiss-Cystatin.

Darüberhinaus können transgene Pflanzen eine Resistenz gegen Pflanzenschädlinge aufweisen, wenn sie durch in vivo Synthese einen Metallproteinase-Inhibitor produzieren. Zu den bevorzugten Inhibitoren dieses Typs gehören vor allem jene, die Carboxypeptidase A, Carboxypeptidase B, Aminopeptidase, Kollagenase, Calcium-abhängige neutrale Proteinase, Thermolysin, Angiotensin-umbauendes Enzym, renale Dipeptidase, Enkephalinase, Gelatinase und Keratinase hemmen, oder Inhibitoren von Proteinasen, die eine wesentliche strukturelle und funktionelle Aehnlichkeit mit den vorgenannten Proteinasen aufweisen.

Besonders bevorzugte Inhibitoren sind: Säugetier Kollagenase-Inhibitor, $\alpha_2$-Makroglobulin, Gewebe-Bradykinin-verstärkendes Peptid, Phosphoramidon, Bestatin und Amastatin sowie Inhibitoren, die eine wesentliche strukturelle und funktionelle Aehnlichkeit mit den vorgenannten Inhibitoren aufweisen.

Schliesslich wird eine Hemmung auch dann beobachtet, wenn der Inhibitor eine saure Proteinase hemmt. Bevorzugte Inhibitoren dieses Typs hemmen Pepsin, Renin, Cathepsin D, Chymosin, Penicillinopepsin und *Scytalidium* saure Protease B sowie Inhibitoren von Proteinase, die wesentliche strukturelle und funktionelle Aehnlichkeit mit einer der vorgenannten aufweisen.

Besonders bevorzugt im Rahmen dieser Gruppe sind: Pepstatin, *Ascaris* Carboxylproteinase-Inhibitor, *Bauhinia* Pepsin Inhibitor, *Scopolia japonica* Proteinase-Inhibitor sowie ein Inhibitor, der eine wesentliche strukturelle und funktionelle Aehnlichkeit mit den vorgenannten Inhibitoren aufweist.

Zu den typischen Pflanzenschädlingen gehören Insekten, Milben, Pilze und Bakterien; dabei sind Insekten von besonderer Bedeutung. Tabelle B gibt einige bevorzugte Repräsentanten von Zielpflanzen an. Sie sind genetisch manipuliert, produzieren die angegebenen Proteinase-Inhibitoren und sind so in der Lage, die angegebenen Zielinsekten zu bekämpfen. Diese beispielhafte Auflistung soll auf keinen Fall limitierend für die Erfindung sein.

Primäre Zielschädlinge von Monokotyledonen, beispielsweise Mais, sind Vertreter der Ordnungen *Coleoptera* (Käfer) und *Lepidoptera* (Schmetterlinge), insbesondere der Gattungen *Diabrotica, Diatraea, Ostrinia* und *Heliothis*. Primäre Zielschädlinge von Dikotyledonen, beispielsweise Kartoffeln, Raps, Tomaten, Sojabohnen, Erbsen oder Baumwolle, sind ebenfalls Vertreter der Ordnungen *Coleoptera* und *Lepidoptera*, insbesonders der Gattungen *Diabrotica, Diatraea, Ostrinia, Heliothis, Spodoptera* und *Anthonomus*.

Die Proteinase-Inhibitoren können in jedem Teil der Pflanze exprimiert werden, beispielsweise in den Wurzeln, Stengeln, Blättern, Samen oder Pollen der Pflanze. Vorzugsweise wird der Proteinase-Inhibitor in dem Teil der Pflanze exprimiert, der der primäre Angriffspukt des zu bekämpfenden Schädlings ist.

<u>(2) Transgene Pflanzen, die Nicht-Trypsin-Proteinase-Inhibitoren enthalten, und die Verwendung dieser Inhibitoren</u>

Die vorliegende Erfindung stellt auch Mittel bereit, transgene ein- und zweikeimblättrige Zielpflanzen vor Pflanzenschädlingen zu schützen, bzw. die Schädlinge mittels transgener Pflanzen zu bekämpfen. Dieser Schutz basiert auf Proteinase-Inhibitoren, die repräsentativ für alle vier üblichen Klassen sind und die biologisch in diesen Pflanzen synthetisiert werden. Insbesondere basiert dieser Schutz auf nicht-pflanzlichen Inhibitoren von Nicht-Trypsin-Proteinasen aller vier Klassen, vorzugsweise auf Inhibitoren von Nicht-Trypsin-Serin-Proteinasen. Schädlinge, die auf diese Art bekämpft werden können, sind vorherrschend Insekten, Milben, Pilze und Bakterien.

Pflanzen von besonderem Interesse unter diesem Aspekt der vorliegenden Erfindung schliessen, wie bereits erwähnt, Mono- und Dikotyledonen aus Tabelle A ein und sind typmässig ausgewählt aus der Gruppe bestehend aus Getreide, Protein-liefernden Pflanzen, Obst-liefernden Pflanzen, Gemüse- und Knollen-liefernden Pflanzen, Nüsse-liefernden Pflanzen, Oelpflanzen, Zuckerpflanzen, Futter- und Rasengräsern, Futterleguminosen, Fasern- und Holz-liefernden Pflanzen, sowie Gewürz- und Duftpflanzen und darüber hinaus Drogen-enthaltenden Pflanzen.

Bevorzugte einkeimblättrige Pflanzentypen schliessen Getreide, Gemüse-und Knollen-liefernde Pflanzen, Zuckerpflanzen sowie Futter- und Rasen-gräser ein. Besonders bevorzugt sind Pflanzen der Gattungen *Avena* (Hafer), *Hordeum* (Gerste), *Oryza* (Reis), *Sorghum* (Hirse), *Triticum* (Weizen), *Dactylis* (Knäuelgras) und *Saccharum* (Zuckerrohr), sowie *Zea mays* (Mais). Am bevorzugtesten sind Pflanzen der Gattung *Dactylis* und *Zea mays*.

Bevorzugte zweikeimblättrige Pflanzentypen schliessen Obst-liefernde Pflanzen, Gemüse- und Knollen-liefernde Pflanzen, Oelpflanzen, Zuckerpflanzen, Futterleguminosen sowie Fasern- und Holz-liefernde Pflanzen und Drogen-enthaltende Pflanzen ein. Besonders bevorzugt sind Pflanzen der Gattungen *Lycopersicon* (Tomate), *Solanum* (Kartoffel), *Pisum* (Erbse), *Beta* (Rübe), *Glycine* (Sojabohne), *Brassica* (Raps und Kohl), *Gossypium* (Baumwolle) und *Nicotiana*(Tabak). Am bevorzugtesten sind Pflanzen der Gattungen *Lycopersicon, Solanum, Gossypium* und *Nicotiana*.

Eine Hemmung kann beispielsweise bei transgenen Pflanzen beobachtet werden, die mindestens ein fremdes Gen oder eine fremde Gengruppe enthalten, das/die einen Inhibitor einer Nicht-Trypsin Serin-Proteinase kodiert. Bevorzugt sind Inhibitoren wenigstens einer Nicht-Trypsin Serin-Proteinase, ausgewählt aus der Gruppe bestehend aus Thrombin, Plasmin, Elastase, Kallikrein, Subtilisin, Cathepsin G, Chymase, Acrosin, Plasminogen-Aktivator, $C\bar{I}$-Esterase, Enterokinase, Tryptase, Post-Prolin-schneidendem Enzym (Prolyl-Endoproteinase), ATP-abhängiger Protease, Thermitase, Mastzellen-Proteinase I und II, *Streptomyces griseus*-Proteinase A, *Staphylococcus aureus* V8-Proteinase, *Tenebrio* α-Proteinase, Urokinase, Blutgerinnungsfaktoren, Komplement-aktivierenden Faktoren und den Serin-Carboxypeptidasen sowie einer Proteinase, die eine wesentliche strukturelle und funktionelle Aehnlichkeit mit einer der vorgenannten hat. Ausserdem kann der Inhibitor ein Chymotrypsin-Inhibitor sein.

Die Hemmung äussert sich auch als Ergebnis der in vivo Synthese von anderen Inhibitoren von Nicht-Trypsin Serin-Proteinasen, wenn einer oder mehrere solcher Inhibitoren ein Vertreter der Bowman-Birk Inhibitor-Familie, der *Streptomyces*-Subtilisin Inhibitor-Familie, der Kartoffel-Inhibitor I-Familie, der Kartoffel-Inhibitor II-Familie, der $\alpha_1$-Proteinase Inhibitor-Familie, der Hirudin-Familie, der Bdellin-Familie, der Eglin-Familie, der Inter-$\alpha_1$-Trypsin Inhibitor-Familie, der Serpin-Ueberfamilie, der $C\bar{I}$-Inhibitor-Familie, der *Ascaris* Inhibitor-Familie, der Leupeptine, der Antipaine, Elastinal oder Chymostatin ist. Eingeschlossen sind Inhibitoren, die eine wesentliche strukturelle und funktionelle Aehnlichkeit mit einem der vorgenannten Inhibitoren haben.

Eine unter diesem speziellen Aspekt der Erfindung bevorzugte Gruppe von Inhibitoren, die biologisch in einer transgenen Pflanze synthetisiert werden und nützlich bei der Schädlingsbekämpfung, besonders von Insekten, sind, enthält wie bereits erwähnt, einen Inhibitor einer Thiolproteinase. Bevorzugte und besonders bevorzugte Beispiele solcher Inhibitoren sind in den vorangehenden Abschnitten aufgelistet und sind auch unter diesem Gesichtspunkt der Erfindung bevorzugt.

Ausserdem können transgene Pflanzen eine Resistenz gegen Schädlinge aufweisen, wenn durch die in vivo Synthese ein Inhibitor einer Metallproteinase produziert wird. Bevorzugte und besonders bevorzugte Inhibitoren unter diesem Gesichtspunkt der Erfindung sind jene, die als bevorzugt in den vorangehenden Abschnitten genannt werden. Schliesslich wird eine Hemmung beobachtet, wenn der Inhibitor ein Inhibitor einer sauren Proteinase ist. Auch hier sind unter diesem Aspekt der Erfindung bevorzugte und besonders bevorzugte Inhibitoren solche, die als bevorzugt in den vorangegangenen Abschnitten genannt werden.

Zu den typischen Pflanzenschädlingen gehören Insekten, Milben, Pilze und Bakterien; dabei sind Insekten von besonderer Bedeutung; Tabelle B gibt einige bevorzugte Repräsentanten von Zielpflanzen an. Sie sind genetisch manipuliert, produzieren die angegebenen Proteinase-Inhibitoren und sind so in der Lage, die angegebenen Zielinsekten zu bekämpfen. Diese beispielhafte Auflistung soll auf keinen Fall limitierend für die Erfindung sein. Typische zu bekämpfende Schädlinge werden in den vorangehenden Abschnitten erwähnt.

D. DNA-Sequenzen

Bei der Transformation von Pflanzen, die nötig ist, um die erfindungsgemässen transgenen Pflanzen zu erzeugen und das erfindungsgemässe Verfahren anzuwenden, werden DNA-Sequenzen, die die Proteinase-Inhibitoren oder Vorläufer der Proteinase-Inhibitoren kodieren, für die Manipulation eingesetzt. Entsprechend betrifft diese Erfindung auch die erfindungsgemäss verwendeten DNA-Sequenzen. Die Transformationen werden nachfolgend detailliert beschrieben.

Generisch gesehen betrifft die Erfindung eine im wesentlichen reine DNA-Sequenz, die eine kodierende Sequenz für einen Proteinase-Inhibitor umfasst und die ausgewählt ist aus der Gruppe bestehend aus Inhibitoren von Thiolproteinasen, Metallproteinasen, sauren Proteinasen und Nicht-Trypsin-Serin-Proteinasen, oder eine kodierende Sequenz für einen oder mehrere Vorläufer, die an der biologischen Synthese eines Proteinase-Inhibitors teilhaben, vorzugsweise eines Nicht-Trypsin-Proteinase-Inhibitors, ausgewählt aus der Gruppe bestehend aus Thiolproteinasen, Metallproteinasen, sauren Proteinasen und Serin-Proteinasen. Ausserdem betrifft die Erfindung eine im wesentlichen reine DNA-Sequenz, die einen Proteinase-Inhibitor kodiert, ausgewählt aus der Gruppe bestehend aus Inhibitoren von Thiolproteinasen, Metallproteinasen, sauren Proteinasen und Nicht-Trypsin-Serin-Proteinasen.

Die Erfindung ist auch so zu verstehen, dass sie Vektoren umfasst, die Sequenzen tragen, die Proteinase-Inhibitoren oder Vorläufer von Proteinase-Inhibitoren kodieren, und solche Sequenzen, die in im wesentlichen reiner Form isoliert wurden.

Bevorzugt sind solche Sequenzen für nicht-pflanzlichen Inhibitoren, die wirksam gegen Pflanzenschädlinge, insbesondere Insekten, Milben, Pilze und Bakterien sind. Von besonderer Bedeutung sind solche Sequenzen, die nicht-pflanzlichen Inhibitoren oder Vorläufer von nicht-pflanzlichen Inhibitoren kodieren, die wirksam gegen Insekten sind.

Es handelt sich typischerweise um Sequenzen, die entweder aus einem Tier, einem Bakterium, oder einem Pilz stammen oder die eine wesentliche Sequenzhomologie mit Proteinase-Inhibitorgenen aufweisen, die aus einem der vorgenannten Organismen stammen.

In den Fällen, in denen die Erfindung auf einen Inhibitor einer Nicht-Trypsin-Serin-Proteinase gerichtet ist, handelt es sich um einen Inhibitor, ausgewählt aus der Gruppe bestehend aus Inhibitoren von Chymotrypsin, Thrombin, Plasmin, Elastase, Kallikrein, Subtilisin, Cathepsin G, Chymase, Acrosin, Plasminogen-Aktivator, C$\bar{\text{I}}$-Esterase, Enterokinase, Tryptase, Post-Prolin-schneidendem Enzym (Prolyl-Endoproteinase), ATP-abhängiger Protease, Thermitase, Mastzellen-Proteinase I und II, *Streptomyces griseus*-Proteinase A, *Staphylococcus aureus* V8-Proteinase, *Tenebrio* $\alpha$-Proteinase, Urokinase, Blutgerinnungsfaktoren, Komplement-aktivierenden Faktoren und den Serin-Carboxypeptidasen sowie einem Inhibitor einer Proteinase, die eine wesentliche strukturelle und oder funktionelle Aehnlichkeit mit einer der genannten hat.

Wie bereits erwähnt, umfasst die Erfindung auch DNA-Sequenzen, die einen oder mehrere Vorläufer kodieren, die an der biologischen Synthese eines Proteinase-Inhibitors teilhaben, wobei besagter Inhibitor ausgewählt ist aus der Gruppe bestehend aus Thiolproteinasen, Metallproteinasen, sauren Proteinasen und Serin-Proteinasen. In diesem Fall ist die Serin-Proteinase ausgewählt aus der Gruppe bestehend aus Trypsin, Chymotrypsin, Thrombin, Plasmin, Elastase, Kallikrein, Subtilisin, Cathepsin G, Chymase, Acrosin, Plasminogen-Aktivator, C$\bar{\text{I}}$-Esterase, Enterokinase, Tryptase, Post-Prolin-schneidendem Enzym (Prolyl-Endoproteinase), ATP-abhängiger Protease, Thermitase, Mastzellen-Proteinase I und II, *Streptomyces griseus*-Proteinase A, *Staphylococcus aureus* V8-Proteinase, *Tenebrio* $\alpha$-Proteinase, Urokinase, Blutgerinnungsfaktoren, Komplementaktivierenden Faktoren und den Serin-Carboxypeptidasen und einer Proteinase, die eine wesentliche strukturelle und funktionelle Aehnlichkeit mit einer der genannten hat.

Spezifischer gesehen kann es einer Sequenz sein oder können es mehrere Sequenzen sein, die Vorläufer kodieren, die zu einem Inhibitor führen, der ein Vertreter der Bowman-Birk Inhibitor-Familie, der Sojabohnen-Kunitz Inhibitor-Familie, der Rinderpankreas-Trypsin (Kunitz) Inhibitor-Familie, der Kazal-Trypsin Inhibitor-Familie, der *Streptomyces*-Subtilisin Inhibitor-Familie, der Kartoffel-Inhibitor I-Familie, der Kartoffel-Inhibitor II-Familie, der $\alpha_1$-Proteinase Inhibitor-Familie, der Hirudin-Familie, der Bdellin-Familie, der Eglin-Familie, der Inter-$\alpha_1$-Trypsin Inhibitor-Familie, der Serpin-Ueberfamilie, der C$\bar{\text{I}}$-Inhibitor-Familie, der *Ascaris* Inhibitor-Familie, der Leupeptine, der Antipaine, der Elastinal oder Chymostatin ist oder ein Inhibitor von Chymotrypsin oder ein Inhibitor, der im wesentlichen strukturelle und funktionelle Aehnlichkeit mit einem Chymotrypsin-Inhibitor hat.

Ein weiterer Erfindungsgegenstand besteht darin, dass die DNA-Sequenz einen Proteinase-Inhibitor oder einen oder mehrere Vorläufer eines Proteinase-Inhibitors kodiert, der zur Klasse der Inhibitoren von Thiolproteinasen gehört. Bevorzugte Beispiele hierfür sind die Inhibitoren von Papain, Bromelain, Ficin, Calpain, Cathepsin B, Cathepsin C, Cathepsin L, Cathepsin H, Cathepsin S, Chymopapain, Clostripain, Asclepain, Prolyl-Endopeptidase, Pyroglutamyl-Peptidase, Dipeptyl-Proteinase I, Hefe-Proteinase B, *Streptococcus* Proteinase, *Staphylococcus* Thiolproteinase und Actinidin sowie von Proteinasen, die eine wesentliche strukturelle und funktionelle Aehnlichkeit mit einer der vorgenannten aufweisen.

Unter diesem Gesichtspunkt ist ein Inhibitor besonders bevorzugt, der entweder ein Cystatin, Calpastatin, Bromelain-Inhibitor, Antipain, Leupeptin, Chymostatin oder E64 oder ein Abkömmling davon ist, oder einer, der eine wesentliche strukturelle und funktionelle Aehnlichkeit damit hat. In den Fällen, in denen der Inhibitor ein Cystatin ist, kann er ausgewählt sein aus der Gruppe bestehend aus Hühnereiweiss-Cystatin, Human-Cystatin A, Human-Cystatin B, Human-Cystatin C, Human-Cystatin S, Ratten-Cystatin α, Ratten-Cystatin β und Kininogen, beispielsweise L-Kininogen und H-Kininogen.

Des weiteren kann es sich bei der DNA-Sequenz um eine Sequenz handeln, die einen Proteinase-Inhibitor oder einen oder mehrere Vorläufer eines Proteinase-Inhibitors kodiert, wobei die Proteinase eine Metallproteinase ist. Beispielsweise kann es sich dabei um den Inhibitor der Carboxypeptidase A, Carboxypeptidase B, Aminopeptidase, Kollagenase, Calcium-abhängigen neutralen Proteinase, Thermolysin, des Angiotension-umbauenden Enzyms, der renalen Dipeptidase, der Enkephalinase, Gelatinase oder Keratinase handeln oder um einen Inhibitor einer Proteinase, die eine wesentliche strukturelle und funktionelle Aehnlichkeit mit einer der vorgenannten hat.

Besonders bevorzugt unter diesem Blickwinkel sind DNA-Sequenzen, die Inhibitoren oder einen oder mehrere Vorläufer von nicht-pflanzlichen Inhibitoren kodieren, und die Inhibitoren vorzugsweise aus der Gruppe bestehend aus Säugetier Kollagenase-Inhibitor, α₂-Makroglobulin, Gewebe-Bradykinin-verstärkendem Peptid, Phosphoramidon, Bestatin und Amastatin sowie Inhibitoren, die eine wesentliche strukturelle und funktionelle Aehnlichkeit mit dem vorgenannten haben, sind.

Schliesslich betrifft die Erfindung auch DNA-Sequenzen, die einen Proteinase-Inhibitor oder einen oder mehrere Vorläufer eines Proteinase-Inhibitors kodieren, der ein Inhibitor einer sauren Proteinase ist. Von besonderem Interesse sind unter diesem Aspekt jene Fälle, in denen es sich um einen Inhibitor von Pepsin, Renin, Cathepsin D, Chymosin, Penicillinopepsin oder *Scytalidium* saurer Protease B oder um einen Inhibitor einer Proteinase handelt, die eine wesentliche strukturelle und funktionelle Aehnlichkeit mit einer der vorgenannten hat.

Besonders bevorzugt sind dabei solche Fälle, in denen der Inhibitor ein Pepstatin, *Ascaris* Carboxylproteinase-Inhibitor, *Bauhinia* Pepsin Inhibitor, oder *Scopolia japonica* Proteinase-Inhibitor ist oder eine wesentliche strukturelle und funktionelle Aehnlichkeit mit einem dieser Inhibitoren aufweist.

E. Vektoren

Vektoren, die durch Standardverfahren hergestellt werden und die die oben beschriebenen DNA-Sequenzen enthalten, stellen einen zusätzlichen Gegenstand der Erfindung dar. Vektoren sind rekombinante DNA-Sequenzen, die zu Isolierungs- und Vermehrungszwecken der erwähnten DNA-Sequenzen und für die Transformation von geeigneten Wirten mit diesen Sequenzen benutzt werden können. Bevorzugte Vektoren für Isolierung und Vermehrung sind Plasmide, die in einem geeigneten Wirtsmikroorganismus, beispielsweise *E. coli*, vermehrt werden können. Bevorzugte Vektoren für die Transformation sind jene, die für die Transformation von Pflanzenzellen oder von *Agrobacterium* geeignet sind. Insbesondere in den Fällen, in denen Pflanzenzellen, nicht Protoplasten, transformiert werden sollen, ist der bevorzugte Vektor ein vom Ti-Plasmid abgeleiteter Vektor. Für den direkten Gentransfer in Protoplasten kann jeder der erwähnten Vektoren benutzt werden. Geeignete Vektoren, die als Ausgangsmaterial benutzt werden können, sind Stand der Technik. Geeignete Vektoren zur Transformation von Pflanzengewebe und Protoplasten sind in de Framond et al. (1983), An et al. (1985), Potrykus et al. (1985) und Rothstein et al. (1987) beschrieben. Ausserdem sind viele weitere Vektoren bekannt, die im Rahmen der vorliegenden Erfindung als Ausgangsmaterial geeignet sind.

Die Konstruktion und die Vermehrung der Vektoren kann in einem geeigneten Wirt, beispielsweise in *E. coli*, erfolgen. Geeignete *E. coli*-Stämme schliessen HB101, JM83, DH1, DH5α, LE392 usw. ein. Die erfindungsgemässen Vektoren können als solche im direkten Gentransfer oder in der Mikroinjektionstechnik benutzt werden. In bestimmten Fällen kann es vorteilhaft sein, den Vektor vor dem Gebrauch zu linearisieren. Alternativ dazu können die Vektoren in einen *Agrobacterium*-Wirt transferiert werden. Dieser Transfer wird durch konventionelle Techniken, wie "biparental mating" (Simon et al., 1983b), "triparental mating" (Ditta et al., 1980) oder Transformation (Holsters et al., 1978) erreicht. Geeignete *Agrobacterium*-Stämme schliessen *A. tumefaciens* LBA4404, CIB542 und C58Z707 ein, ohne jedoch darauf beschränkt zu sein.

Bevorzugte Vektoren sind solche, die die oben erwähnten bevorzugten DNA-Sequenzen umfassen. Ausserdem sind Vektoren bevorzugt, die in Pflanzenzellen oder in *Agrobacterium* funktionieren. Besonders bevorzugt sind die in den Beispielen beschriebenen Vektoren.

F. Verfahren der Herstellung

Die Erfindung umfasst ausserdem ein Verfahren zur Herstellung der erwähnten DNA-Sequenzen, in denen die DNA-Sequenz aus einer natürlich vorkommenden Quelle isoliert wird, gegebenenfalls mutiert, oder chemisch oder enzymatisch synthetisiert.

Die Erfindung umfasst auch ein Verfahren zur Herstellung der erwähnten Vektoren, die eine wie oben definierte DNA-Sequenz enthalten, dadurch gekennzeichnet, dass die besagte DNA-Sequenz in einen Vektor eingefügt wird, der in Pflanzenzellen oder in *Agrobacterium* funktioniert.

Des weiteren umfasst die Erfindung ein Verfahren zur Herstellung von wie oben definierten transgenen Pflanzen, dadurch gekennzeichnet, dass eine Pflanzenzelle mit einem Vektor, der eine erfindungsgemässe DNA-Sequenz enthält, transformiert oder mit *Agrobacterium*, das solche einen Vektor enthält, kokultiviert wird, und dass die Pflanzenzelle zu einer Pflanze regeneriert wird.

Bevorzugte Verfahren sind solche, die zu den bevorzugten erfindungsgemässen DNA-Sequenzen, Vektoren und transgenen Pflanzen führen. Besonders bevorzugt sind die in den Beispielen beschriebenen Verfahren. **BEISPIELE** Die folgenden Beispiele dienen dazu, sowohl allgemeine als auch spezielle Aspekte der Erfindung zu veranschaulichen. Pflanzliche Proteinase-Inhibitoren auf die Bezug genommen wird, sowie, Eglin B oder C sind nicht Gegenstand der Erfindung.

ABKUERZUNGEN

Bp: Basenpaare.
2,4-D: 2,4-Dichlorphenoxyessigsäure.
Dicamba: 3,6-Dichlor-2-methoxybenzoesäure.
EDTA: 1-Ethylendiamin N,N,N′, N′-tetraessigsäure.
kBp: Kilobasenpaare.
MES: 2-(N-Morpholino)ethansulfonsäure.
MW: Molekulargewicht.
NAA: α-Naphthalinessigsäure.
PEG: Polyethylenglykol.
SDS: Natriumdodecylsulfat.
Tris-HCl: Tris(hydroxymethyl)methylaminhydrochlorid.
Upm: Umdrehungen pro Minute.

MEDIEN

SH-O-Medium: Medium von Schenk und Hildebrandt (1972); ohne Hormone. SH-Medium kann flüssig oder fest sein, verfestigt durch 0,8 % Agar oder 0,5% GelRite$^{®}$. Das Medium wird normalerweise hitzesterilisiert, indem 15 bis 20 Minuten bei etwa 110° bis 121°C autoklaviert wird.

SH-30 Medium: Sh-O Medium, das 30 μM Dicamba enthält.

SH-45 Medium: SH-O Medium, das 45 μM Dicamba enthält.

MS-Medium und OMS-Medium: Medien von Murashige und Skoog (1962). Die Medien können durch 0.8 % Agar oder Agarose oder mit 0.5 % GelRite$^{®}$ verfestigt sein.

Beasley und Ting Medium (Keimungsmedium für Embryonen): Medium nach Beasley und Ting (1973).

KM-8p Medium: Dieses Medium umfasst Makrolemente, Mikroelemente und Fe-EDTA wie von Kao und Michayluk (1975) beschrieben sowie die folgenden organischen Verbindungen:
Biotin (0,01 mg/Liter), Pyridoxin-HCl (1 mg/Liter), Thiamin-HCl (10 mg/Liter), Nicotinamid (1 mg/Liter), Nicotinsäure (0,1 mg/Liter), Folsäure (0,4 mg/Liter), D-Ca-Pantothenat (1 mg/Liter), p-Aminobenzoesäure (0,02 mg/Liter), Cholinchlorid (1 mg/Liter), Riboflavin (0,2 mg/Liter), Vitamin B12 (0,02 mg/Liter), Glycin (0,1 mg/Liter), Saccharose (0,25 g/Liter), Glucose (68,4 g/Liter), Mannit (0,25 g/Liter), Sorbit (0,25 g/Liter), Cellobiose (0,25 g/Liter), Fructose (0,25 g/Liter), Mannose (0,25 g/Liter), Rhamnose (0,25 g/Liter), Ribose (0,25 g/Liter), Xylose (0,25 g/Liter), myo-Inosit (0,1 g/Liter), Zitronensäure (40 mg/Liter), Fumarsäure (40 mg/Liter), Apfelsäure (40 mg/Liter), Na-Pyruvat (20 mg/Liter), Adenin (0,1 mg/Liter), Guanin (0,03 mg/Liter), Thymidin (0,03 mg/Liter), Uracil (0,03 mg/Liter), Hypoxanthin (0,03 mg/Liter), Cytosin (0,03 mg/Liter) Glutamin (5,6 mg/Liter), Alanin (0,6 mg/Liter), Glutaminsäure (0,6 mg/Liter), Cystein (0,2 mg/Liter), Asparagin, Asparaginsäure, Cystin, Histidin, Isoleucin, Leucin, Lysin, Methionin, Phenylalanin, Prolin, Serin, Threonin, Tryptophan, Tyrosin und Valin (jede 0,1 mg/Liter).

Die Lösung wird filtersterilisiert. Der End-pH 5.8. Die Makroelemente werden als 10fach konzentrierte, die Mikroelemente als 1000fach konzentrierte Stammlösung angesetzt. Zitronen-, Fumar- und Apfelsäure sowie Na-Pyruvat werden als 100fach konzentrierte Stammlösung angesetzt und mit $NH_4OH$ auf pH 6,5 eingestellt. Adenin, Guanin, Thymidin, Uracil, Hypoxanthin und Cytosin werden als 1000fach konzentrierte Stammlösung angesetzt und mit $NH_4OH$ auf pH 6,5 eingestellt. Die Aminosäuren werden als 10fach konzentrierte Stammlösung (pH 6,5 mit $NH_4OH$) hinzugefügt, um die Endkonzentrationen zu erhalten. Die Vitaminstammlösung wird normalerweise als 100fach konzentrierte Lösung bereitet.

N6 Medium: Dieses Medium umfasst Makroelemente, Mikroelemente und Fe-EDTA wie von Chu et al. (1975) beschrieben, sowie die folgenden organischen Verbindungen: Pyridoxin-HCl (0,5 mg/Liter), Thiamin-HCl (0,1 mg/Liter), Nicotinsäure (0,5 mg/Liter), Glycin (2,0 mg/Liter), und Saccharose (30 g/Liter). Die Lösung wird autoklaviert. Der End pH ist 5,6. Makroelemente werden als 10fach konzentrierte Stammlösung bereitet und Mikroelemente als 1000fach konzentrierte Stammlösung. Die Vitaminstammlösung wird normalerweise als 100fach konzentrierte Lösung bereitet.

YEB Medium: 5 g/Liter Rindfleischextrakt, 1 g/Liter Hefeextrakt, 5 g/Liter Pepton, 5 g/Liter Saccharose, mit NaOH auf pH 7,2 eingestellt, 2 mM $MgCl_2$-Zugabe nach dem Autoklavieren.

Agarose: Die Herstellung und Reinigung werden beispielsweise durch Guiseley und Renn (1975) beschrieben. Agarose ist einer der Bestandteile von Agar. Kommerziell erhältlicher Agar besteht normalerweise aus einer Mischung von neutraler Agarose und ionischem Agaropektin mit einer grossen Anzahl von Seitengruppen. Gewöhnlicherweise bleibt eine bestimmte Zahl von Seitenketten intakt und bestimmt die physikochemischen Eigenschaften der Agarose, wie Gelbildung und Schmelztemperatur. Agarose, die bei niedrigen Temperaturen schmilzt, besonders Sea-Plaque® Agarose, ist ein bevorzugtes Festigungsmittel in dem hier beschriebenen Verfahren.

Caseinhydrolysat: Caseinhydrolysat - Enzymatisches Hydrolysat von Rindermilch, Typ 1, Sigma Co., St. Louis, MO, USA.

Cellulase RS und R-10: Yakult Honsha Co. Ltd., Tokio, Japan.

GelRite®: GelRite Gellan Gum, Scott Laboratories Inc., Fiskersville, R.I., USA.

Nalgene® Filter: Nalge Co., Division von Sybron Corp., Rochester, New York, USA.

Pectolyase Y-23®: Seishin Pharmaceutical Co. Ltd., Tokio, Japan.

Parafilm®: Parafilm® Labor Film - American Can Co., Greenwich, CT, USA.


ALLGEMEINE VERFAHREN REKOMBINANTEN DNA TECHNOLOGIE

Da viele der im Rahmen dieser Erfindung verwendeten Techniken für den Fachmann auf dem Gebiet der rekombinanten DNA-Technologie Routine sind, wird im Folgenden eine kurze Beschreibung der allgemein verwendeten Verfahren gegeben, sodass diese in den nachfolgenden konkreten Ausführungsbeispielen nicht jedesmal wieder neu angegeben werden müssen. Alle diese Routineverfahren sind bei Maniatis et al. (1982) beschrieben, es sei denn, es wird gesondert darauf hingewiesen.

A. Schneiden mit Restriktionsendonukleasen. Typischerweise sind in dem Reaktionsansatz etwa 50 bis 500 μg/ml DNA in der vom Hersteller, New England Biolabs, Beverly, MA., empfohlenen Pufferlösung enthalten. 2 bis 5 Einheiten Restriktionsendonuklease werden für jedes μg DNA hinzugefügt und der Reaktionsansatz wird bei der vom Hersteller empfohlenen Temperatur eine bis drei Stunden lang inkubiert. Die Reaktion wird durch 10 minütiges Erhitzen auf 65°C oder durch Extraktion mit Phenol beendet; es folgt eine Präzipitation der DNA mit Ethanol. Diese Technik wird auch auf den Seiten 104 bis 106 der Maniatis et al.-Referenz beschrieben.

B. Behandlung der DNA mit Polymerase zur Herstellung glatter Enden. DNA-Fragmente werden in dem vom Hersteller, New England Biolabs, empfohlenen Puffer in einer Konzentration von 50 bis 500 μg/ml einem Reaktionsansatz hinzugefügt. Der Reaktionsansatz enthält alle vier Desoxynukleotidtriphosphate in einer Konzentration von 0.2 mM. Die Reaktion erfolgt während 30 Minuten bei 15°C und wird anschliessend durch 10 minütiges Erhitzen auf 65°C beendet. Für Fragmente, die durch Schneiden mit Restriktionsendonukleasen erhalten werden, welche 5′-überstehende Enden erzeugen, wie EcoRI und BamHI, wird das grosse Fragment, oder Klenow-Fragment, der DNA-Polymerase verwendet. Für Fragmente, die durch Endonukleasen erhalten werden, welche 3′-überstehende Enden erzeugen, wie PstI und SacI, wird die T4-DNA-Polymerase verwendet. Die Verwendung dieser beiden Enzyme wird auf den Seiten 113 bis 121 der Maniatis et al.-Referenz beschrieben.

C. Agarose-Gelelektrophorese und Reinigung von DNA-Fragmenten aus den Gelen. Die Agarose-Gelelektrophorese wird in einem horizontalen Apparat durchgeführt, wie auf den Seiten 150 bis 163 der Maniatis et al.-Referenz beschrieben. Als Puffer wird der dort beschriebene Tris-Boratpuffer verwendet. Die DNA-Fragmente werden durch 0.5 μg/ml Ethidiumbromid gefärbt, das entweder im Gel- oder Tankpuffer bereits während der Elektrophorese vorhanden ist oder aber erst nach der Elektrophorese zugegeben wird. Die DNA wird durch Beleuchtung mit kurz- oder langwelligem Ultraviolettlicht sichtbar gemacht. Wenn die Fragmente vom Gel abgetrennt werden sollen, wird eine Agarose verwendet, die bei niedriger Temperatur geliert und von Sigma Chemical, St. Louis, Missouri, bezogen werden kann. Nach der Elektrophorese wird das gewünschte Fragment ausgeschnitten, in ein Plastikröhrchen gegeben, etwa 15 Minuten auf 65°C erhitzt, dreimal mit Phenol extrahiert und zweimal mit Ethanol gefällt. Dieses Verfahren ist gegenüber dem von Maniatis et al. auf Seite 170 beschriebenen leicht verändert.

D. Anknüpfen synthetischer Linkerfragmente an DNA-Enden. Wenn eine neue Endonukleaseschnittstelle an das Ende eines DNA-Moleküls angefügt werden soll, wird das Molekül gegebenenfalls zuerst mit DNA-Polymerase behandelt, um glatte Enden zu erzeugen, wie in Abschnitt B beschrieben. Etwa 0.1 bis 1.0 µg dieses Fragments wird zu etwa 100 ng phosphorylierter Linker-DNA (New England Biolabs) zugegeben, die in einem Volumen von 20 µl bis 30 µl eines vom Hersteller empfohlenen Puffers vorliegt, zusammen mit 2 µl T4 DNA-Ligase (New England Biolabs) und 1 mM ATP. Nach einer Inkubation über Nacht bei 15°C wird die Reaktion durch 10 minütiges Erhitzen auf 65°C beendet. Der Reaktionsansatz wird in einem für die Restriktionsendonuklease, welche die synthetische Linkersequenz schneidet, geeigneten Puffer auf etwa 100 µl verdünnt. Ungefähr 50 bis 200 Einheiten dieser Endonuklease werden diesem Ansatz hinzugefügt. Die Mischung wird 2 bis 6 Stunden bei der angemessenen Temperatur inkubiert, dann wird das Fragment einer Agarose-Gelelektrophorese unterworfen und wie in Abschnitt C beschrieben gereinigt. Das resultierende Fragment sollte nun Endigungen aufweisen, wie sie durch Schneiden mit der jeweiligen Restriktionsendonuklease erzeugt werden. Diese Enden sind gewöhnlich kohäsiv, so dass das resultierende Fragment nun leicht mit anderen Fragmenten, die die gleichen kohäsiven Enden aufweisen, verknüpft werden kann.

E. Entfernen von 5′-terminalen Phosphaten von DNA-Fragmenten. Die Rezirkularisation eines Vektors während der Plasmidklonierung kann durch die Behandlung des Vektorplasmids mit Phosphatase vermindert werden (diskutiert auf Seite 13 der Maniatis et al.-Referenz). Nach der Verdauung der DNA mit der richtigen Restriktionsendonuklease wird eine Einheit alkalische Phosphatase aus dem Darm von Kälbern zugegeben, die von Boehringer-Mannheim, Indianapolis, IN, bezogen werden kann. Die DNA wird eine Stunde bei 37°C inkubiert und anschliessend zweimal mit Phenol extrahiert und mit Ethanol gefällt.

F. Verknüpfen der DNA-Fragmente. Wenn Fragmente mit komplementären kohäsiven Enden miteinander verknüpft werden sollen, werden etwa 100 ng von jedem Fragment in einem Reaktionsgemisch von 20 µl bis 40 µl mit etwa 0.2 Einheiten T4 DNA-Ligase (New England Biolabs) in dem vom Hersteller empfohlenen Puffer inkubiert. Die Inkubationszeit beträgt zwischen 1 bis 20 Stunden bei einer Temperatur von 15°C. Sollen DNA-Fragmente mit glatten Enden verknüpft werden, so werden diese in der zuvor angegebenen Weise inkubiert, wobei die Menge an T4 DNA-Ligase in diesem Fall auf 2 bis 4 Einheiten erhöht wird.

G. Transformation von *E. coli*. Der *E. coli* Stamm HB101 wird für die meisten Experimente verwendet. Die DNA wird dabei mit dem Kalziumchloridverfahren, das von Maniatis et al., auf den Seiten 250 bis 251 beschrieben wird, in *E. coli* eingeführt. Transformierte Bakterien sind zu einem selektiven Wachstum auf Medien befähigt, die ein geeignetes Antibiotikum enthalten. Diese Fähigkeit zum selektiven Wachstum macht es möglich, die gewünschten Bakterien von denjenigen Wirtsbakterien zu unterscheiden, die keine transformierende DNA erhalten. Die Bestimmung geeigneter Antibiotika für die Selektion von Wirtsbakterien ist Routine und basiert auf der Kenntnis der Resistenzgene, die sich auf der eingeschleusten DNA befinden, sowie der Sensitivität der Wirtsbakterien gegenüber bestimmten Wirkstoffen. Wenn beispielsweise bekannt ist, dass ein bestimmtes Wirtsbakterium gegenüber dem Antibiotikum Ampicillin sensitiv ist und auf der eingeschleusten transformierenden DNA ein entsprechendes Resistenzgen gegen Ampicillin vorliegt, dann ist Ampicillin ein geeignetes Antibiotikum für die Selektion der Transformanten.

H. Screening von *E. coli* auf Plasmide. Nach der Transformation werden die resultierenden Kolonien von *E. coli* mit Hilfe eines schnellen Plasmidisolationsverfahrens auf das Vorhandensein des gewünschten Plasmids geprüft. Zwei gebräuchliche Verfahren werden auf den Seiten 366 bis 369 der Maniatis et al.-Referenz beschrieben.

I. Isolierung von Plasmid-DNA in grossem Massstab. Verfahren zur Isolierung von Plasmiden aus *E. coli* in grossem Massstab werden auf den Seiten 88 bis 94 der Maniatis et al.-Referenz beschrieben.

J. Klonierung in M13 Phagenvektoren. Für die folgende Beschreibung gilt selbstverständlich, dass für Routineverfahren, wie Schneiden mit Restriktionsendonuklease, Verknüpfen etc., die doppelsträngige replikative Form der Phage M13-Abkömmlinge benutzt wird.

**I. Identifizierung von Proteinase-Inhibitoren**

Beispiel 1: In vitro Test zur Selektion von Proteinase-Inhibitoren, die wirksam gegen Zielinsekten sind

A. Test-Verfahren. Proteinase-Inhibitoren werden am Anfang auf ihre Aktivität gegenüber dem gewünschten Zielinsekt geprüft, indem die Fähigkeit einzelner Inhibitoren gemessen wird, die Proteolyse durch Homogenate aus dem Darm des Insekts zu hemmen. Die Därme werden aus mit $CO_2$ anästhesierten oder gefrorenen Larven im zweiten oder dritten Häutungsstadium herausgeschnitten; die Därme werden sofort nach dem Herausschneiden auf Trockeneis gefroren. Die Därme werden in 100 mM Tris, 10 mM EDTA, pH 8,5 homogenisiert (Wolfson und Murdock, 1987), indem 5 bis 10 µl Puffer pro Darm verwendet werden. Das Homogenat wird mit 5000 Upm 5 Minuten lang bei 4°C zentrifugiert, um Partikel abzutrennen, und der Ueberstand wird in 1 ml Aliquots bei -20°C aufbewahrt. Die Protease-Aktivität wird im wesentlichen nach der Methode von Wolfson und Murdock (1987) gemessen, indem $^{14}$C-BSA (New England Nuclear) als Substrat verwendet und die Solubilisierungsrate der Radioaktivität mit der Zeit während 32 Minuten gemessen wird. Das pH-Optimum der Protease-Aktivität wird bestimmt, indem das Ausmass der Hydrolyse in jedem der folgenden Puffer bestimmt wird:

| pH | Puffer |
|-----|--------|
| 2.0 | 200 mM Glycin/HCl |
| 3.0 | 200 mM Glycin/HCl |
| 4.0 | 200 mM β-Alanin/HCl |
| 5.0 | 200 mM Natriumacetat |
| 6.0 | 100 mM Natriumphosphat/bisphosphat |
| 7.0 | 100 mM Natriumphosphat/bisphosphat |
| 8.0 | 100 mM Tris/HCl |
| 9.0 | 200 mM Glycin/NaOH |

Nachfolgende Experimente werden bei dem pH der maximalen Aktivität durchgeführt. Der Inhibitor wird dem Homogenat und dem Testpuffer 5 Minuten bei 25°C inkubiert. Das Substrat, $^{14}$C-methyliertes Rinderserumalbumin (NEN Products, Boston), mit unmarkiertem BSA auf eine spezifische Aktivität von ~ 0,5 µCi/mg eingestellt, wird hinzugefügt und 20 µl Aliquots werden nach 0, 2, 4, 8, 16 und 32 Minuten entnommen. Diese Aliquots werden zu 200 µl eiskalter 10 % Trichloressigsäure gegeben und 30 Minuten in Eis aufbewahrt. Proben werden 10 Minuten mit 5000 Upm bei 4°C zentrifugiert, um unlösliches Protein zu präzipitieren. Zwei 75 µl Aliquots eines jeden Ueberstandes werden in 8 bis 10 ml Scintiverse II Szintillationscocktail gegeben und der radioaktive Zerfall gezählt. Die Solubilisierungsrate der Radioaktivität wird bestimmt und die Rate für jeden Inhibitor wird mit der der Kontrolle verglichen.

B. Screening mit Darmhomogenaten von *Diabrotica undecimpunctata*. In einem typischen Experiment, in dem Darmhomogenate von *D. undecimpunctata* verwendet werden, ist der optimale pH für die Hydrolyse pH 4. Eine Anzahl von Inhibitoren wird in einem Standardtest untersucht, der 10 µl Homogenat, 80000 cpm $^{14}$C-BSA in einem Endvolumen von 200 µl 0,2 M β-Alanin/HCl, pH 4,0 enthält. Die Pepstatin-Aktivität wird in 0,2 M Glycin/HCl, pH 2,0 gemessen. Typische Ergebnisse sind in Tabelle 1 abgebildet.

Tabelle 1

| Wirkung von Proteinase-Inhibitoren auf die Proteinase-Aktivitäten von Darmhomogenaten von *D. undecimpunctata* | | | |
|---|---|---|---|
| Klasse | Inhibitor | Konz. (µg/µl) | % der Kontrolle |
| Thiol | E-64 | 0.25 | 7.2 |
| | Antipain | 0.1 | 19.8 |
| | Cystatin | 0.072 | 30.9 |
| Thiol + Serin | Leupeptin | 0.1 | 21.6 |
| Serin | Aprotinin (Rinderpankreas-Trypsin-Inhibitor) | 0.1 | 85.4 |
| | Hühner-Trypsin-Inhibitor | 0.1 | 62 |
| | Kartoffel I (Chymotrypsin-Inhibitor) | 0.1 | 55.4 |
| | Kartoffel II (Chymotrypsin-Inhibitor) | 0.1 | 42.8 |
| | Soja I (Kunitz) Trypsin-Inhibitor) | 0.1 | 69.7 |
| | Soja II (Bowman-Birk) | 0.1 | 72.5 |
| | Limabohnen-Trypsin-Inhibitor | 0.1 | 161 |
| saure | Pepstatin | 0.02 | 2.1 |

Die Proteolyse durch Darmhomogenate von *D. virgifera* und *D. balteata* zeigt eine ähnlich starke Hemmung durch Inhibitoren der Thiolproteinasen.

C. Hemmung der Proteolyse in Darmhomogenaten von *Diabrotica virgifera* und *Ostrinia nubilalis*. Proteinase-Inhibitoren werden auf ihre Fähigkeit, die proteolytische Aktivität in Darmhomogenaten von *D. virgifera* und *O. nubilalis* zu hemmen, untersucht, indem die oben beschriebenen Verfahren verwendet werden. Die in Tabelle 2 gezeigten Ergebnisse stimmen mit Daten überein, die mit anderen Inhibitoren erhalten werden, die zeigen, dass Darmproteinasen in *Ostrinia*, nicht jedoch in *Diabrotica* durch Serin-Proteinase-Inhibitoren gehemmt werden. Insbesondere wird für Eglin C (Arg45) gezeigt, dass es die Proteolyse durch Darmhomogenate von *O. nubilalis* sehr wirksam hemmt; Eglin C (Arg45) ist bedeutend wirksamer als Eglin C und ein anderer Trypsin-Inhibitor, *Vigna unguiculata* Trypsin-Inhibitor (Hilder et al., 1987).

Tabelle 2

| Hemmung der Proteolyse in Darmhomogenaten von Insekten | | | |
|---|---|---|---|
| Inhibitor | Konz. (µg/µl) | % der Kontrollaktivität | |
| | | *D. virgifera* | *O. nubilalis* |
| Elgin C | 0.5 | 107.6 % | 29.9 % |
| Elgin C (Arg45) | 0.5 | 110.8 % | 3.4 % |
| Leupeptin | 0.002 | 6.2 % | 24.9 % |
| *V. unguiculata* Trypsin-Inhibitor | 0.5 | 99.2 % | 29.5 % |

Tabelle 3

| Konzentration von Inhibitoren, die für eine 70 % Hemmung der Proteolyse durch Darmhomogenate von *O. nubilalis* benötigt wird | |
| --- | --- |
| Inhibitor | IC70 |
| Elgin C (Arg45) | 12 µM |
| Elgin C | 62 µM |
| *V. unguiculata* Trypsin-Inhibitor | 50 µM |

Beispiel 2: Fütterungsversuch zur Selektion von Proteinase-Inhibitoren, die wirksam gegen Zielinsekten sind

Ein anderes Verfahren, die Wirksamkeit von Inhibitoren gegen ein einzelnes Zielinsekt zu bestimmen, ist, den Inhibitor der Nahrung zuzufügen. Die Inhibitorlösung wird auf die Oberfläche der normalen Nahrung (z.B. junge Maisblätter für *Ostrinia nubilalis* und *Heliothis zea*, Tabakblattscheiben für *H. virescens*, Maiswurzeln oder kultiviertes Maisgewebe für *Diabrotica* spec.) aufgetragen und getrocknet. Diese Nahrung wird an frisch geschlüpfte Larven verfüttert und die Ueberlebensrate und das Gewicht nach 5 bis 6 Tagen gemessen. Alternativ dazu können Larven im 2. oder 3. Häutungsstadium vor und 3 Tage nach der Fütterung mit der behandelten Nahrung gewogen werden.

A. Einfluss von Proteinase-Inhibitoren auf das Wachstum von *Heliothis zea*. In einem typischen Experiment werden Maisblattstreifen (1 cm$^2$) mit 12 µl Aliquots von Lösungen von 10 mg/ml Testmaterial benetzt und in einzelne Nahrungstöpfe mit je einer frischgeschlüpften *H. zea*-Larve gelegt. Das Gewicht der Larven wird 5 Tagen gemessen. Kontrollproteine wie Rinderserumalbumin oder Thaumatin verursachen einen signifikanten ($P < 0,05$) Anstieg im Gewicht verglichen mit unbehandelten Blättern. Blätter mit Serin-Proteinase-Inhibitoren wie Kunitz-Trypsin-Inhibitor aus Soja oder Limabohnen Trypsin-Inhibitor verursachen eine signifikante Abnahme im Larvengewicht verglichen mit der Kontrolle (Tabelle 4).

Tabelle 4

| Wirkung von Proteinase-Inhibitoren auf das Wachstum von frisch geschlüpften *H. zea* | | | |
| --- | --- | --- | --- |
| Behandlung | Durchschnittsgewicht (mg) | n | p |
| unbehandelt | 3.41 | 15 | |
| BSA | 6.65 | 13 | 0.005 |
| Thaumatin | 4.57 | 15 | 0.1 |
| Soja Trypsin-Inhibitor I | 1.59 | 19 | 0.005 |
| Limabohnen Trypsin Inhibitor | 2.38 | 13 | 0.1 |

B. Einfluss von Proteinase-Inhibitoren auf das Wachstum von *Diabrotica*. Fütterungsversuche mit *Diabrotica* spec. bestätigen den in vitro Test, der in Beispiel 1 beschrieben ist. In einem repräsentativen Experiment werden Zellen aus einer Maiszellsuspension mit 2,5 % Saccharoselösung, die gewünschte Mengen an Inhibitoren enthält, durchtränkt und an *D. undecimpunctata*-Larven im 2. Häutungsstadium verfüttert. Die Gewichtszunahme nach 5 Tagen ist signifikant (t-Test $p < 0.05$) niedriger als bei Kontrollen, wenn die Zellen mit Leupeptin, Antipain oder Pepstatin in Konzentrationen, die 0.3 mg/ml überschreiten, behandelt werden. In ähnlichen Experimenten wird das Larvenwachstum von *D. virgifera* durch so niedrige Leupeptindosen wie 0.1 mg/ml (Tabelle 5) gehemmt.

Tabelle 5

| Wirkung von Protease-Inhibitoren auf das Wachstum von *Diabrotica*-Larven | | | | |
|---|---|---|---|---|
| Species | Inhibitor | Dosis (mg/ml) | Durchschnittsgewicht (mg) | p |
| *D. undecimpunctata* | keiner | | 7.54 | |
| | Leupeptin | 1.0 | 5.53 | 0.05 |
| | | 3.0 | 5.02 | 0.05 |
| | | 10.0 | 3.55 | 0.05 |
| | Antipain | 1.0 | 5.44 | 0.05 |
| | | 3.0 | 5.79 | 0.05 |
| | | 10.0 | 3.69 | 0.05 |
| | Pepstatin | 0.1 | 7.42 | NS |
| | | 0.3 | 6.18 | 0.05 |
| | | 1.0 | 5.99 | 0.05 |
| | | 3.0 | 6.2 | 0.05 |
| *D. virgifera* | keiner | | 3.67 | |
| | Leupeptin | 0.1 | 2.51 | 0.05 |
| | | 0.3 | 1.91 | 0.05 |
| | | 1.0 | 1.56 | 0.05 |
| | | 3.0 | 1.28 | 0.05 |

C. Einfluss von Proteinase-Inhibitoren auf das Wachstum von *Ostrinia*. Der Einfluss von Eglin C (Arg45) auf das Wachstum von frisch geschlüpften *O. nubilalis*-Larven wird dem oben beschriebenen Test bestimmt. Aliquots der Inhibitor-Lösung werden auf 1 cm$^2$ grosse Maisblattscheiben gegeben; jedes Maisblatt wird in einen separaten Futtertopf zusammen mit einer frisch geschlüpften Larve gegeben. 20 Larven werden für jede Dosis getestet. Die Töpfe werden 6 Tage lang bei 29°C inkubiert und anschliessend die Ueberlebensrate, das Insekten-Endgewicht und die gefressene Blattfläche bestimmt. Die Applikation von 125 µg Eglin C (Arg45) auf jedes Blattstück bewirkt einen bedeutenden Abfall im Endgewicht der Larven (p > 0,05). Typischerweise ergibt der Kontakt mit Eglin C (Arg45) eine Abnahme von etwa 30 % im durchschnittlichen Endgewicht der Larven, verglichen mit unbehandelten Kontrollen.

**II. Gene and Vektoren**

Ein Proteinase-Inhibitor-Gen wird aus der Quelle, in der es natürlicher-oder künstlicherweise vorkommt, isoliert und, wenn nötig, durch konventionelle Verfahren charakterisiert. Wenn das Gen induzierbar ist, wird es durch den zugehörigen Regulator aktiviert. Die RNA, die aus dieser Aktivierung resultiert, wird isoliert und benutzt, um eine cDNA-Bibliothek anzulegen. Diese Bibliothek wird für ein unterscheidendes Screening benutzt, indem radioaktiv markierte cDNA verwendet wird, die aus (1) RNA, die aus dem aktivierten System isoliert wurde, und (2) RNA, die aus einem zweiten, nichtaktivierten System isoliert wurde, erzeugt wurde. cDNA-Klone, die den induzierten Klonen entsprechen, werden anschliessend isoliert und sequenziert.

Wenn von dem gewünschten Gen nicht bekannt ist, ob es induzierbar ist, kann es unter Verwendung immunologischer Verfahren isoliert werden. Das Protease-Inhibitor-Protein wird als Antigen zur Produktion eines Antikörpers verwendet, der dann benutzt wird, um eine rekombinante Phagen cDNA-Bibliothek in λ-GT11 entsprechend dem Verfahren von Young und Davis (1983) zu screenen. Positive Klone werden mit radioaktiv markierten gemischten Oligonukleotiden (Wood et al., 1985) gescreent, die entsprechend der bekannten Aminosäuresequenz des Proteins erzeugt wurden.

Beispiel 3: Proteinase-Inhibitor-Gene

A. Reinigung von Proteinase-Inhibitoren. Viele Proteinase-Inhibitoren sind kommerziell in gereinigter oder teilweise gereinigter Form erhältlich und die Reinigungsvorschriften für zahlreiche andere sind bekannt [siehe Barrett und Salvesen (1986) wegen ausführlicher Referenzen]. Weitere Reinigung von Material kann durch Affinitätschromatographie an Säulen mit den zugehörigen inaktivierten Enzymen erfolgen.

In einer typischen Reinigung mit dem Ananas Bromelain-Inhibitor wird die Enzym-Säule mit Bromelain im wesentlichen wie von Anastasi et al. (1983) für Carboxymethylpapain beschrieben vorbereitet. Das Bromelain wird mit Jodacetamid behandelt, um es zu inaktivieren. Das Carboxymethylbromelain wird anschliessend gemäss der vom Hersteller empfohlenen Methode an CNBr-aktivierte Sepharose (Pharmacia) gekoppelt. Nach intensivem Waschen ist die Säule in 0,05 M $NaPO_4$, pH 4,0, 0,5 M NaCl, 0,1 % Brij 35 äquilibriert und wird mit einem Säulenvolumen 50 mg/ml Rinderserumalbumin im gleichen Puffer behandelt, um unspezifische Bindungsstellen zu blockieren. Die Säule wird sorgfältig gewaschen und das teilweise gereinigte Bromelain (Sigma), gelöst in pH 4,0 Puffer, der 10 % Glycerin an Stelle von Brij 35 enthält, wird auf die Säule aufgetragen. Nach sorgfältigem Waschen mit pH 4,0 Puffer wird die Säule mit 0,05 M $NaPO_4$, pH 11,5, 0,5 M NaCl, 10 % Glycerin eluiert. Die Aktivität wird geprüft, indem die Fähigkeit, den Abbau von $^{14}$C-BSA durch Bromelain zu hemmen, getestet wird.

Wenn Inhibitoren isoliert werden, die einen hohen Aktivitätsgrad gegenüber der Protease eines einzelnen Insektes haben, isoliert werden, kann die Effizienz der Reinigung durch den in Beispiel 1 beschriebenen Test überprüft werden. Um die Selektivität zu erhöhen, kann das Insektenenzym durch Affinitätschromatographie an einer Inhibitor-Säule gereinigt und anschliessend verwendet werden, um eine Affinitätssäule zur Reinigung neuer Inhibitoren herzustellen. Falls nötig können weitere Reinigungen von Inhibitoren zur Proteinsequenzierung mit Hilfe der Reverse Phase HPLC erreicht werden.

B. Aminosäuresequenzierung von Proteinase-Inhibitoren. Im Falle von Proteinen, deren Aminosäurensequenz noch nicht aus der Literatur bekannt ist, wird die Aminosäurensequenz durch automatischen Edman Abbau bestimmt, indem ein Modell 470A Protein Sequencer (Applied Biosystems, Foster City, CA), ausgestattet mit einer on-line Reverse Phase HPLC zur Analyse der Phenylthiohydantoinderivate der Aminosäuren und einem Modell 900 Datenanalysesystem, verwendet wird. Peptide werden durch enzymatischen Abbau mit Trypsin, Lys-C (Lysin-Endopeptidase), Arg-C (Arginin-Endopeptidase) oder Glu-C (*Staphylococcus aureus* Protease V8) hergestellt und vor der Sequenzierung durch Reverse Phase HPLC getrennt.

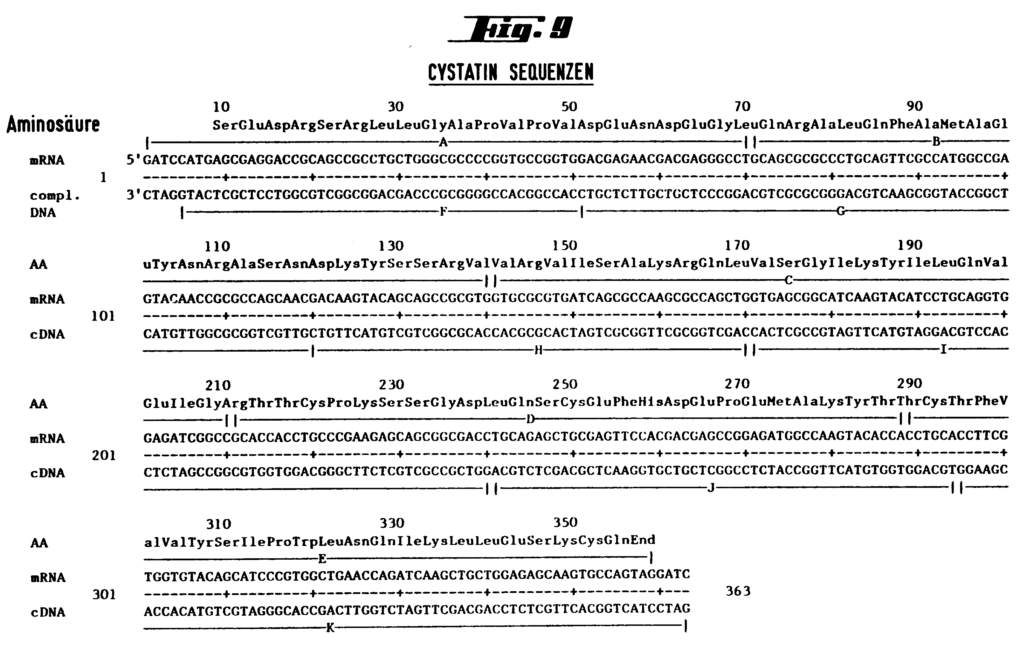

C. Synthese eines Proteinase-Inhibitor-kodierenden Gens: Hühnereiweiss-Cystatin. Für Proteine mit weniger als 150 Aminosäuren, deren Aminosäurensequenz vollständig bekannt ist, kann ein Gen durch DNA-Synthese konstruiert werden. Im Fall von Hühnereiweiss-Cystatin wird die Aminosäurensequenz (Schwabe et al., 1984; Turk et al., 1983) rücktranslatiert, indem der genetische Code mit der Codonfrequenz verwendet wird, die aus allen verfügbaren Maisproteinen der GenBank Datenbank unter Benutzung der Computerprogramme der Genetik-Computer-Gruppe der Universität von Wisconsin berechnet wurde. Translations-Stop- und Startsignale werden zusammen mit BamHI Linkern zur Erleichterung bei nachfolgenden Manipulationen an beiden Enden angefügt. Dieser Prozess ergibt die in Fig. 9 gezeigte Sequenz.

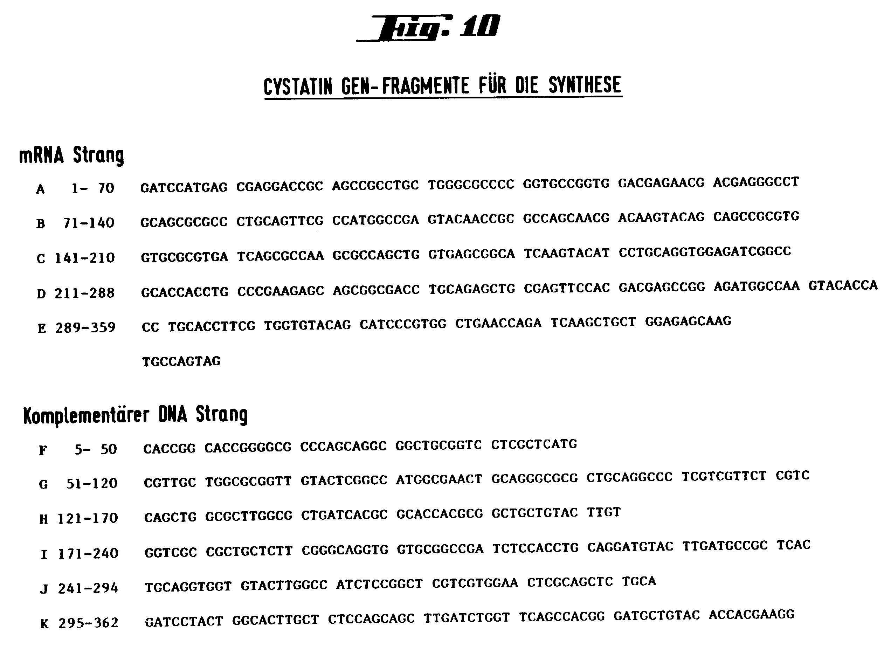

Oligonukleotide, die den Abschnitten A bis K (Fig. 10) entsprechen, werden mit Hilfe eines Modell 380A DNA Synthesizers (Applied Biosystems, Foster City CA) mit β-Cyanoethylchemie synthetisiert.

Das Gen wird in drei Schritten zusammengesetzt:

(1) Anfügen von 5′-Phosphat: Ein 5′-Phosphat wird an die 5′-Enden aller Fragmente mit Ausnahme der Fragmente A und K angefügt, indem 40 pmol jedes Fragments B, C, D, E, F, G, H, I und J und die T4 Polynukleotid-Kinase vermischt werden, gemäss dem von Maniatis et al. (1982) beschriebenen Verfahren.

(2) Hybridisierung: Nach dem Entfernen von überschüssigem Reagens durch Phenol/Chloroform-Extraktion, Chloroform-Extraktion und Ethanol-Fällung wird der Niederschlag, der die phosphorylierten Fragmente enthält, in T4 Ligase-Puffer gelöst. 40 pmol von jedem Fragment A und K werden hinzugefügt, die Mischung wird auf 85°C erhitzt, anschliessend langsam auf 15°C abgekühlt und bei dieser Temperatur für wenigstens 4 Stunden inkubiert, damit die Fragmente hybridisieren können.

(3) Verknüpfen: ATP wird bis zu einer Konzentration von 1 mM zusammen mit T4 Ligase hinzugefügt und die Inkubation wird 4 Stunden lang fortgesetzt. Die Reagentien werden durch Extraktion und Fällung wie in Schritt (1) entfernt. Um die Wirksamkeit der Reaktion zu überprüfen, wird ein Aliquot der Produkte in einem 10 bis 15 % Acrylamidgel analysiert. Eine Bande, die 363 Bp entspricht, ist sichtbar. Falls nötig, kann das entsprechende Fragment durch präparative Gelelektrophorese gereinigt werden, bevor fortgefahren wird, es mit dem Vektor zu verknüpfen.

D. Synthese eines Gens, das einen Proteinase-Inhibitor kodiert: Eglin C und Eglin C-Mutanten.

(1) Eglin C-Gen: Die Herstellung des Plasmids pML147, das das Eglin C-Gen enthält, wird in EP 146 785 beschrieben. Der *E. coli* Stamm HB101/pML147, der dieses Plasmid enthält, wurde am 28. Januar 1988 bei der Deutschen Sammlung von Mikroorganismen (DSM), Mascheroder Weg 1b, D-3300 Braunschweig, Bundesrepublik Deutschland unter der Hinterlegungsnummer DSM 4380 hinterlegt. Das 230 Bp EcoRI-BamHI Fragment, das das komplette Eglin C-Gen enthält, wird aus 10 μg Plasmid pML147 durch Schneiden mit den Restriktionsendonukleasen EcoRI und BamHI und nachfolgende Elektrophorese in 1,5 % Agarose mit niedrigem Schmelzpunkt isoliert. Das EcoRI Ende wird durch Verknüpfen mit einem Linker, der so gewählt ist, dass die kodierende Sequenz im richtigen Leserahmen ist, ist ein BamHI Ende überführt. Nach der Isolierung wird das modifizierte Fragment in die BamHI-Schnittstelle des CaMV35S Promotors im pCIB710 Vektor eingefügt, wie es in Beispiel 5 beschrieben ist.

(2) M13 Klonierung des Eglin C-Gens: Etwa 0,5 μg des 230 Bp EcoRI-BamHI Fragments, das das komplette Eglin C Gen enthält, wird aus 10 μg Plasmid pML147 wie oben beschrieben isoliert. Dieses DNA-Fragment (10 ng) wird mit 40 ng M13mp8 vermischt, das vorher mit EcoRI und BamHI geschnitten wurde, und in 50 mM Tris-HCl pH 7,4, 10 mM MgCl$_2$, 10 mM ATP, 10 mM Dithiothreitol in Gegenwart von 0,125 Einheiten T4 DNA-Ligase in einem Volumen von 15 μl inkubiert (Zoller et al., 1983). Die resultierende Lösung wird zur Transformation von *E. coli* Stamm JM101 benutzt (Zoller et al., 1983). Die Transformationsmischung wird auf X-Gal(IPTG-Indikator Agar)Platten aufgetragen (Zoller et al., 1983). 40 blaue (Wildtyp) und 650 farblose Plaques werden erhalten.

(3) Herstellung von M13mp8 Einzelstrang-DNA: 2 ml *E. coli* JM101 Kultur, die in L Medium (10 g/Liter Bactotrypton, 5 g/Liter Bacto Hefeextrakt, 5 g/Liter NaCl, 5 g/Liter Glucose, 0,1 g/Liter Ampicillin) bis zu einer optischen Dichte (OD$_{623}$) von etwa 0,5 wuchsen, werden mit einem farblosen Plaque, der von der Agarplatte entnommen wurde (siehe oben), inokuliert und während etwa 4 bis 5 Stunden bei 37°C mit 180 Upm bewegt. Anschliessend wird die gewachsene Kultur 5 Minuten in einer Eppendorf Zentrifuge zentrifugiert. Der Ueberstand wird in ein frisches Zentrifugenröhrchen gebracht und erneut zentrifugiert. 200 μl 20 % PEG und 1,5 M NaCl werden hinzugefügt und die Mischung wird 20 Minuten bei Raumtemperatur inkubiert und noch einmal zentrifugiert. Der Ueberstand wird verworfen und das Pellet wird in 100 μl 50 mM Tris-HCl, pH 7,8, 1 mM EDTA (TE Puffer) aufgelöst. Die Mischung wird mit 50 μl Phenol/TE Puffer vermischt, 15 Minuten bei Raumtemperatur inkubiert und anschliessend 5 Minuten in einer Eppendorf Zentrifuge zentrifugiert. 10 μl Na-Acetat-Puffer, pH 6, und 250 μl absoluter Alkohol werden zu 100 μl Ueberstand gegeben. Die Mischung wird bei -20°C über Nacht inkubiert und anschliessend wie oben beschrieben 10 Minuten zentrifugiert. Das Pellet wird mit 1 ml 80 % Ethanol gewaschen und wiederum zentrifugiert. Das Pellet wird 10 Minuten bei Raumtemperatur getrocknet und anschliessend in 50 μl TE Puffer gelöst. Die Lösung enthält etwa 5 μg M13mp8 Einzelstrang-DNA.

(4) Herstellung des Gens, das Eglin C (Arg45) kodiert: Es wird ein Verfahren benutzt, das als "site directed mutagenesis" bekannt ist (Zoller et al., 1983). Für die Mutagenese des Eglin C Gens wird das folgende Nukleotid durch chemische Synthese hergestellt:

$$5'\text{-CT CCT GTT ACT C}\overset{*}{\text{G}}\text{G GAC C-3'}$$

Die mit dem Stern versehene Base unterscheidet sich von der entsprechenden Base im kodierenden Strang des Eglin C Gens (ein T) und sorgt letztlich für die Mutation von Leu45 zu Arg45. 10 μl des Oligonukleotids (1 OD/ml = 500 ng) werden in 20 μl 0,07 M Tris-HCl pH 7,6, 0,01 M MgCl$_2$, 50 mM Dithiothreitol mit γ-$^{32}$P ATP und T4 Polynukleotid-Kinase (Boehringer) gemäss des Verfahrens von Maniatis et al. (1982) (S. 125) aktiviert. Das aktivierte Oligonukleotid wird in 10 μl TE Puffer (50 ng/μl) gelöst.

1 μg M13mp8 Einzelstrang-DNA und 50 ng des aktivierten Oligonukleotid-Primers werden in 10 μl 50 mM Tris-HCl pH 7,8 und 100 mM MgCl$_2$ 30 Minuten bei 45°C und anschliessend bei Raumtemperatur 5 Minuten zur Paarung inkubiert. Je 1 μl 10 mM dATP, dGTP, dCTP und dTTP, 1 μl T4 DNA-Ligase, 2 μl 50 mM Dithio-

threitol, 1 μl 10 mM ATP, 1 μl Gelatine (5 mg/ml), 1 μl 10fach konzentrierter Klenow-Puffer (0,66 M Tris-HCl pH 7,6, 50 mM MgCl$_2$, 50 mM Dithiothreitol) und 1 μl (2,5 Einheiten) DNA-Polymerase (Klenow-Fragment) werden hinzugefügt. Die Mischung wird 5 Minuten bei 22°C und dann 16 Stunden bei 15°C inkubiert und anschliessend elektrophoretisch in 1 % Agarose aufgetrennt. Die resultierende zirkuläre, doppelsträngige DNA wird mit Ethidiumbromid angefärbt und durch Elektroelution aus dem Gel gelöst (etwa 10 ng in 15 μl TE Puffer). Mit 5 μl (etwa 3,5 ng) der auf diese Weise erhaltenen DNA wird *E. coli* Stamm JM101 transformiert und auf X-Gal/IPTG-Indikatorplatten ausgestrichen (siehe oben). Etwa 100 farblose Plaques werden erhalten.

40 dieser Plaques werden benutzt, um jeweils 2 ml *E. coli* JM101 Kultur (siehe Abschnitt (3)) zu inokulieren. Nach der Kultivierung werden die Ueberstände, die Phagen und Einzelstrang-DNA enthalten, durch Zentrifugation von den *E. coli*-Zellen getrennt. Die Zell-Pellets enthalten bereits die entsprechende mutierte doppelsträngige DNA. Jeweils 50 μl der 40 Phagenüberstände werden durch Nitrocellulose gefiltert, zweimal mit TE Puffer gewaschen, im Vakuum 2 Stunden bei 80°C inkubiert und gemäss Southern (1975) auf das Vorhandensein der mutierten DNA-Sequenz geprüft, indem der Oligonukleotid Primer als radioaktive Sonde bei der Hybridisierung verwendet wird. 12 Phagenüberstände, die möglicherweise das Eglin C (Arg45)-Gen enthalten, werden identifiziert. 4 dieser positiven Phagenüberstände werden etwa 1:10$^5$ verdünnt, mit *E. coli* JM101 vermischt und auf Indikator-Agar aufgetragen. Phagen von jeweils 3 der resultierenden Plaques werden isoliert. Daraus wird die Einzelstrang-DNA in der oben beschriebenen Weise isoliert. Diese 12 Einzelstrang-DNAs werden gemäss Sanger (1981 und 1977) sequenziert. Alle 12 Einzelstrang-DNAs enthalten die gewünschte mutierte Eglin C-Sequenz. In einer Minipräparation wird dann die entsprechende Doppelstrang-DNA (Eglin C (Arg45)-Gen in Plasmid M13mp8) aus den entsprechenden *E. coli* Zell-Pellets präpariert (siehe oben). Durch Schneiden mit den Restriktionsendonukleasen EcoRI und BamHI wird der EcoRI-BamHI-Einschub, der das mutierte Gen enthält, aus dem Vektor herausgeschnitten, isoliert und in dem Vektor pHRi148/EcoRI/BamHI kloniert (EP 146 785). Das daraus resultierende Plasmid pJPG18 wird isoliert und damit *E. coli* Stamm HB101 transformiert.

Das 230 Bp EcoRI Fragment, das das mutierte Eglin C (Arg45)-Gen enthält, wird nochmals aus dem Vektor pJPG18 geschnitten und adaptiert, wie für das nicht mutierte Eglin C Gen in Abschnitt (1) beschrieben.

(5) Herstellung des Gens, das Eglin C (Pro44) kodiert: Die Mutation von Thr44 zu Pro44 wird in einer Weise durchgeführt, die analog zu der in Abschnitt (4) beschriebenen ist. Das verwendete mutagene Oligonukleotid hat die folgende Struktur:

$$5'\text{-CT CCT GTT } \overset{*}{C}\text{CT CTG GAC-3'}$$

Die mit dem Stern versehene Base unterscheidet sich von der entsprechenden Base im kodierenden Strang des Eglin C Gens (ein A) ein sorgt letztlich für die Mutation von Thr44 zu Pro44. Wenn die Mutationsmischung aufgearbeitet wird, werden 18 mutmassliche Eglin C (Pro44) Mutanten erhalten. Indem die Eglin C (Pro44) DNA in dem Vektor pHRil148/EcoRI/BamHI kloniert wird, wird das Plasmid pJB591 erhalten und wie beschrieben verwendet.

Beispiel 4: Konstruktion eines vom Ti-Plasmid abgeleiteten Vektors

Beim Vektor pCIB10 (Rothstein et al., 1987) handelt es sich um einen vom Ti-Plasmid abgeleiteten Vektor, der für den Transfer chimärer Gene auf Pflanzen via *Agrobacterium tumefaciens* verwendet werden kann. Der Vektor leitet sich von dem Plasmid pRK252 ab, das einen weiten Wirtsbereich aufweist und das von Dr. W. Barnes, Washington University, St. Louis, Mo. bezogen werden kann. Der Vektor enthält weiterhin ein Gen, welches eine Kanamycinresistenz in *Agrobacterium* vermittelt und das dem Transposon Tn903 stammt, sowie linke und rechte T-DNA Grenzsequenzen aus dem Ti-Plasmid pTiT37. Zwischen den Grenzsequenzen befinden sich eine Polylinkerregion aus dem Plasmid pUC18 und ein chimäres Gen, welches eine Kanamycinresistenz in Pflanzen hervorruft.

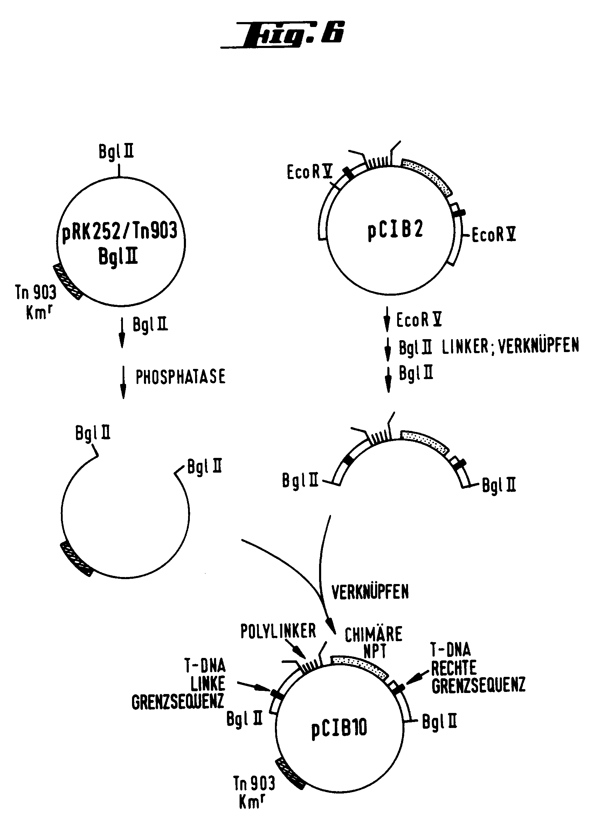

In einem ersten Verfahrensschritt wird das Plasmid pRK252 in der Weise modifiziert, dass das Tetracyclinresistenzgen gegen das Kanamycinresistenzgen aus dem Transposon Tn903 (Oka et al., 1981) ausgetauscht wird. Eine weitere Modifikation betrifft den Austausch der einzigen EcoRI Schnittstelle in pRK252 gegen eine BGIII Schnittstelle (siehe Fig. 6, die einen zusammenfassenden Ueberblick über die oben angegebenen Modifikationen gibt). Das Plasmid pRK252 wird zunächst mit den Endonucleasen SalI und SmaI geschnitten und anschliessend mit der grossen Untereinheit der DNA Polymerase I zur Herstellung glatter Enden behandelt. Das grosse Vektorfragment wird über eine Agarose-Gelelektrophorese gereinigt. Als nächstes wird das Plasmid p368 mit der Endonuklease BamHI geschnitten und mit dem grossen Fragment der DNA Polymerase behandelt. Das etwa 1050 Bp umfassende Fragment wird dann nach Durchführung einer Agarose-Gelelektrophorese isoliert. Dieses Fragment enthält das Gen aus dem Transposon Tn903, das eine Resistenz gegenüber dem Antibiotikum Kanamycin vermittelt (Oka et al., 1981). Plasmid p368 ist bei der ATCC unter der Hinterlegungsnummer 67700 hinterlegt worden. Zur Erzeugung glatter Enden werden beide Fragmente mit der grossen Untereinheit der DNA-Polymerase behandelt. Anschliessend werden beide Fragmente vermischt und über Nacht bei einer Temperatur von 15°C mit T4 DNA-Ligase inkubiert. Nach der Transformation des *E. coli*-Stamms HB101 und einer Selektion Kanamycinresistenter Kolonien erhält man das Plasmid pRK252/Tn903.

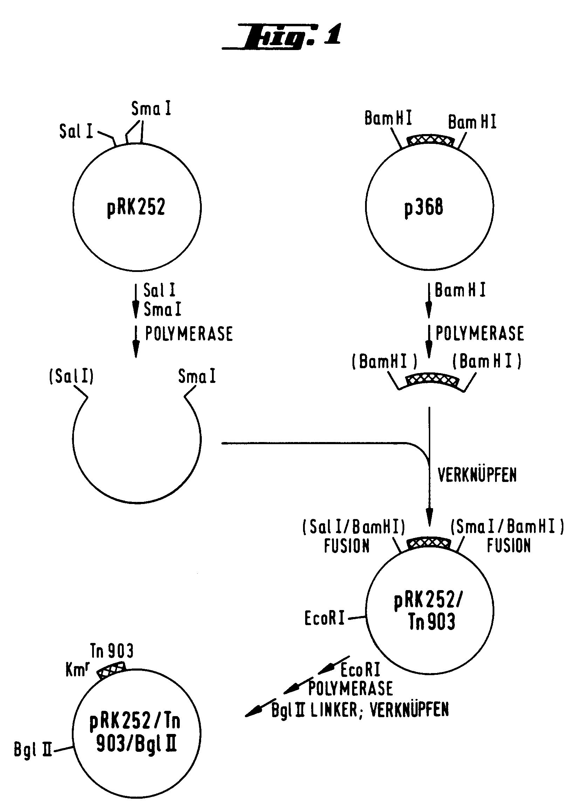

Das so erhaltene Plasmid pRK252/Tn903 wird an seiner einzigen EcoRI Schnittstelle geschnitten und anschliessend zur Herstellung glatter Enden mit der grossen Untereinheit der *E. coli* DNA-Polymerase behandelt. Dieses Fragment wird mit synthetischen Linkern, die BglII Restriktionsschnittstellen enthalten, vermischt und über Nacht mit T4 DNA-Ligase inkubiert. Die aus einer Behandlung resultierende DNA wird mit einem Ueberschuss an Bg1II Restriktionsendonuklease geschnitten und das grössere Vektorfragment mit Hilfe einer Agarose-Gelelektrophorese gereinigt. Das resultierende Fragment wird erneut mit T4 DNA Ligase inkubiert, um das Fragment über seine neu hinzugefügten kohäsiven Bg1II Enden zu rezirkularisieren. Nach Transformation des *E. coli*-Stamms HB101 erhält man das Plasmid pRK252/Tn903/BglII (Fig. 1).

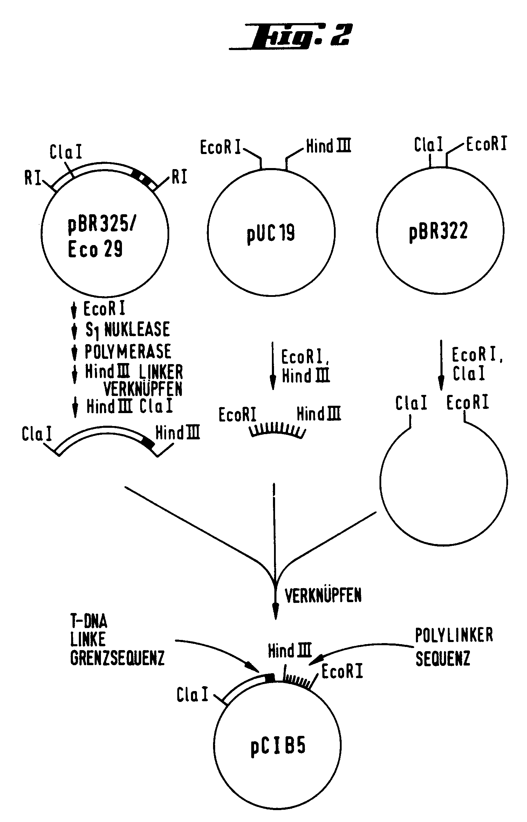

In einem weiteren Verfahrensschritt wird ein Derivat des Plasmids pBR322 konstruiert, das neben den T-DNA Grenzsequenzen aus dem Ti-Plasmid und der Polylinkerregion aus dem Plasmid pUC19 ein selektierbares Gen für Kanamycinresistenz in Pflanzen enthält (Fig. 2). Das Plasmid pBR325/Eco29 enthält das 1.5 kBp umfassende EcoRI Fragment aus dem Nopalin Ti-Plasmid pTiT37. Dieses Fragment enthält die linke T-DNA Grenzsequenz (Yadav et al., 1982). Für den Austausch der EcoRI Enden dieses Fragments durch HindIII Enden wird das Plasmid pBR325/Eco29 mit EcoRI geschnitten und anschliessend mit Nuclease S1 inkubiert. Es folgt eine Inkubation mit dem grossen Fragment der DNA-Polymerase zur Herstellung glatter Enden. Dieser Reaktionsansatz wird mit synthetischen HindIII-Linkern vermischt und mit T4 DNA-Ligase inkubiert. Die resultierende DNA wird mit den Endonukleasen ClaI und einem Ueberschuss an HindIII geschnitten; das resultierende 1.1 kBp umfassende Fragment, das die linke T-DNA Grenzsequenz enthält, wird mit Hilfe einer Gelelektrophorese gereinigt. Als nächstes wird die Polylinkerregion des Plasmids pUC19 isoliert, indem man die Plasmid DNA mit den Endonukleasen EcoRI und HindIII schneidet und das kleinere Fragment (annähernd 53 Bp) über eine Agarose-Gelelektrophorese isoliert. Anschliessend wird das Plasmid pBR322 mit den Endonukleasen EcoRI und ClaI geschnitten, mit den beiden anderen, zuvor isolierten Fragmenten vermischt, mit T4 DNA-Ligase inkubiert und damit der *E. coli* Stamm HB101 transformiert. Das resultierende Plasmid pCIB5, enthält die Polylinkerregion und die linke T-DNA Grenzsequenz integriert in eine Abkömmling des Plasmids pBR322 (Fig. 2).

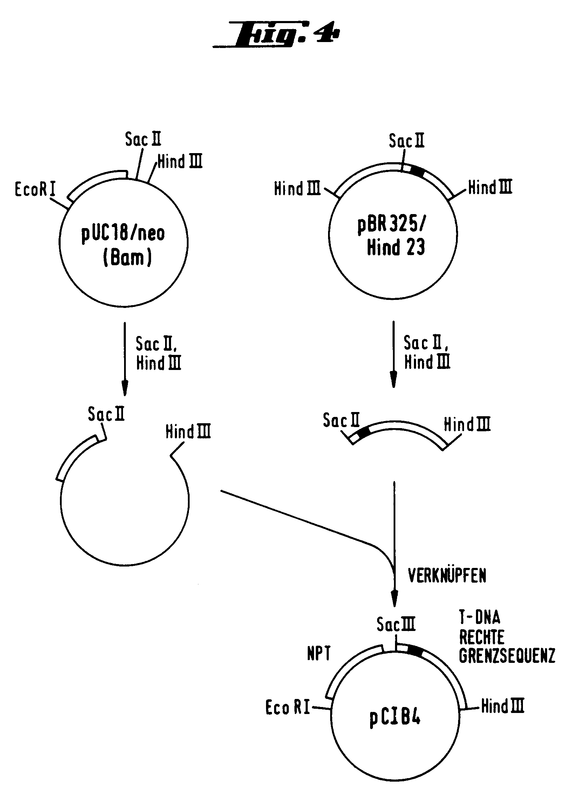

In einem weiteren Verfahrensschritt wird ein Plasmid konstruiert, das ein Gen enthält, welches die Expression einer Kanamycinresistenz in Pflanzen vermittelt (Fig. 4). Das Plasmid Bin 6 ist erhältlich bei Dr. M. Bevan, Plant Breeding Institute, Cambridge, UK. Dieses Plasmid wird ausserdem bei Bevan (1984) beschrieben. Das Plasmid Bin 6 wird mit EcoRI und HindIII geschnitten. Das etwa 1.5 kBp umfassende Fragment, welches das chimäre Neomycinphosphotransferase (NPT) Gen enthält, wird isoliert und anschliessend über eine Agarosegelelektrophorese gereinigt. Das Fragment wird mit pUC18 Plasmid DNA vermischt, die zuvor mit den Endonukleasen EcoRI und HindIII geschnitten wurde. Nach Inkubation mit T4 DNA-Ligase wird mit der resultierenden DNA der *E. coli* Stamm HB101 transformiert. Das entstehende Plasmid wird als pUC18/neo bezeichnet. Dieses Plasmid enthält eine unerwünschte BamHI-Schnittstelle zwischen dem Neomycinphosphotransferase-Gen und der Terminator-Sequenz des Nopalinsynthase-Gens (siehe Bevan, 1984). Um diese Erkennungssequenz zu entfernen, wird das Plasmid pUC18/neo mit der Endonuklease BamHI geschnitten, gefolgt von einer Behandlung mit der grossen Untereinheit der DNA-Polymerase zur Erzeugung glatter Enden. Um das Fragment zu rezirkularisieren, wird es anschliessend mit T4 DNA-Ligase inkubiert. Mit diesem Fragment wird der *E. coli* Stamm HB101 transformiert. Das resultierende Plasmid, pUC18/neo(Bam) besitzt keine BamHI-Erkennungssequenz mehr.

In einem weiteren Verfahrensschritt wird die rechte T-DNA Grenzsequenz unmittelbar neben das chimäre NPT Gen inseriert (Fig. 4). Das Plasmid pBR325/Hind23 enthält das 3.4 kBp HindIII-Fragment des Plasmids pTiT37. Dieses Fragment besitzt die rechte T-DNA Grenzsequenz (Bevan et al., 1983). Das Plasmid pBR325/Hind23 wird mit den Endonukleasen SacII und HindIII geschnitten und ein 1.9 kBp umfassendes Fragment, welches die rechte Grenzsequenz enthält, im Anschluss an eine Agarosegelelektrophorese in gereinigter Form isoliert. Das Plasmid pUC18/neo(Bam) wird mit den Endonukleasen SacII und HindIII geschnitten und das 4.0 kBp umfassende Vektorfragment mit Hilfe einer Agarosegelelektrophorese isoliert. Die beiden Fragmente werden miteinander vermischt, mit T4 DNA Ligase inkubiert und damit der *E. coli* Stamm HB101 transformiert. Das resultierende Plasmid pCIB4 (Fig. 4) enthält die rechte T-DNA Grenzsequenz sowie den in Pflanzen selektierbaren Marker für Kanamycinresistenz in einem Abkömmling des Plasmids pUC18.

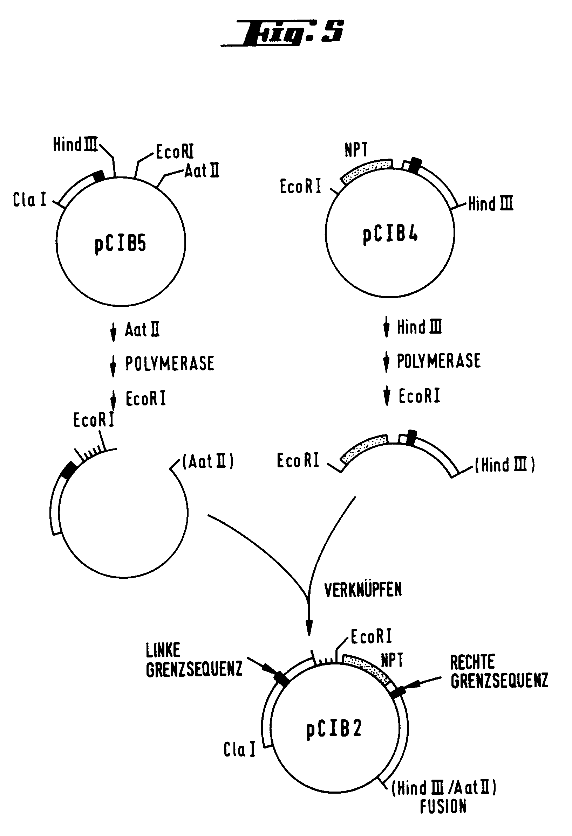

In einem letzten Verfahrensschritt wird in Plasmid konstruiert, das sowohl die linke als auch die rechte T-DNA Grenzsequenz und zwischen diesen Grenzsequenzen das in Pflanzen selektierbare Kanamycinresistenzgen und den Polylinker des Plasmids pUC18 enthält (Fig. 5). Zunächst wird das Plasmid pCIB4 mit der Endonuklease HindIII geschnitten, gefolgt von einer Behandlung mit der grossen Untereinheit der DNA Polymerase zur Herstellung glatter Enden sowie einer Fragmentierung mit der Endonuklease EcoRI. Das 2.6 kBp umfassende Fragment, welches das chimäre Kanamycinresistenzgen und die rechte T-DNA Grenzsequenz enthält, wird mit Hilfe einer Agarosegelelektrophorese isoliert. Anschliessend wird das Plasmid pCIB5 mit der Endonuklease AatII geschnitten, zur Erzeugung glatter Enden mit T4 DNA-Polymerase behandelt und dann mit der Endonuklease EcoRI geschnitten. Das grössere Vektorfragment wird mit Hilfe einer Agarosegelelektrophorese gereinigt, mit dem pCIB4-Fragment vermischt und mit T4 DNA-Ligase inkubiert. Mit diesem Fragment wird der *E. coli* Stamm HB101 transformiert. Das resultierende Plasmid pCIB2 (Fig. 5) ist ein Derivat des Plasmids pBR322, das die gewünschten Sequenzen zwischen den beiden T-DNA Grenzsequenzen enthält.

Die folgenden Schritte komplettieren die Konstruktion des Vektors pCIB10. Sie sind in Fig. 6 dargestellt. Das Plasmid pCIB2 wird mit der Endonuklease EcoRV geschnitten und, wie zuvor beschrieben, mit synthetischen Linkern, die eine BglII Erkennungsstelle besitzen, versehen. Nach Schneiden mit einem Ueberschuss an BglII wird das annähernd 2.6 kBp umfassende Fragment mit Hilfe einer Agarosegelelektrophorese isoliert. Das zuvor bereits beschriebene Plasmid pRK252/Tn903/BglII (Fig. 1) wird mit der Endonuklease BglII geschnitten und anschliessend mit Phosphatase behandelt, um eine Rezirkularisierung zu verhindern. Diese beiden DNA Fragmente werden miteinander vermischt und mit T4 DNA-Ligase inkubiert. Anschliessend wird *E. coli* Stamm HB101 transformiert. Das resultierende Plasmid ist der vervollständigte Vektor pCIB10.

Beispiel 5: Konstruktion eines chimären Gens mit dem CaMV 35S Promotor

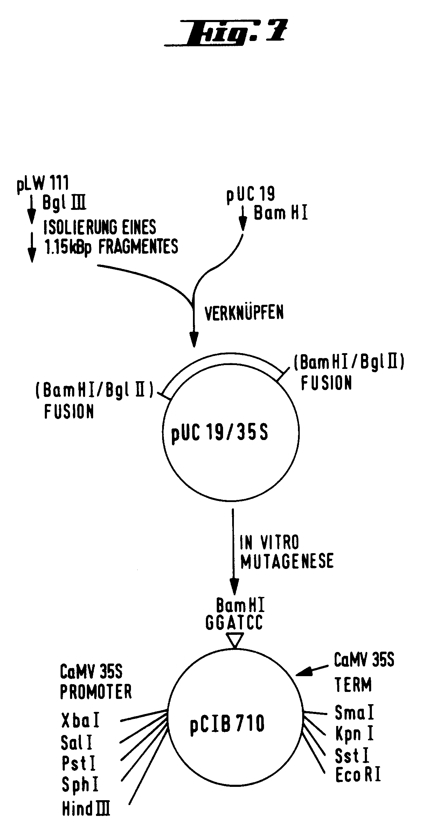

A. Konstruktion eines CaMV 35S Promotor-Kassette-Plasmids: pCIB710 wird wie in Fig. 7 gezeigt konstruiert. Dieses Plasmid enthält CaMV Promotor-und Transkriptionsterminationssequenzen für das 35S RNA-Transkript (Covey et al., 1981). Ein 1149 Bp BglII-Restriktionsfragment der CaMV-DNA (Bp 6494 bis 7643; Hohn et al., 1982) wird von dem Plasmid pLW111, das am 14. Mai 1986 bei der American Type Culture Collection unter der Hinterlegungsnummer ATCC 40235 hinterlegt wurde, isoliert; alternativ dazu kann das Fragment durch präparative Agarose-Gelelektrophorese direkt isoliert werden. Es wird mit BamHI-geschnittener pUC19 Plasmid-DNA vermischt, mit T4 DNA-Ligase behandelt und damit *E. coli* transformiert (Beachte, dass die BamHI-Restriktionsschnittstelle im resultierenden Plasmid durch Verknüpfen der BglII kohäsiven Enden mit den BamHI kohäsiven Enden zerstört wird). Das resultierende Plasmid, genannt pUC19/35S, wird anschliessend in einer Oligonukleotid-vermittelten in vitro Mutagenese dazu verwendet, die BamHI-Erkennungssequenz GGATCC direkt im Anschluss an das CaMV Nukleotid 7483 (gemäss der Bezeichnung in Hohn et al., 1982) einzufügen. Das resultierende Plasmid pCIB710 enthält die CaMV 35S Promoter- und Transkriptionsterminationsregion, getrennt durch eine BamHI-Restriktionsschnittstelle. In diese BamHI-Schnittstelle eingefügte DNA-Sequenzen werden in Pflanzen durch diese CaMV Transkriptions-Regulationssequenzen exprimiert (Beachte auch, dass pCIB710 keine ATG Translationsinitiationskodons zwischen dem Start der Transkription und der BamHI-Schnittstelle enthält).

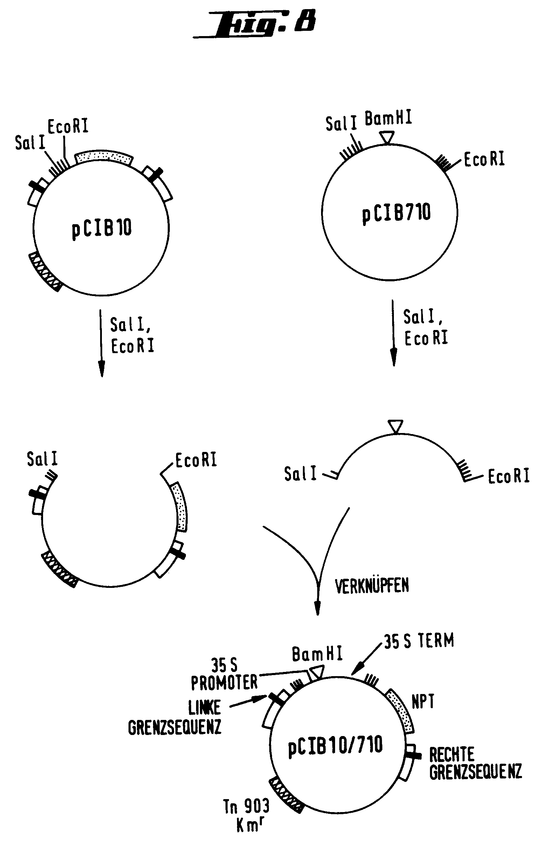

B. Einfügen der CaMV 35S Promoter/Terminatorkassette in CIB10. Die folgenden Schritte sind in Fig. 8 dargestellt. Die pCIB10- und pCIB710-Plasmid-DNA wird mit EcoRI und SalI geschnitten, vermischt und verknüpft. Das resultierende Plasmid pCIB10/710 enthält die CaMV 35S Promotor-/Terminatorkassette, eingefügt in den Pflanzentransformationsvektor pCIB10. Die CaMV 35S-Sequenzen befinden sich zwischen den T-DNA-Grenzsequenzen in pCIB10 und werden so bei Pflanzentransformationsexperimenten in das pflanzliche Genom eingefügt.

C. Transfer in *Agrobacterium.* Für den Transfer des binären von pCIB10 abgeleiteten Plasmids von *E. coli* HB101 auf *Agrobacterium* wird ein intermediärer *E. coli*-Wirt verwendet und zwar der *E. coli* Stamm S17-1. Dieser *E. coli* Stamm, der von Agrigenetics Research Co., Boulder Co., bezogen werden kann, wird in Simon et al. (1983a) beschrieben. Er enthält Mobilisierungsfunktionen, die einen direkten Transfer des Plasmids pCIB10 auf *Agrobacterium* via einer Konjugation erlauben. Damit kann die Notwendigkeit eines direkten Transfers nackter Plasmid DNA in *Agrobacterium* umgangen werden. Zunächst wird pCIB10 Plasmid DNA in mit Kalziumchlorid behandelte S17-1 Zellen eingeschleust. Danach werden Kulturen transformierter S17-1 Zellen mit *A. tumefaciens* LBA4404 (Ooms et al., 1981) vermischt und auf N Agar (Difco) Platten über Nacht bei Raumtemperatur gepaart. Von den resultierenden Bakterien wird eine Probe entnommen und auf AB Minimalmedium, das 50 μg/ml Kanamycin enthält, überimpft (Ditta et al., 1980) und ausplattiert. Die Inkubation erfolgt bei 28°C. Die gewachsenen Kolonien werden auf demselben Medium ein zweites Mal und anschliessend ein weiteres Mal auf N Agarplatten ausgestrichen. Langsam wachsende Kolonien werden auf einem AB Minimalmedium mit Kanamycin ausgestrichen und einzelne Kolonien isoliert. Nach diesem Verfahren werden Agrobakterien isoliert, die das Plasmid pCIB10 enthalten.

### III. Transformation und Regenerierung

Beispiel 6: Transformation von Tabak

Protoplasten von *Nicotiana tabacum* cv. "Coker 176" werden wie folgt hergestellt: Vier bis fünf Wochen alte Sprosskulturen werden unter aseptischen Bedingungen in einem MS Medium ohne Hormone bei einer Temperatur von 16°C und einer Photoperiode von 16 Stunden Licht/8 Stunden Dunkelheit herangezogen. Ca. 1,5 g Blattgewebe werden von der Pflanze entnommen und gleichmässig auf 8 bis 10 Petrischalen (100 x 25 mm, Lab-Tek), die jeweils 10 ml einer Enzymlösung enthalten, verteilt. Die Enzymlösung enthält 1 % Cellulase R-10, 0,25 % Macerase, von Calbiochem Ca., 1 % Pectolyase Y-23, von Seishin Pharmaceuticals Co., 0,45 M Mannit und 0,1 x K3 Salze (Nagy und Maliga, 1976). Die Tabakblätter werden mit Hilfe eines Skalpells in dünne Streifen geschnitten. Die Petrischalen werden anschliessend verschlossen und auf einer Rundschüttelmaschine bei einer Umdrehungsgeschwindigkeit von 35 Upm und Raumtemperatur für einen Zeitraum von 4 bis 5 Stunden mit den Enzymen inkubiert.

Anschliessend wird der Inhalt der Petrischalen durch einen mit einem feinmaschigen Gewebe (Mull) ausgelegten Trichter filtriert und in einem Auffanggefäss gesammelt. Das Filtrat wird dann in "Babcock" Flaschen pipettiert, die jeweils 35 ml einer Waschlösung enthalten (Die Waschlösung enthält: 0,45 M Saccharose, 0,1 % MES sowie 0.1 x K3 Salze). Die Flaschen werden 10 Minuten bei 80 x g zentrifugiert, wodurch die Protoplasten an der Oberfläche der Flaschen angereichert werden. Die Protoplasten werden mit Hilfe einer 1 ml Pipette entnommen, in einer Flasche gesammelt und zwei weitere Male gewaschen. Die resultierenden Protoplasten werden in K3 Medium in einem 15 ml Einmalzentrifugenröhrchen suspendiert. Die Konzentration der Protoplasten wird durch Auszählen in einem Fuchs-Rosenthal Hämocytometer bestimmt. Die Protoplasten werden dann in Petrischalen (100 x 20 mm, Corning), die 6 ml eines flüssigen K3 Mediums enthalten, in einer Dichte von 100 000/ml ausplattiert. Die Petrischalen mit den Protoplasten werden anschliessend zwei Tage lang bei einer Temperatur von 26°C im Dunkeln inkubiert. Während dieser Zeit erfolgt die Regenerierung der Zellwand.

Nach Beendigung der zweitätigen Inkubation werden 5 µl einer stationären *A. tumefaciens* Kultur (gewaschen in einem YEB-Medium), das 50 µg/ml Kanamycin als Zusatz enthält, bei einer Temperatur von 28°C bis die stationäre Phase erreicht ist), die die gewünschten Plasmide enthalten, zu den Protoplasten gegeben. Nach einer Inkubationszeit von drei weiteren Tagen bei 26°C wird Cefotaxim (Calbiochem) hinzugegeben bis eine Endkonzentration von 500 µg/ml erreicht ist, um die Agrobakterien abzutöten. Am darauffolgenden Tag werden die Zellen mit 3 ml frischem K3 Medium pro Petrischale verdünnt. Anschliessend wird erneut Cefotaxim bis zu einer Endkonzentration von 500 µg/ml zugegeben. Die Zellen werden dann bei einer Temperatur von 26°C 2 bis 3 Wochen lang kultiviert und anschliessend auf Selektivmedien gescreent wie bei de Block et al. (1984) beschrieben.

Beispiel 7: Regenerierung ganzer Baumwollpflanzen ausgehend von Keimblatt-Explantaten

A. Baumwoll-Varietät Acala SJ2 in einem Medium, das einen gleichmässigen Kallus induziert. Samen von *Gossypium hirsutum* (Baumwolle) Varietät Acala SJ2 werden drei Minuten mit 95 % Alkohol sterilisiert, zweimal mit sterilem Wasser gewaschen, anschliessend für die Dauer von 15 Minuten in eine 15 %ige Natriumhypochlorit-Lösung eingebracht und erneut in sterilem Wasser gewaschen. Zur Herstellung von Keimlingen werden die so sterilisierten Samen für eine Zeitraum von etwa 14 Tagen im Dunkeln auf einem der gebräuchlichen Agar-Medien zur Keimung gebracht. Die Keimblätter dieser Keimlinge werden in Segmente zerschnitten, die anschliessend unter sterilen Bedingungen auf ein Kallus-induzierendes Medium transferiert werden, das sich aus den Makro- und Mikrosalzen des Murashige und Skoog Medium (MS) zusammensetzt und zusätzlich Thiamin HCl (0,4 mg/Liter), Glucose (30 mg/Liter), NAA (2 mg/Liter), Kinetin (1 mg/Liter), myo-Inosit (100 mg/Liter) sowie Agar (0.8 %) enthält. Die Kulturen werden bei etwa 30°C mit einer Photoperiode von 16 Stunden Licht/8 Stunden Dunkelheit in einem "Percival" Inkubator inkubiert. Die Beleuchtung erfolgt mit Hilfe von fluoreszierendem Licht (kaltes Tageslicht), bei einer Beleuchtungsstärke von etwa 2000 bis 4000 1x. An den kultivierten Gewebefragmenten entwickeln sich innerhalb von drei bis vier Wochen Kalli, die eine weisse bis grau-grüne Färbung aufweisen. Die gebildeten Kalli werden alle drei bis vier Wochen auf ein Kallus-Wachstumsmedium überimpft, das myo-Inosit (100 mg/Liter), Saccharose (2,0 g/Liter), NAA (2 mg/Liter) und Agar enthält, und dort erneut kultiviert. Vier bis sechs Monate nach dem Transfer der Gewebeexplantate auf das Kallus-induzierende Medium erfolgt die Ausbildung somatischer Embryonen. Der Kallus und die Embryonen werden durch drei- bis vierwöchiges Ueberimpfen auf frisches Kallus-Wachstumsmedium und erneutes Kultivieren am Leben erhalten.

Somatische Embryonen, die sich an Gewebestücken entwickeln, werden entweder auf frisches Kallus-Wachstumsmedium überimpft oder aber auf ein spezifisches Keimungsmedium für Embryonen (Beasley und Ting, 1973) übertragen. Die Pflänzchen, die sich aus den somatischen Embryonen entwickeln, werden auf das Beasley und Ting Medium transferiert, das als Zusatz Ammoniumnitrat (15 mg/Liter) sowie Caseinhydrolysat (15 mg/Liter) als organische Stickstoffquelle enthält. Das Medium wird durch ein Verfestigungsmittel (Gelrite$^®$) stabilisiert und die Pflänzchen werden in Töpfe übertragen. Die somatischen Embryonen entwickeln sich innerhalb von etwa drei Monaten zu Pflänzchen. Die Pflänzchen sind mit Erreichen des 6-bis 8-Blatt-Stadiums (die Grösse der Pflanzen beträgt dann zwischen 7,5 und 10 cm) bewurzelt und werden in Erde übertragen. Die Kultivierung erfolgt für einen Zeitraum von drei bis vier Wochen in einem Inkubator bei hoher Luftfeuchtigkeit. Danach werden die Pflänzchen ins Gewächshaus transferiert. Wenn die Pflänzchen abgehärtet sind, werden sie in offene, bearbeitete Ackererde ausgepflanzt.

B. Baumwoll-Varietät Acala S72 in auf die Hälfte verdünntem Kallusinduzierenden Medium. Das Verfahren von A wird wiederholt, wobei in diesem Fall ein halbkonzentriertes MS-Medium verwendet wird, in dem alle Medienbestandteile in ihrer angegebenen Konzentration auf die Hälfte reduziert sind. Man erhält im wesentlichen die gleichen Resultate wie bei Verwendung des voll-konzentrierten MS-Mediums.

C. Verschiedene Baumwoll-Varietäten. Die in A und B beschriebenen Verfahrensmassnahmen werden unter Verwendung der Acala Baumwoll-Varietäten SJ4, SJ2C-1, GC510, B1644, B2724, B1810, der "Picker" Varietät Siokra und der "Stripper" Varietät FC2017 durchgeführt. Alle aufgezählten Varietäten können erfolgreich regeneriert werden.

D. Regenerierung von Baumwollpflanzen ausgehend von Keimblattexplantaten unter Zwischenschaltung einer Zell-Suspensionskultur. Die in A beschriebenen Verfahrensmassnahmen werden bis zur Herstellung eines Kallus, der zur Ausbildung somatischer Embryonen befähigt ist, wiederholt. Stücke von 750 mg bis 1000 mg des aktiv wachsenden embryogenen Kallus werden in 8 ml Aliquots eines flüssigen Suspensionskultur-Mediums, das sich aus den Makro- und Mikro-Salzen des MS-Mediums zusammensetzt und darüberhinaus Thiamin HCl (0,4 mg/Liter), Saccharose (20 g/Liter), myo-Inosit (100 mg/Liter) und NAA (2 mg/Liter) als Zusatz enthält, in Schalen suspendiert und anschliessend auf einer rotierenden Trommel bei einer Umdrehungsgeschwindigkeit von 1,5 Upm und einem Licht/Dunkel Rhythmus von 16 Stunden/8 Stunden inkubiert. Das Licht stammt wiederum aus fluoreszierenden Lampen (kaltes Tageslicht) und hat eine Stärke zwischen 2000 und 4500 1x. Nach vier Wochen wird die Suspensionskultur durch ein Nylonnetz mit einer Maschenweite von 840 μm filtriert, um grössere Zellklumpen zu entfernen. Die Fraktion, die nur Partikel mit einer Grösse von unter 840 μm umfasst, lässt man absetzen und wäscht anschliessend einmal mit 20 bis 25 ml eines frischen Suspensionskultur-Mediums. Diese Zellsuspension wird in Schalen überführt (2 ml pro Schale) und jede Schale wird mit 6 ml frischem Suspensionskultur-Medium verdünnt. Die Kulturen werden durch Wiederholung der oben beschriebenen Verfahrensschritte in Intervallen von 10 bis 12 Tagen am Leben erhalten. Bei jeder Wiederholung wird die Suspensionskultur erneut durch das 840 μm Netz filtriert und nur diejenige Fraktion, die Zellaggregate kleiner als 840 μm enthält, wird auf frisches Suspensionskultur-Medium überführt. In allen Fällen wird die Fraktion, die Zellklumpen von mehr als 840 μm aufweist, auf ein Kallus-Wachstumsmedium gegeben, um auf diese Weise somatische Embryonen zu erhalten. Die somatischen Embryonen, die sich auf dem Kallus-Wachstumsmedium entwickeln, werden entfernt und auf ein Keimungsmedium für Embryonen übertragen. Unter Anwendung der in A beschriebenen Versuchsbeschreibung findet zunächst die Keimung der Embryonen statt, die sich dann zu Pflänzchen und anschliessend zu im Freiland wachsenden Pflanzen entwickeln.

E. Alternative zwischengeschaltete Suspensionskultur. Die in D beschriebenen Verfahrensschritte werden wiederholt bis darauf, dass in diesem Fall 750 bis 1000 mg embryogenen Kallusgewebes in eine DeLong Flasche übertragen werden, die 15 bis 20 ml flüssiges MS-Medium mit 2 mg/Liter NAA enthält. Die Flasche mit der Kultur wird auf einer Rundschüttelmaschine bei einer Umdrehungsgeschwindigkeit von 100 bis 110 Upm inkubiert. Nach drei Wochen wird die Suspensionskultur durch ein Nylonnetz mit einer Maschenweite von 840 μm filtriert, um auf diese Weise die grossen Zellklumpen für die Entwicklung ganzer Pflanzen zu gewinnen, wie in D beschrieben. Diejenige Fraktion, die Zellaggregate mit einer Grösse von weniger als 840 μm aufweist, lässt man zunächst absetzen, wäscht diese anschliessend einmal in einem flüssigen MS-Medium und resuspendiert dann in 2 ml bis 5 ml eines flüssigen MS-Mediums. Die Zellsuspension wird erneut kultiviert und zwar durch Uebertragung von 1 ml bis 2 ml der Suspension in eine DeLong Flasche, die 15 ml frisches MS-Medium enthält. Die Kulturen werden durch Wiederholen der oben beschriebenen Verfahrensschritte in Intervallen von sieben bis zehn Tagen am Leben erhalten. Für jede erneute Kultivierung werden lediglich diejenigen Suspensionen verwendet, die Zellaggregate mit einer Grösse von weniger als 840 μm aufweisen. Die grösseren Zellklumpen (840 μm und grösser) werden für die Entwicklung ganzer Pflanzen verwendet.

F. Herstellung von Pflanzen aus grossen Klumpen von Zellen, die in Suspensionen kultiviert werden. Nach drei- bis viermaliger Wiederholung der Kultivierung unter Anwendung der in D und E beschriebenen Versuchsbeschreibungen, werden 1,5 ml bis 2 ml der Zellsuspension von den Schalen bzw. den DeLong Flaschen entnommen und auf mit Agar verfestigtes MS-Medium, das 2 mg/Liter NAA enthält, sowie Beasley und Ting Medium, das 500 mg/Liter Caseinhydrolysat enthält, übertragen. Nach drei bis vier Wochen werden embryogene Kalli mit sich entwickelnden Embryonen sichtbar. Auch in diesem Fall werden Zellklumpen mit einer Grösse von 840 μm und mehr auf ein Kallus-Wachstumsmedium übertragen. Aus diesen Zellklumpen erhält man embryogene Klumpen mit sich entwickelnden Embryonen, die letztendlich zu Pflanzen heranwachsen.

Beispiel 8: Transformation von Baumwoll-Suspensionskulturzellen zu einem Tumor-Phänotyp mit Hilfe von *Agrobacterium* LBA 4434

A. Wachstum der pflanzlichen Suspensionskultur. Eine Acala Baumwoll-Suspensionskultur wird in Schalen kultiviert, wobei das Medium (MS-Medium mit 2 g/Liter NAA) alle sieben bis zehn Tage gewechselt wird. Nach dem Mediumwechsel wird die Schale jeweils um 90° gedreht, und man lässt die Zellen sich absetzen. Vor der Transformation wird der Ueberstand mit Hilfe einer Pipette abgehoben und die resultierenden Zellen werden die zuvor beschrieben behandelt.

B. Beschreibung des *Agrobacterium* Vektors. Der *Agrobacterium* Stamm LBA 4434 (Hoekema et al., 1983) enthält ein binäres, auf dem Ti-Plasmid basierendes Pflanzen-Transformationssystem. In einem solchen binären Transformationssystem enthält eines der Plasmide die T-DNA Region des Ti-Plasmids, während das zweite Plasmid die vir-Region des Ti-Plasmids aufweist. Die Transformation der Pflanze wird durch das Zusammenwirken beider Plasmide gewährleistet. Im *Agrobacterium* Stamm LBA 4434 enthält das T-DNA Plasmid pAL1050 die $T_L$ Sequenz von pTiAch5, einem Octopin-Ti-Plasmid. Das vir-Plasmid im Stamm LBA 4434, pAL4404, enthält dagegen die intakte Virulenzregion von pTiAch5 (Ooms et al., 1982). Der *Agrobacterium* Stamm 4434 kann von Dr. Robert Schilperoort, Abteilung für Biochemie, Universität Leiden, Niederlande, bezogen werden.

C. Kultivierung der Agrobakterien; Wachstumsbedingungen. Der transformierende *Agrobacterium* Stamm wird einer Glycerin-Stammkultur entnommen und kultiviert. Am nächsten Tag wird aus dieser Uebernachtkultur ein Aliquot entnommen und damit eine 50 ml-Kultur angeimpft. Die Agrobakterien werden auf YEB Medium kultiviert, dem Antibiotika angemessen zugefügt wurden. Man bestimmt die optische Dichte der 50 ml Uebernachtkultur bei 600 nm, zentrifugiert die Kultur und resuspendiert das Pellet in einem Wachstumsmedium für Pflanzenzellen (MS-Medium, das 2 mg/ml NAA enthält) bis eine $OD_{600}$ von 0,5 erreicht ist. 8 ml dieser bakteriellen Suspension wird zu jeder Schale, die die pflanzlichen Zellen gemäss Abschnitt A enthält, gegeben.

D. Infektion. Die Schalen mit den Pflanzen- und Bakterienzellen werden zunächst geschüttelt, um alle Zellen zu resuspendieren, und anschliessend erneut für einen Zeitraum von 3 Stunden auf einer Schüttelvorrichtung inkubiert, um den Agrobakterien die Möglichkeit zu geben, sich an die Pflanzenzellen anzuheften. Man lässt dann die Zellen absetzen und entfernt den restlichen Ueberstand. Anschliessend wird ein Aliquot frisches Wachstumsmedium zu den Schalen gegeben und 18 bis 20 weitere Stunden in Gegenwart eventuell noch vorhandener Agrobakterien auf einer Schüttelvorrichtung inkubiert. Danach lässt man die Zellen erneut absetzen, entfernt den Ueberstand und wäscht die Zellen zweimal mit einer Lösung aus Wachstumsmedium und Cefotaxim (200 μg/ml). Nach dem Waschen werden die Zellen aus den einzelnen Schalen in jeweils 10 ml Wachstumsmedium mit Cefotaxim (jeweils 200 μg/ml) resuspendiert, und jeweils 1 ml Aliquots davon werden auf Petrischalen ausplattiert.

E. Kultivierung des transformierten Gewebes. Die mit Agrobakterien infizierten Zellen wachsen auf dem Wachstumsmedium, das keine Hormonzusätze enthält, und zeigen dadurch, dass das Gewebe die Wild-Typ Phytohormongene in der T-DNA erhielt. Die entsprechenden Zellen entwickeln sich zu Tumoren und bieten damit ein weiteres Indiz für die erfolgreiche Transformation der Kulturen.

Beispiel 9: Transformation von Baumwoll-Suspensionskulturzellen zu einem Kanamycin-resistenten, nicht-tumorösen Phänotyp

Es werden die gleichen Verfahrensmassnahmen, die zuvor in Beispiel 8 beschrieben wurden, angewendet, abgesehen davon, dass andere transformierende Agrobacterien verwendet werden und dass das Pflanzen-Selektionsmedium ein Antibiotikum für die Selektionierung transformierter pflanzlicher Gewebe enthält.

A. Kultivierung des pflanzlichen Gewebes. Die Kultivierung des pflanzlichen Gewebes erfolgt analog der in Beispiel 8, Abschnitt A angegebenen Verfahrensmassnahmen.

B. Beschreibung des *Agrobacterium*-Vektors. Die transformierenden Agrobakterien besitzen des T-DNA-enthaltenden binären Vektor pCIB10 (Rothstein et al., 1987) sowie das vir-Plasmid pAL4404. Die T-DNA von pCIB10 enthält ein chimäres Gen, das aus dem Promotor des Nopalinsynthasegens, der kodierenden Region von Tn5 (die das Enzym Neomycinphosphotransferase kodiert) und dem Terminator des Nopalinsynthasegens zusammengesetzt ist.

C. Kultivierung der Agrobacterien, Wachstumsbedingungen. Agrobakterien mit dem Plasmid pCIB10 werden auf YEB Medium mit 50 µg/ml Kanamycin kultiviert. Die übrigen Kultivierungsbedingungen entsprechen denjenigen, die in Beispiel 8, Abschnitt C beschrieben sind.

D. Infektion. Die Transformation pflanzlicher Zellen erfolgt gemäss den in Beispiel 8 beschriebenen Verfahrensmassnahmen. In diesem Fall werden jedoch die aus Abschnitt C resultierenden Aliquots (1 ml) direkt auf mit selektiven Antibiotika angereicherten Medien ausplattiert. Das Selektionsmedium enthält entweder Kanamycin (50 µg/ml) oder G418 (25 µg/ml). Die Expression des chimären nos/neo/nos Gens im transformierten pflanzlichen Gewebe erlaubt die Selektion dieses Gewebes auf eines der zuvor genannten Antibiotika.

E. Kultivierung des transformierten Gewebes. In diesem und in allen folgenden Beispielen enthalten die Pflanzen-Wachstumsmedien Phytohormone gemäss den in Beispiel 7 gemachten Angaben.
Nach 2 bis 4 Wochen wird das transformierte Gewebe auf den Selektionsplatten sichtbar. Nicht-infiziertes sowie Kontrollgewebe zeigt keine Anzeichen von Wachstum. Es wird schliesslich braun und stirbt ab. Transformiertes Gewebe wächst dagegen auch in Gegenwart von Kanamycin und G418 sehr gut. Zu diesem Zeitpunkt werden gut wachsende Gewebestücke auf frisches Selektionsmedium überimpft und dort weiter kultiviert.

F. Kultivierung somatischer Embryonen. Aus den Gewebestücken bilden sich somatische Embryonen aus. Sie werden auf frisches Medium überimpft (nicht-selektives Medium).

G. Keimung. Wenn die Embryonen beginnen, auszudifferenzieren und zu keimen, d.h. zu dem Zeitpunkt, da sie mit der Bildung von Wurzeln beginnen und 2 bis 3 Blätter aufweisen, werden sie in Behälter mit Wachstumsmedium übertragen. Die Pflänzchen werden noch bis zum 6 bis 8 Blattstadium weiterkultiviert, bevor sie von dem Agar-Medium entfernt werden.

H. Kultivierung der Pflänzchen. Die Pflänzchen werden in Topferde gepflanzt und zur Aufrechterhaltung einer hohen Luftfeuchtigkeit mit einem Becherglas abgedeckt und in einem Inkubator 4 bis 8 Wochen lang kultiviert. Zu diesem Zeitpunkt wird das Becherglas entfernt und die Pflanzen werden ins Gewächshaus gebracht. Die Pflanzen wachsen im Gewächshaus heran, entwickeln Blüten und bilden schliesslich Samen.

Beispiel 10: Transformation von Zellen einer Baumwoll Suspensionskultur zu einem Hygromycin-resistenten, nicht-tumorösen Phänotyp

Sofern im folgenden keine anderslautenden Angaben gemacht werden, erfolgt die Durchführung der Experimente gemäss den Angaben in Beispiel 9. Im Unterschied zu Beispiel 8 werden in diesem Fall andere transformierende Agrobakterien verwendet. Darüberhinaus enthält das Pflanzenselektionsmedium ein Antibiotikum, das für die Selektion von transformiertem pflanzlichen Gewebe geeignet ist.

A. Kultivierung des pflanzlichen Gewebes. Die Kultivierung des pflanzlichen Gewebes erfolgt analog der in Beispiel 8, Abschnitt A angegebenen Verfahrensmassnahmen.

B. Beschreibung des *Agrobacterium*-Vektors. Die transformierenden Agrobakterien enthalten den binären T-DNA Vektor pCIB715 (Rothstein et al., 1987) sowie das vir-Plasmid. Die T-DNA von pCIB715 enthält ein chimäres Gen, das aus dem Promotor und dem Terminator des 35S Transkripts von CaMV (Odell et al., 1985) sowie der kodierenden Sequenz für die Hygromycin-B-Phosphotransferase (Gritz und Davies, 1983) zusammengesetzt ist.

C. Kultivierung der Agrobakterien. Die Agrobakterien, welche das Plasmid pCIB715 enthalten, werden auf YEB Medium, das 50 µg/ml Kanamycin enthält, kultiviert.

D. Infektion. Die Transformation wird gemäss den in Beispiel 8 im Einzelnen ausgeführten Verfahrensschritten durchgeführt. In diesem Fall werden jedoch die in Abschnitt (c) resultierenden Aliquots (je 1 ml) direkt auf die Medien mit den selektiven Antibiotika ausplattiert. Das Selektionsmedium enthält 50 μg/ml Hygromycin. Die Expression des chimären Hygromycin-Gens in transformiertem pflanzlichen Gewebe erlaubt die Selektion dieses Gewebes auf Hygromycin-haltigen Medien.

E. Kultivierung des transformierten Gewebes. Die Kultivierung erfolgt analog der in Beispiel 9, Abschnitt E beschriebenen Verfahrensmassnahmen. Das in dem Pflanzen-Selektionsmedium verwendete Antibiotikum ist in diesem Fall Hygromycin.

Beispiel 11: Transformation von Pflanzenzellen durch Beschuss mit Mikroprojektilen

Ein anderes Verfahren, fremde DNA-Sequenzen in Pflanzenzellen einzuführen, umfasst das Anheften besagter DNA an Partikel, die anschliessend den Pflanzenzellen mit Hilfe eines Apparates, wie er von Klein et al. (1988) beschrieben wird, eingeschossen werden. Jedes pflanzliche Gewebe oder pflanzliche Organ kann als Ziel dieser Prozedur verwendet werden, einschliesslich, aber nicht darauf beschränkt, Embryonen, apikale und andere Meristeme, Knospen, somatische und sexuelle Gewebe in vivo und in vitro. Transgene Zellen und Kallus werden gemäss etablierter Verfahren, die in der Fachwelt bekannt sind, ausgewählt. Getroffene Gewebe werden veranlasst, somatische Embryonen zu bilden oder Sprosse zu regenerieren, um transgene Pflanzen gemäss etablierter, der Fachwelt bekannter Verfahren zu erhalten. Die geeigneten Verfahren können entsprechend der verwendeten Pflanzenart gewählt werden.

Die regenerierte Pflanze kann hinsichtlich der eingebauten fremden DNA chimär sein. Wenn die Zellen, die die fremde DNA enthalten, sich entweder in Mikro- oder Makrosporen entwickeln, wird die integrierte fremde DNA der sexuellen Nachkommenschaft vererbt werden. Wenn die Zellen, die die fremde DNA enthalten, somatische Zellen der Pflanze sind, werden nichtchimäre transgene Pflanzen durch konventionelle Verfahren der vegetativen Vermehrung entweder in vivo, d.h. aus Knospen oder Stengelabschnitten, oder in vitro gemäss etablierter der Fachwelt bekannter Verfahren hergestellt. Solche Verfahren können entsprechend der verwendeten Pflanzenart gewählt werden.

Beispiel 12: Transformation von Pflanzenzellen durch Injektion

Der Transfer von DNA in Pflanzenzellen wird auch durch Injektion in isolierte Protoplasten, kultivierte Zellen und Gewebe, wie es von Reich et al. (1986 a und b) beschrieben wird, und durch Injektion in meristematische Gewebe von Keimlingen und Pflanzen erreicht, wie von de la Peña et al. (1987), Graves und Goldman (1986), Hooykaas-Van Slogteren et al. (1984) und Grimsley et al. (1987 und 1988) beschrieben. Transgene Pflanzen und deren Nachkommenschaft werden durch der Fachwelt bekannte Verfahren erhalten.

Beispiel 13: Herstellung eines speziellen Kallustyps von *Zea mays*, Elite Inzuchtlinie Funk 2717

*Zea mays*-Pflanzen der Elite Inzuchtlinie Funk 2717 werden bis zur Blüte im Gewächshaus kultiviert und selbstbestäubt. Unreife Kolben mit etwa 2 bis 2,5 mm langen Embryonen werden von den Pflanzen genommen und in 10 % Clorox®-Lösung 20 Minuten desinfiziert. Die Embryonen werden steril aus den Maiskörnern entfernt und mit der embryonalen Achse nach unten auf OMS-Medium gelegt, das 0,1 mg/Liter 2,4-D, 6 % (w/v) Saccharose und 25 mM L-Prolin enthält und mit 0,24 % (w/v) Gelrite® verfestigt ist (Initiationsmedium). Nach zweiwöchiger Kultivierung im Dunkeln bei 27°C wird der Kallus, der sich am Scutellum gebildet hat, vom Embryo entfernt und auf B5-Medium (Gamborg et al., 1968) überführt, das 0,5 mg/Liter 2,4-D enthält und zuvor mit 0,24 % (w/v) Gelrite® verfestigt wird. Der Kallus wird alle 2 Wochen auf frisches Medium übertragen. Insgesamt 8 Wochen, nachdem die Embryonen auf das Initiationsmedium gegeben wurden, kann der Kallusspezialtyp anhand seiner charakteristischen Morphologie identifiziert werden. Dieser Kallus wird auf dem gleichen Medium weiter kultiviert. Nach weiteren 2 Monaten wird der Kallus auf N6-Medium, das 2 mg/Liter 2,4-D enthält und durch Zusatz von Gelrite® verfestigt ist, transferiert und periodisch überimpft.

Beispiel 14: Herstellung einer Suspensionskultur von *Zea mays*-Elite Inzuchtlinie Funk 2717

Der in diesem Beispiel beschriebene Kallus wird wenigstens 6 Monate weiterkultiviert. Der Kallustyp, der für die Weiterkultivierung ausgewählt wird, ist vergleichsweise wenig schleimig, ist körnig und sehr krümelig, so dass er in kleine einzelne Zellaggregate zerfällt, wenn er in Flüssigmedium gegeben wird. Kulturen, die Aggregate von stark vakuolisierten Zellen enthalten, werden verworfen. Ungefähr 500 mg Aliquots des oben beschriebenen Spezialkallus von *Zea mays* der Elite-Inzuchtlinie Funk 2717 werden in 30 ml N6-Medium mit 2 mg/Liter 2,4-D in 125 ml Delong-Kolben gegeben. Nach einwöchiger Kultivierung bei 26°C im Dunkeln auf einer Rundschüttelmaschine (130 Upm, 2,5 cm Dreh-Radius) wird das Medium durch frisches ersetzt. Die Suspensionen werden wieder unter gleichen Bedingungen eine weitere Woche kultiviert. Danach werden die Kulturen geprüft und diejenigen zurückbehalten, die eine kleine Zahl grosser, vakuolisierter Zellen zeigen. Suspensionskulturen, die Aggregate mit grossen, vakuolisierten Zellen enthalten, werden verworfen. Das bevorzugte Gewebe besteht aus cytoplasmareichen, sich teilenden Zellaggregaten, die charakteristischerweise eine glattere Oberfläche als der gewöhnliche Typ der Zellaggregate aufweisen. Bei den zurückbehaltenen Kulturen sind wenigstens 50 % der Zellen in diesen kleinen Aggregaten vorhanden. Dies ist die gewünschte Morphologie. Diese Suspensionen besitzen darüberhinaus eine grosse Wachstumsrate mit einer Verdoppelungszeit von weniger als einer Woche. Die Suspensionskulturen werden wöchentlich überimpft, indem 0,5 ml PCV (packed cell volume: Volumen sedimentierender Zellen in einer Pipette) in 25 ml frisches Medium gebracht werden. Nach 4 bis 6 Wochen, in denen die Weiterkultivierung in der zuvor beschriebenen Weise durchgeführt wird, nehmen die Kulturen um das 2 bis 3fache pro wöchentlicher Ueberimpfung zu. Kulturen, in denen mehr als 75 % der Zellen die gewünschte Morphologie aufweisen, werden für die Weiterkultivierung verwendet. Die Linien werden aufrechterhalten, indem zum Ueberimpfen immer derjenige Kolben ausgewählt wird, dessen Inhalt die beste Morphologie zeigt. In einigen Fällen wird die Dispersion der Kulturen durch regelmässige Filtration alle 2 Wochen durch nichtrostende Stahlsiebe mit einer Porengrösse von 630 µm erhöht, ohne dass dieses Vorgehen unbedingt notwendig ist.

Eine *Zea mays* Suspensionskultur ist bei der American Type Culture Collection (ATCC) hinterlegt worden, Hinterlegungsnummer 40326. Diese Hinterlegung erfolgte gemäss dem Budapester Vertrag (Hinterlegungsdatum: 20. Mai 1987).

Beispiel 15: Herstellung von Protoplasten aus Zellkulturen von *Zea mays*

1 bis 1,5 ml PCV von Zellen der Suspensionskultur, die nach dem vorangehenden Beispiel hergestellt wurde, werden in 10 bis 15 ml einer Filter-sterilisierten Lösung inkubiert, die 4 % (w/v) Cellulase RS und 1 % (w/v) Rhozyme in KMC-Salzlösung (8.65 g/Liter KCl, 16,47 g/Liter $MgCl_2 \cdot 6H_2O$ und 12,5 g/Liter $CaCl_2 \cdot 2H_2O$, 5 g/Liter MES pH 5,6) enthält. Die Reaktion findet bei 30°C während 3 bis 4 Stunden auf einem langsamen Schütteltisch statt. Mit einem umgekehrten Mikroskop (Planktonmikroskop) wird die Entstehung der Protoplasten verfolgt. Die freigesetzten Protoplasten werden folgendermassen gesammelt: Der Reaktionsansatz wird nacheinander durch Siebe mit 100 µm und 50 µm Maschenweite filtriert. Die Protoplasten werden mit dem Volumen, das der ursprünglichen Enzymlösung entspricht, durch die Siebe gespült. 10 ml Protoplastensuspension wird in je ein Einweg-Plastikzentrifugenröhrchen gegeben und mit 1,5 bis 2 ml einer 0,6 M Saccharoselösung (gepuffert bei pH 5,6 mit 0,1 % (w/v) MES und KOH) unterschichtet. Die Röhrchen werden 10 Minuten mit 60 bis 100 x g zentrifugiert und die Protoplasten, die sich in der Zwischenschicht angereichert haben, mit einer Pipette aufgenommen und in ein neues Röhrchen gegeben. Die Protoplasten werden in 10 ml neuer KMC-Salzlösung resuspendiert und 5 Min. mit 60 bis 100 x g zentrifugiert. Der Ueberstand wird entfernt und anschliessend verworfen. Die Protoplasten werden vorsichtig in dem verbleibenden Tropfen resuspendiert. Anschliessend werden schrittweise 10 ml einer 13/14 konzentrierten KMC-Lösung dazugegeben. Nach erneuter fünfminütiger Zentrifugation wird der Ueberstand entfernt und die Protoplasten in einer 6/7 konzentrierten KMC-Lösung erneut resuspendiert. Ein Aliquot wird zum Zählen entnommen, und die Protoplasten werden noch einmal durch Zentrifugieren sedimentiert. Die Protoplasten werden in soviel KM-8p-Medium oder einer 0,5 M Mannitlösung mit 6 mM $MgCl_2$ oder in jedem beliebigen anderen Medium, das für die nachfolgend in den Beispielen beschriebene Transformation geeignet ist, resuspendiert, dass $10^7$ Protoplasten in einem ml enthalten sind.

Beispiel 16: Transformation von *Zea mays*-Protoplasten durch Elektroporation

A. Alle Schritte bis auf die Hitzeschockbehandlung werden bei Raumtemperatur (22° bis 28°C) durchgeführt. Die Protoplasten aus dem vorangehenden Beispiel werden in 0,5 M Mannit mit 0,1 % (w/v) MES und 6 mM $MgCl_2$ resuspendiert. Der Widerstand dieser Suspension wird in der Kammer eines Dialog Electroporator® (DIA-LOG, GmbH, D-4000 Düsseldorf 13, Bundesrepublik Deutschland) gemessen und mit einer 300 mM $MgCl_2$-Lösung auf 1 bis 1,2 kΩ eingestellt. Die Protoplasten werden einer Hitzeschockbehandlung unterzogen, indem der Becher mit der Probe 5 Minuten in ein Wasserbad von 45°C getaucht wird, gefolgt von einem Abkühlen auf Raumtemperatur im Eis. Zu Aliquots von 0,25 ml dieser Suspension werden 4 µg linearisiertes Plasmid, das ein Pflanzen-selektierbares Hygromycin-Resistenzgen wie es von Rothstein et al. (1987) beschrieben wurde oder chimäre Genkonstrukte enthält, die in den Beispielen 32, 36, 41 und 46 beschrieben werden, und 20 µg Kalbsthymus-Carrier DNA gegeben. 0,125 ml 24 % (w/v) PEG (MW: 8000) in 0,5 M Mannit mit 30 mM $MgCl_2$ werden zu den Protoplasten gegeben. Die Mischung wird gut aber vorsichtig vermischt und 10 Minuten inkubiert. Die Probe wird in die Kammer des Elektroporators gegeben und dreimal 10 Sekunden mit Anfangsspannungen von 1500, 1800, 2300 oder 2800 $Vcm^{-1}$ gepulst, mit einer exponentiellen Abfallzeit von 10 µs.

Die Protoplasten werden wie folgt kultiviert: Die Proben werden bei Raumtemperatur in 6 cm Petrischalen gegeben. Nach weiteren 5 bis 15 Minuten werden 3 ml KM-8p-Medium mit 1,2 % (w/v) SeaPlaque® Agarose und 1 mg/Liter 2,4-D hinzugefügt. Die Agarose und die Protoplasten werden gut gemischt, dann lässt man das Medium gelieren.

B. Das Verfahren von A wird mit einer oder mehreren der folgenden Modifikationen wiederholt:

(1) Der Widerstand der Protoplastenpräparation wird auf 0,5 bis 0,7 kΩ eingestellt.
(2) Bei dem verwendeten PEG handelt es sich um ein PEG mit einem Molekulargewicht von 4000.
(3) Kein PEG oder ein halbes Volumen von 12 % (w/v) PEG wird hinzugefügt.
(4) Es wird in Intervallen von 3 Sekunden gepulst.
(5) Die Protoplasten werden nach der Elektroporation in Schalen gegeben, die sich auf einer Platte befinden, welche auf 16°C gekühlt wird.
(6) Die Protoplasten werden nach der Elektroporation in Röhrchen gegeben, mit 10 ml einer 6/7 konzentrierten KMC-Lösung oder W5-Lösung, die 380 mg/Liter KCl; 18,375 g/Liter $CaCl_2 \cdot 2H_2O$; 9 g/Liter NaCl; 9 g/Liter Glucose; pH 6,0 enthält, gewaschen, durch 10 minütige Zentrifugation bei 60 x g sedimentiert, in 0,3 ml KM-Medium resuspendiert und wie in A angegeben ausplattiert.
(7) Keine Kalbsthymus-Carrier-DNA wird hinzugefügt.

Beispiel 17: Transformation von *Zea mays*-Protoplasten durch Behandlung mit Polyethylenglykol

A. Die Protoplasten werden beim letzten Schritt von Beispiel 15 in einer 0,5 M Mannit-Lösung mit 12 bis 30 mM $MgCl_2$ resuspendiert. Die Protoplasten werden, wie in Beispiel 16 beschrieben, einem 5 minütigen Hitzeschock von 45°C ausgesetzt. Die suspendierten Protoplasten werden für die Transformation in Aliquots von je 0,3 ml in Zentrifugenröhrchen verteilt. Während der nächsten 10 Minuten wird DNA (wie in Beispiel 16A und PEG-Lösung (MG 6000, 40 % (w/v); $Ca(NO_3)_2$ 0,1 M; Mannit 0,4 M; pH 8 bis 9 mit KOH) hinzugefügt, sodass die Endkonzentration an PEG 20 % beträgt. Die Aliquots werden 30 Minuten unter gelegentlichem leichten Schütteln inkubiert, die Protoplasten in Petrischalen gegeben (0,3 ml ursprüngliche Protoplasten-Suspension pro 6 cm Durchmesser-Schale) und kultiviert, wie in Beispiel 16A beschrieben.
B. Das Verfahren von A wird wiederholt. Die Protoplasten werden aber nach 30 Minuten Inkubation in der PEG-Lösung gewaschen, indem fünfmal in 2 bis 3 Minuten-Abständen 0,3 ml W5-Lösung hinzugefügt werden. Die Protoplastensuspension wird anschliessend zentrifugiert und nach Entfernen des Ueberstandes kultiviert, wie in Beispiel 16(A) beschrieben.
C. Die Verfahren von A und B werden wiederholt mit der Modifikation, dass die Endkonzentration von PEG zwischen 13 und 25 % (w/v) ist.

Beispiel 18: Regenerierung von Kallus aus Protoplasten

Die Platten, die die Protoplasten in Agarose enthalten, werden bei 26°C im Dunkeln gehalten. Nach 14 Tagen entstehen aus den Protoplasten Kolonien. Agarose mit Kolonien wird auf die Oberfläche einer Petrischale mit 9 cm Durchmesser gegeben, die 30 ml N6-Medium enthält, das 2 mg/Liter 2,4-D, verfestigt mit 0,24 % (w/v) Gelrite® enthält. Dieses Medium wird als 2N6-Medium bezeichnet. Der Kallus wird weiter im Dunkeln bei 26°C kultiviert, Kallusstücke werden alle 2 Wochen auf frisches, festes 2N6-Medium überimpft.

Beispiel 19: Selektion transformierten Kallus von *Zea mays*

Beispiel 18 wird wiederholt mit der Modifikation, dass 100 mg/Liter oder 200 mg/Liter Hygromycin B zum 2N6-Medium hinzugefügt wird, um transformiertes Material selektionieren zu können.

Beispiel 20: Regenerierung von Mais-Pflanzen

A. Kallus wird auf 2N6-Erhaltungsmedium und auf 0N6 (N6-Medium ohne 2,4-D)- sowie N61 (0,25 mg/Liter 2,4-D und 10 mg/Liter Kinetin)-Medium gegeben, um die Regenerierung zu initiieren. Kallus, der auf 0N6- und N61-Medien wächst, wird im Licht kultiviert (16 Stunden/Tag, 800 bis 8000 lx, weisse fluoreszierende Lampen). Auf N61 gewachsener Kallus wird nach 2 Wochen auf 0N6-Medium transferiert, weil eine länger dauernde Kultivierung auf N61-Medium schädlich für den Kallus ist. Der Kallus wird alle 2 Wochen überimpft, selbst wenn er wieder auf dieselbe Medium-formulierung übertragen werden soll. Pflänzchen erscheinen in etwa 4 bis 8 Wochen. Wenn die Pflänzchen eine Höhe von wenigstens 2 cm erreicht haben, werden sie auf 0N6-Medium in GA7-Container transferiert. Wurzeln bilden sich in 2 bis 4 Wochen. Wenn die Wurzeln aussehen, als wenn sie gut Wachstum unterstützen könnten, werden die Pflänzchen in Erde in Torftöpfe übertragen und 4 bis 7 Tage beschattet. Es ist oft hilfreich, den ausgepflanzten Pflänzchen zur Abhärtung 2 bis 3 Tage einen durchsichtigen Plastiktopf überzustülpen. Wenn die Pflanzen voll entwickelt sind, werden sie wie normale Mais-Pflanzen behandelt und im Gewächshaus bis zur Reife kultiviert. Um Nachkommen zu erhalten, werden die Pflanzen selbstbestäubt oder mit Wildtypen gekreuzt.

B. Das Verfahren von A wird wiederholt mit der Modifikation, dass 100 mg/Liter oder 200 mg/Liter Hygromycin B zum Erhaltungsmedium für den Kallus hinzugefügt wird.

Beispiel 21: Herstellung embryogener Suspensionen aus Gewebe von *Dactylis glomerata* L. (Knauelgras)

A. Embryogener Kallus wird aus basalen Abschnitten der jüngsten Blätter von im Gewächshaus gezogenen *D. glomerata* L. initiiert, wie von Hanning und Conger (1982) beschrieben. Die Blätter werden durch etwa zehnminütiges Untertauchen in eine 1:10 verdünnte Clorox®-Lösung [eine Lösung von 5,25 % (w/v) Natriumhypochlorit; The Clorox Company, Oakland, CA 94623, USA] oberflächensterilisiert und anschliessend aseptisch in kleine Abschnitte von 1 bis 5 mm Länge oder Durchmesser geschnitten. Diese Abschnitte werden auf steriles SH-30-Medium gelegt, das 0,8 % (w/v) Agarose als Gelierungsmittel enthält. Kallus und/oder embryogene Strukturen treten innerhalb von 2 bis 6 Wochen nach Auflegen während Kultivierung bei etwa 25°C auf. Embryogener Kallus wird durch Ueberimpfen auf frisches SH-30-Medium im Abstand von 2 bis 4 Wochen und Kultivierung im Dunkeln bei 25°C aufrechterhalten.

B. Embryogene Suspensionskulturen werden durch Einbringen von etwa 0,5 g embryogenen Kallus (Frischgewicht) in 50 ml Flüssigmedium initiiert, das von Gray und Conger (1985) beschrieben ist und 45 µM Dicamba und 4 g/Liter Caseinhydrolysat enthält. Die Suspensionskulturen werden bei 27°C und einer Photoperiode von 16 Stunden Licht (3300 lx), 8 Stunden Dunkel auf einer Rundschüttelmaschine bei etwa 130 Upm in 125 ml Delong-Gefässen, die mit einer Metallkappe und Parafilm® versiegelt sind, kultiviert. Nach etwa 4 Wochen lässt man die grossen Klumpen sich etwa 30 Sekunden lang absetzen, und 10 ml Aliquots des überstehenden Mediums, das kleine Zellhaufen enthält, werden entfernt und in 50 ml frisches Medium übertragen. Dieser Prozess wird alle 3 bis 4 Wochen wiederholt, indem die gelungensten Kulturen verwendet werden, die durch ihre kleinere Haufengrösse und bessere Qualität auf Grund des Vorhandenseins von kleinen, cytoplasmatischen Zellen bestimmt werden. Nach 5 bis 8 Uebertragungen sind die Suspensionen im wesentlichen frei von embryogenen Zellen und die Mehrheit der embryogenen Zellhaufen ist ziemlich klein (150 bis 2000 µm).

Beispiel 22: Isolierung und Reinigung von *Dactylis glomerata* L. Protoplasten

Protoplasten werden aus den embryogenen Suspensionskulturen des vorangegangenen Beispiels hergestellt, indem die Zellen aseptisch durch eine Nalgene® 0,2 µm Filtereinheit gefiltert und anschliessend 0,5 g Zellen (Frischgewicht) zu je 12,5 ml Protoplasten-Enzymmischung in eine Petrischale gegeben werden. Die filtersterilisierte Enzymmischung besteht aus 2 % (w/v) Cellulase RS, 7 mM $CaCl_2 \cdot H_2O$, 0.7 mM $NaH_2PO_4 \cdot H_2O$, 3 mM MES (pH 5,6), Glucose (550 mOs/kg $H_2O$ von pH 5,6). Die Mischung wird auf einem kreisenden Schüttler bei etwa 50 Upm in Dämmerlicht (< 400 lx) etwa 4 bis 5 Stunden lang bewegt. Alles zusammen wird anschliessend durch ein nichtrostendes Stahlsieb (100 µm Maschengrösse) gesiebt, in 12 ml Zentrifugenröhrchen verteilt und bei etwa 60 bis 100 x g etwa 5 Minuten lang zentrifugiert. Das Protoplasten-enthaltende Sediment wird anschliessend dreimal mit Protoplastenkulturmedium KM-8p, das auf 550 mOs/kg $H_2O$ mit Glucose eingestellt ist, gewaschen. An dieser Stelle kann ein weiterer Schritt zur Reinigung der Protoplasten eingeschoben werden. In diesem Fall werden die gewaschenen Protoplasten auf 10 ml KM-8p Kulturmedium, das auf 700 mOs/kg $H_2O$ mit Saccharose eingestellt ist, geschichtet. Nach der etwa zehnminütigen Zentrifugation bei 60 bis 100 x g werden die Protoplasten, die eine Bande an der Grenzfläche bilden, mit Hilfe einer feinen Pipette gesammelt. Schliesslich werden die Protoplasten in 1 bis 2 ml KM-8p-Kulturmedium resuspendiert und durch ein nichtrostendes Maschensieb gesiebt (20 µm Maschenweite). Die freigesetzten Protoplasten werden gesammelt, gewaschen und zur Kultur in KM-8p-Medium resuspendiert oder in osmotisch eingestellten Medium, das für die Transformation geeignet ist.

Beispiel 23: *Dactylis glomerata* L. Protoplastenkultur und Kalluswachstum

A. Die gereinigten Protoplasten werden in einer Dichte von etwa 5 x $10^5$ Protoplasten/ml in KM-8p-Kulturmedium ausplattiert, das 1,3 % (w/v) SeaPlaque® Agarose (FMC Corp., Marine Colloids Division, Rockland, Maine, USA) und 30 bis 40 % (v/v) konditioniertes Medium (erhalten von 3 bis 4 Wochen alten *D. glomerata* L. embryogenen Suspensionskulturen, indem das Medium durch ein steriles Nalgene® 0,2 µm Filter gefiltert, das Medium durch Hinzufügen von Glucose auf 550 mOs/kg $H_2O$ eingestellt und nochmal sterilfiltriert wird) enthält. Die Platten werden anschliessend im Dunklen bei einer konstanten Temperatur von 28°C aufgestellt. Nach 10 bis 14 Tagen wird die Agarose in Keile geschnitten und in eine "bead" Kultur, wie sie von Shillito et al. (1983) beschireben wird, gegeben, indem 20 ml SH-45 Suspensionskulturmedium mit 3 % (w/v) Saccharose pro 3 ml ursprünglicher in Agarose eingebetteter Kultur verwendet wird. Die Platten werden auf eine Schüttelvorrichtung gestellt und mit etwa 50 Upm im Licht von 670 lx geschüttelt. Neue Suspensionskulturen bilden sich, während die Kolonien aus der Agarose herauswachsen und Zellen in das Flüssigmedium freilassen. Die resultierenden, in der Suspension kultivierten Zellen werden auf durch Agar verfestigtes SH-30-Medium ausplattiert und im Dunklen aufgestellt bis sich Kallus bildet.
B. Protoplasten werden wie in A beschrieben kultiviert bis darauf, dass das Kulturmedium kein konditioniertes Medium enthält.

Beispiel 24: Transformation von *Dactylis glomerata* L. Protoplasten mit Hilfe der Elektroporation

A. Sofort nach der Reinigung der Protoplasten wird die Elektroporation gemäss Shillito et al. (1985) durchgeführt, indem ein linearisiertes Plasmid verwendet wird, wie es im Beispiel 16 beschrieben ist. Die Protoplasten werden nach dem letzten Waschen in einer Dichte von etwa 7 x $10^6$ Protoplasten/ml im Elektroporationspuffer (0,4 M Mannit, 6 mM $MgCl_2$) resuspendiert. Die Protoplasten werden in Aliquots von 0,7 ml in 10 ml Plastikzentrifugenröhrchen gegeben. Plasmid DNA in einer Endkonzentration von 10 µg/ml und beschallte Kalbsthymus DNA in einer Konzentration von 50 µg/ml werden zu den Röhrchen gegeben. Anschliessend werden 0,38 ml PEG-Lösung [24 % (w/v) PEG 6000 in 0,4 M Mannit, 30 mM $MgCl_2$, 0,1 % (w/v) MES (pH 5,6)] hinzugefügt und die Lösung leicht gemischt. Die Protoplastensuspension wird in die Kammer eines Dialog® Elektroporators überführt und 10 Pulse mit einer Anfangsspannung von 3250 $Vcm^{-1}$ und einer exponentiellen Abfallkonstante von 10 µs werden in 30 s Abständen gegeben. Die Probe wird aus der Kammer entfernt und in eine Petrischale mit einem Durchmesser von 10 cm gegeben. 10 ml KM-8p-Medium, das 1,2 % (w/v) SeaPlaque® Agarose enthält, wird hinzugefügt, die Protoplasten gleichmässig im gesamten Medium verteilt und die Agarose gelieren lassen.
B. Beispiel 24 A wird wiederholt bis darauf, dass die benutzte Anfangsspannung 2500 $Vcm^{-1}$, 3000 $Vcm^{-1}$, 3500 $Vcm^{-1}$, 4000 $Vcm^{-1}$ oder 5000 $Vcm^{-1}$ ist.
C. Beispiels 24 A und B werden wiederholt, bis darauf, dass ein PEG mit MW 4000 oder ein PEG mit MW 8000 verwendet wird.
D. Die Beispiele 24 A bis C werden wiederholt, bis darauf, dass die Endkonzentration von PEG zwischen 10 % und 30 % (w/v) ist.

Beispiel 25: Transformation von *Dactylis glomerata* L. Protoplasten durch Behandlung mit Polyethylenglykol (PEG)

A. Der von PEG vermittelte direkte Gentransfer wird gemäss Negrutiu et al. (1987) durchgeführt. Nach dem letzten Waschen werden die Protoplasten in 0,5 M Mannit mit 15 mM $MgCl_2$ in einer Dichte von etwa 2 x $10^6$ pro ml resuspendiert. Die Protoplastensuspension wird in 1 ml Aliquots in 10 ml Plastikzentrifugenröhrchen verteilt. Die DNA wird wie in Beispiel 24 oben beschrieben hinzugefügt und anschliessend werden 0,5 ml der PEG-Lösung hinzugeben [40 % (w/v) PEG 4000 in 0,4 M Mannit, 0,1 M $Ca(NO_3)_2$, pH 7,0]. Die Lösungen werden leicht vermischt und 30 Minuten lang bei Raumtemperatur (etwa 24°C) unter gelegentlichem Schütteln inkubiert. Anschliessend werden 1,4 ml der Waschlösung hinzugefügt und der Inhalt des Röhrchens leicht gemischt. Die Waschlösung besteht aus 87 mM Mannit, 115 mM $CaCl_2$, 27 mM $MgCl_2$, 39 mM KCl, 7 mM Tris/HCl und 1,7 g/Liter myo-Inosit, pH 9,0. Vier weitere 1,4 ml Aliquots Waschlösung werden in 4 Minuten Abständen hinzugefügt, mit einer Durchmischung nach jeder Zugabe. Das Röhrchen wird anschliessend mit 60 x g etwa 10 Minuten lang zentrifugiert und der Ueberstand verworfen. Die sedimentierten Protoplasten werden in 1 ml KM-8p-Kulturmedium aufgenommen und in eine 10 cm Petrischale gegeben. 10 ml KM-8p-Medium, dass 1,2 % (w/v) Sea-Plaque® Agarose enthält, wird hinzugefügt. Die Protoplasten werden gleichmässig im Medium verteilt und die Agarose gelieren lassen.

B. Das Verfahren von A wird mit einer oder mehreren der folgenden Modifikationen wiederholt:

(1) Der pH der Waschlösung wird auf 5,6 oder 7,0 eingestellt.
(2) Das verwendete PEG hat ein Molekulargewicht von 2000, 6000 oder 8000.
(3) Das Waschmedium besteht aus 154 mM NaCl, 125 mM $CaCl_2$, 5 mM KCl, 5 mM Glucose, mit KOH auf pH 6,0 eingestellt, oder aus 0,2 M $CaCl_2$, 0,1 % (w/v) MES, mit KOH auf pH 6,0 eingestellt, oder aus 0,2 M $CaCl_2$, 7 mM Tris/HCl, mit KOH auf pH 9,0 eingestellt.

Beispiel 26: Transformation von *Dactylis glomerata* L. Protoplasten nach Hitzeschockbehandlung

Die Transformation wird wie in den Beispielen 24 oder 25 beschrieben ausgeführt, bis darauf, dass die Protoplasten vor der Verteilung der Aliquots in die Röhrchen zur Transformation oder nach der Verteilung der Aliquots und vor der Zugabe von PEG 5 Minuten lang bei 45°C inkubiert werden.

Beispiel 27: Selektion transformierter Kolonien

A. Die Kulturplatten (Petrischalen), die die Protoplasten aus den Beispielen 24 bis 26 enthalten, werden 10 Tage lang im Dunklen bei etwa 25°C inkubiert und anschliessend in 5 gleiche Scheiben für die "Bead" Kultur (Shillito et al., 1983) geschnitten. 4 der Scheiben werden je in 20 ml SH-45 Kulturmedium mit 4 g/Liter Caseinhydrolysat und 20 μg/ml Hygromycin B gelegt. Die fünfte Scheibe wird in 20 ml des gleichen Mediums, jedoch ohne Hygromycin B, als nicht selektierte Kontrolle gelegt. Nach 4 bis 5 Wochen werden die mutmasslich transformierten, von Protoplasten abgeleiteten Zellkolonien, die in Hygromycin B wachsen, aus der Agarose herausgeschnitten und in eine 19 mm Petrischale mit 2 ml flüssigem SH-45-Medium, das 20 μg/ml Hygromycin B enthält, gegeben, das mit etwa 50 Upm auf einem kreisenden Schüttler geschüttelt wird. Nach weiteren 4 bis 5 Wochen werden alle Kolonien, die zu neuen Suspensionen heranwachsen, in 125 ml Erlenmeyerkolben überführt, und in einer ähnlichen Weise wie die elterliche Suspensionskultur gezogen, bis darauf, dass 20 μg/ml Hygromycin B im Medium enthalten ist.

Die neuen Suspensionen werden alle 1 bis 3 Wochen in neues Medium überimpft, indem SH-45-Medium, das 4 g/Liter Caseinhydrolysat und 20 μg/ml Hygromycin B enthält, verwendet wird. Zellen zus diesen Suspensionen werden auch auf festem SH-30-Medium, das 20 μg/ml Hygromycin B enthält, ausplattiert und bei etwa 25°C im Dunklen inkubiert. Kalli, die aus den ausplattierten Zellen wachsen, werden alle 2 Wochen in frisches Medium überimpft. Die Zellen, die in Gegenwart von Hygromycin B wachsen, sind mit grosser Wahrscheinlichkeit Transformanten.

B. Die Selektion wird wie in Beispiel 27 (A) beschrieben durchgeführt, bis darauf, dass die von den Protoplasten abgeleiteten Zellkolonien, die in Hygromycin B enthaltendem Medium wachsen, auf SH-30-Medium-Agarplatten, die 20 μg/ml Hygromycin B enthalten, gelegt und bei etwa 25°C im Dunklen inkubiert werden.

Beispiel 28: Regenerierung von transformierten *Dactylis glomerata* L. Pflanzen

A. *D. glomerata* L. Kallus (erhalten wie in Beispiel 27 beschrieben) der von Protoplasten abgeleitet ist, wird auf festem SH-30-Medium gezogen und alle 2 Wochen überimpft. Alle Embryonen, die sich bilden, werden entfernt, auf einem Keimungsmedium ausplattiert (SH-O) und ins Licht gebracht (3700 bis 4600 lx). Die Keimung dieser Embryonen erfolgt in 1 bis 4 Wochen und die resultierenden Pflänzchen werden auf SH-O-Medium im Licht gebracht, um Wurzelsysteme zu bilden. Sie werden im 6- bis 12-Blatt-Stadium ins Gewächshaus gebracht und schrittweise abgehärtet.

B. Kallus (erhalten wie in Beispiel 27 beschrieben), der von Protoplasten abgeleitet ist, wird auf SH-O-Medium, das mit 0,24 % (w/v) GelRite® verfestigt ist, im Licht (3700 bis 4600 lx) gezogen und alle zwei Wochen überimpft. Die resultierenden Pflänzchen werden auf eine 1:1 Mischung von SH-O- und OMS-Medium gebracht, die mit einer Kombination von 0,12 % (w/v) GelRite® und 0,4 % (w/v) Agar verfestigt ist, und beleuchtet, um Wurzelsysteme zu bilden. Sie werden im 6- bis 12-Blatt-Stadium ins Gewächshaus gebracht und schrittweise abgehärtet.

C. Kleine Pflänzchen werden erhalten wie in A und B beschrieben, auf OMS-Medium gebracht, das mit 0,8 % (w/v) Agar verfestigt ist, und beleuchtet, damit sich Wurzelsysteme entwickeln können. Sie werden im 6-bis 12-Blatt-Stadium ins Gewächshaus gebracht und schrittweise abgehärtet.

D. Kleine Pflanzen werden wie in A beschrieben erhalten, auf eine 1:1 Mischung von SH-O und OMS-Medium, verfestigt mit einer Kombination von 0,12 % (w/v) GelRite$^{®}$ und 0,4 % (w/v) Agar, gelegt und beleuchtet, um Wurzelsysteme zu bilden. Sie werden im 6- bis 12-Blattstadium ins Gewächshaus gebracht und schrittweise abgehärtet.

Beispiel 29: Transformation und Regenerierung von Tomaten

Tomaten werden mit manipulierten *A. tumefaciens* transformiert (Nelson et al., 1988), das einen in Pflanzen selektierbaren Marker (z.B. Kanamycin-Resistenz) und das chimäre Gen enthält, das den interessierenden Protease-Inhibitor kodiert. Die Blattscheibentransformation von Tomaten mit *A. tumefaciens* erfolgt gemäss McCormick et al. (1986).

Beispiel 30: Transformation und Regenerierung von Kartoffeln

Kartoffeln werden mit manupulierten *A. tumefaciens* transformiert (Stockhaus et al., 1987), das einen in Pflanzen selektierbaren Marker (z.B. Kanamycin-Resistenz) und das chimäre Gen, das den interessierenden Protease-Inhibitor kodiert, enthält.

**IV. Ergebnisse**

Beispiel 31: Bioassay transformierter Baumwolle

Eier von *Heliothis virescens* werden vom "Tobacco Insect Control Laboratory at North Carolina State University, Raleigh, North Carolina" bezogen. Die Eier befinden sich auf einer Mullenterlage, die in grosse abgedeckte Bechergläser überführt wird. Das Becherglas ist mit feuchtem Filterpapier ausgekleidet, um eine gleichbleibende Feuchtigkeit zu garantieren. Die Inkubation der *Heliothis*-Eier erfolgt bei einer Temperatur von 29°C. Unter diesen Bedingungen schlüpfen die Larven innerhalb von drei Tagen. Nach dem Schlüpfen werden die Larven sobald wie möglich in kleine abgedeckte Plastikbehälter überführt (1 Larve/Behälter), die jeweils eine Baumwollblattscheibe enthalten. Die Uebertragung der Larven erfolgt mit Hilfe eines feinborstigen Pinsels.

Die Blattscheiben, die einen Durchmesser von einem Zentimeter aufweisen, werden zuvor aus den Blättern von Baumwollpflanzen ausgestanzt und in dem Plastikbehälter auf ein rundes angefeuchtetes Filterpapier aufgebracht. Von jeder Pflanze werden mindestens 6 bis 10 Blattscheiben, die sowohl von jungen als auch von alten Blättern stammen, getestet. Die Blattscheiben werden in 2 Tagesintervallen oder aber in Abhängigkeit von der Nahrungsaufnahme nach Bedarf ersetzt. Die Wachstumsraten (die Grösse oder das Gesamtgewicht aller innerhalb einer Versuchsgruppe befindlichen Larven) sowie die Sterblichkeitsrate der Larven, die sich von Blättern transformierter Pflanzen ernähren, werden mit denjenigen der Kontrolltiere, die sich von nichttransformierten Baumwollblättern ernähren, verglichen.

Larven, die sich von Blattscheiben ernähren, die von Baumwollpflanzen stammen, die mit dem Protease-Inhibitor-Gen transformiert sind, zeigen eine stark verminderte Wachstumsrate sowie eine um 100 % erhöhte Sterblichkeit im Vergleich mit den Kontrollen.

Beispiel 32: Mais, der durch die Expression von Hühnereiweiss-Cystatin resistent gegen Schaden durch *Diabrotica* ist

A. Konstruktion des pCIB715/710-Cystatin-Vektors. Das Cystatin-Gen wird wie in Beispiel 3 C beschrieben synthetisiert. Die 363 Bp DNA-Sequenz wird gereinigt und in die BamHI-Schnittstelle des pCIB710-Vektors wie in Beispiel 5 beschrieben eingefügt. Der resultierende Vektor wird als pCIB710-Cystatin bezeichnet. Dieser Vektor wird mit EcoRI geschnitten und die resultierende DNA mit alkalischer Phosphatase behandelt. Plasmid pCIB715 (Rothstein et al., 1987) wird mit EcoRI geschnitten und mit pCIB710-Cystatin von oben verknüpft. Der resultierende Vektor wird pCIB715/710-Cystatin genannt.

B. Transformation und Regenerierung von Mais. Maisgewebe wird mit dem pCIB710 Vektor oder vorzugsweise dem pCIB715/710 Vektor, der den Cystatin Geneinscub trägt, transformiert und Pflanzen werden wie in den Beispielen 16 bis 20 beschrieben regeneriert. Zur Kontrolle werden Pflanzen, die nur mit dem pCIB710 Vektor oder dem pCIB715 Vektor ohne Geneinschub transformiert sind, auf dieselbe Weise hergestellt. Transformiertes Pflanzengewebe wird durch Empfindlichkeit gegenüber dem entsprechenden Antibiotikum selektioniert. Die Ausgangspflanzen werden selbstbefruchtet und man erhält Samen (T1 Samen).

C. Test von Pflanzen auf Cystatin-Expression. Pflanzen, die aus den T1 Samen gewachsen sind, werden auf das Vorhandensein und die Expression des Cystatin-Gens hin untersucht, indem verschiedene Tests angewendet werden.

(1) DNA wird isoliert und mit BamHI geschnitten; die Abbauprodukte werden auf einem 1,5 % Agarose-Gel elektrophoretisch aufgetrennt. Die DNA-Fragmente werden auf Nitrocellulose übertragen und mit dem Cystatin-Gen, das mit $^{32}$P durch nick-Translation markiert ist, hybridisiert [siehe Maniatis et al. (1982) wegen der Techniken]. Die Anwesenheit des Cystatin-Gens wird durch eine Bande ausfindig gemacht, die etwa 363 Basenpaaren entspricht und mit der Probe hybridisiert.

(2) RNA wird durch das Northern Blot Verfahren (Maniatis et al., 1982) als eine Bande ausfindig gemacht, die etwa 380 Basen entspricht, und mit dem $^{32}$P-Cystatin-Gen hybridisiert, das oben beschrieben ist.

(3) Cystatin-Protein wird mit immunologischen Standardtechniken mit einem monoklonalen Kaninchen Antikörper ausfindig gemacht, der gegen kommerziell (Sigma) erhältliches Cystatin produziert wurde.

(4) Cystatin-Aktivität wird dadurch ausfindig gemacht, dass Material aus den Pflanzenextrakten mit immunologischen Methoden gereinigt wird, indem der polyklonale Anti-Cystatin-Antikörper aus Kaninchen und Protein A Sepharose verwendet und das isolierte Material auf die Fähigkeit getestet wird, die Proteolyse von $^{14}$C-BSA durch Papain und durch Darmhomogenate von *Diabrotica* wie in Beispiel 1 beschrieben zu hemmen.

D. Resistenze von transformierten Maispflanzen gegen Schaden durch *Diabrotica*. Aus T1 Samen erhaltene Keimlinge werden in grobem Vermikulit in 100 mm Petrischalen (5 pro Schale, 5 Schalen) gepflanzt. Wenn die zweiten Blätter am Keimling erscheinen, wird jede Schale mit 20 *Diabrotica*-Larven im zweiten Häutungsstadium infiziert. Nach 7 Tagen werden die Zahl, das Gewicht der Ueberlebenden und das Gewicht der gewaschenen Wurzeln der Maispflanze bestimmt. Die Resistenz der transformierten Pflanzen wird durch eine statistisch signifikante (Student's Test p <0,05) Erniedrigung der Gewichtszunahme der Larven, Erniedrigung der Ueberlebensrate der Larven oder in der Erniedrigung im Verlust des Wurzelgewichts, verglichen mit Insekten-freien Pflanzen bestimmt, indem Pflanzen, die das Cystatin-Gen exprimieren, mit Kontrollpflanzen die nur mit dem Vektor ohne Geneinschub transformiert sind, oder mit nicht-transformierten Pflanzen verglichen werden.

Beispiel 33: Kartoffeln und Tomaten, die durch die Expression von Cystatin resistent gegen Schaden durch *Leptinotarsa decemlineata* (Kartoffelkäfer) sind

A. Konstruktion des pCIB10-Cystatin Vektors. Das Cystatin-Gen wird synthetisiert und wie in Beispiel 32A beschrieben mit dem Vektor pCIB710 verknüpft. Das Cystatin-Gen und der 35S CaMV-Promotor werden weiterhin aus dem pCIB710 Vektor heraus, in den pCIB10 Vektor hineinkloniert. Dazu wird pCIB710 mit XbaI und EcoRI geschnitten, das 1593 Bp Fragment isoliert und dieses Fragment mit XbaI und EcoRI geschnittenem pCIB10 verknüpft, um den pCIB10-Cystatin Vektor zu erhalten.

B. Transformation und Regenerierung von Pflanzen. Der pCIB10-Cystatin Vektor oder vorzugsweise der pCIB715/710-Cystatin Vektor wird in *A. tumefaciens*, das ein Virulenzplasmid wie LBA 4404 (Beispiel 5) oder pCIB542 trägt, eingeführt. pCIB542 ist ein manipuliertes *A. tumefaciens* vir-Plasmid, das von pTiBo542 (Hood et al., 1986) abgeleitet ist. Bei pCIB542 ist das bakterielle Kanamycin-Resistenzgen ersetzt durch ein bakterielles Streptomycin/Spectinomycin-Resistenzgen. Der Stamm, der sowohl pCIB710-Cystatin oder pCIB715/710 Cystatin als auch pCIB542 trägt, wird verwendet, um transformierte Tomatenpflanzen gemäss dem Verfahren von Fischhoff et al. (1987) oder wie in Beispiel 29 zu machen.

Kartoffelpflanzen, die pCIB10-Cystatin oder pCIB715/710 Cystatin enthalten, werden nach dem Verfahren von Stockhaus et al. (1987), Beispiel 30, erhalten.

C. Test von Pflanzen auf Cystatin-Expression. Tests der Transformanten auf Cystatin-Expression werden wie oben für Mais beschrieben durchgeführt.

D. Resistenz von transformierten Pflanzen gegen Schaden durch *L. decemlineata*. 10 vier Wochen alte Pflanzen werden je mit 5 Larven von *L. decemlineata* im 2. Häutungsstadium infiziert. Man lässt die Larven 4 Tage lang fressen und bestimmt dann die Insekten-Mortalität, die Gewichtszunahme der Insektion und das Ausmass des den Pflanzen zugefügten Schadens. Die Resistenz der transformierten Pflanzen wird durch eine statistisch signifikante (Student's t Test p <0,05) Abnahme in der Gewichtszunahme sowie Ueberlebensrate der Larven oder einer Abnahme des Pflanzenschadens ermittelt, indem Pflanzen, die das Cystatin-Gen exprimieren, mit Kontrollpflanzen, die nur mit dem Vektor ohne Geneinschub transformiert sind, oder mit nichttransformierten Pflanzen, verglichen werden.

Beispiel 34: Knäuelgras (*Dactylis glomerata*), das durch die Expression von Cystatin resistent gegen Schaden durch *Coleoptera* (Käfer) ist

A. Konstruktion des Vektors. pCIB710 Cystatin und pCIB715/710 Cystatin werden hergestellt wie in Beispiel 32A beschrieben.

B. Transformation und Regenerierung von Pflanzen. Transformierte Pflanzen werden wie in den Beispielen 24 und 28 beschrieben erhalten, indem pCIB710 Cystatin und pCIB715/710 Cystatin verwendet werden.

C. Test der Pflanzen auf Cystatin-Expression. Tests der Transformanten auf Cystatin-Expression werden wie in Beispiel 32C beschrieben durchgeführt.

D. Resistenz von transformierten Pflanzen gegen Schaden durch *Diabrotica undecimpunctata*. Aus T1 Samen erhaltene Keimlinge werden in feine Erde in 100 mm Petrischalen (10/Schale, 5 Schalen) gepflanzt. Wenn die zweiten Blätter am Keimling erscheinen, wird jede Schale mit 20 *Diabrotica*-Larven im zweiten Häutungsstadium infiziert. Nach 7 Tagen werden die Zahl und das Gewicht der Ueberlebenden bestimmt, zusammen mit dem Gewicht der gewaschenen Wurzeln der Pflanze. Die Resistenz der transformierten Pflanzen wird durch eine statistisch signifikante (Student's t Test p<0,05) Erniedrigung der Gewichtszunahme der Larven, Erniedrigung der Ueberlebensrate der Larven oder Erniedrigung im Verlust des Wurzelgewichts, verglichen mit Insekten-freien Pflanzen, ermittelt, indem Pflanzen, die das Cystatin-Gen exprimieren, mit Kontrollpflanzen, die nur mit dem Vektor ohne Geneinschub transformiert sind, oder mit nicht-transformierten Pflanzen verglichen werden.

Beispiel 35: Baumwolle, die durch Expression von Cystatin resistent gegen Schaden durch *Anthonomus grandis* (Amerikanischer Baumwollkapselkäfer) ist

A. Konstruktion des Vektors. pCIB715/710 Cystatin und pCIB10 Cystatin werden wie in den Beispielen 32A und 33A beschrieben hergestellt.

B. Transformation und Regenerierung von Pflanzen. Transformierte Pflanzen werden wie in den Beispielen 7 bis 10 wie beschrieben erhalten, indem pCIB 715/710 Cystatin oder pCIB10 Cystatin in *Agrobacterium* verwendet werden.

C. Test der Pflanzen auf Cystatin-Expression. Tests der Transformanten auf Cystatin-Expression werden wie in Beispiel 32C beschrieben durchgeführt.

D. Resistenz von transformierten Pflanzen gegen Schaden durch *A. grandis*. 10 transformierte Pflanzen werden gezogen, bis sich Kapseln zu bilden beginnen. Jede Pflanze wird mit 3 erwachsenen weiblichen *A. grandis* infiziert. Der den Pflanzen zugefügte Schaden wird nach einer Woche bewertet und überlebende Erwachsene werden entfernt. Der Schaden durch Larven, die Zahl der Larven und das Gewicht pro Pflanze werden in wöchentlichen Abständen 4 Wochen lang gemessen. Die Resistenz der transformierten Pflanzen wird durch eine statistisch signifikante (Student's t Test p<0,05) Erniedrigung der Gewichtszunahme der Larven, Erniedrigung der Ueberlebensrate der Larven oder Erniedrigung im Verlust des Wurzelgewichts verglichen mit Insekten-freien Pflanzen bestimmt, indem Pflanzen, die das Cystatin-Gen exprimieren, mit Kontrollpflanzen, die nur mit dem Vektor ohne Geneinschub transformiert sind, oder mit nicht-transformierten Pflanzen verglichen werden.

Beispiel 36: Mais, der durch Expression des Sojabohnen Kunitz Trypsin-Inhibitors resistent gegen Schaden durch *Lepidoptera* (Schmetterlinge) Larven ist

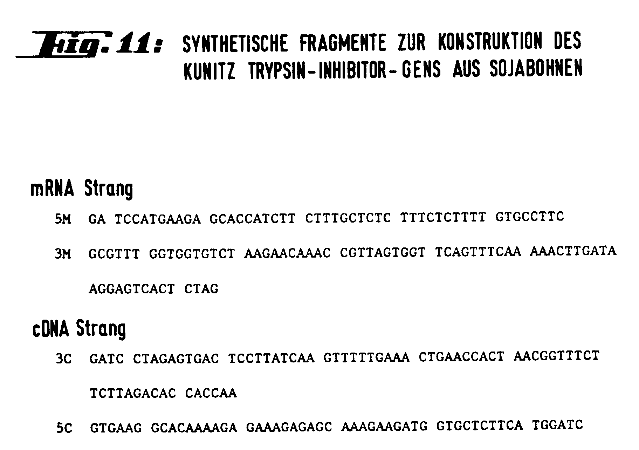

A. Konstruktion des pCIB715/710-KTI Vektors. Die Trypsin-Inhibitor cDNA aus Sojabohnen wird durch das Verfahren von Hoffman et al. (1984) erhalten. Das 652 Bp Fragment wird verändert, so dass die Sequenz am Methionin Startcodon (44 im ursprünglichen Fragment) beginnt. An das 3′-Ende werden Codons für die fehlenden drei Aminosäuren plus einem Stopcodon angefügt. BamHI Linker werden an beide Enden angefügt. Diese Konstruktion wird erreicht, indem zuerst das 652 Bp Fragment mit Eco57I und BstXI geschnitten sowie das resultierende 517 Bp Fragment nach einer Agarose Gelelektrophorese isoliert wird. Die Oligonukleotide, die in Fig. 11 abgebildet sind, werden synthetisiert. Phosphate werden an die 5′-Enden des 517 Bp Fragmentes und die Oligonukleotide 3M und 5C gehängt und alle Fragmente werden wie oben beschrieben verknüpft. Das resultierende Fragment wird isoliert und mit der BamHI-Schnittstelle des pCIB710 Vektors wie oben beschrieben verknüpft. Der resultierende Vektor wird als pCIB710-KTI bezeichnet.

B. Transformation und Regenerierung von Mais. Maisgewebe wird mit dem pCIB710 Vektor oder bevorzugt mit dem pCIB715/710 Vektor, der den Soja Kunitz Trypsin Inhibitor-Geneinschub enthält, transformiert und Pflanzen werden wie in Beispielen 16 bis 20 beschrieben regeneriert. Zur Kontrolle werden Pflanzen auf dieselbe Weise hergestellt, jedoch mit dem pCIB710 Vektor oder dem pCIB715 Vektor ohne Geneinschub. Transformiertes Pflanzengewebe wird auf Grund der Empfindlichkeit gegenüber dem entsprechenden Antibiotikum selektioniert. Die Anfangspflanzen werden selbstbestäubt und Samen (T1 Samen) wird erhalten.

C. Test von Pflanzen auf die Expression des Kunitz Trypsin-Inhibitors aus Sojabohnen. Pflanzen, die aus T1 Samen gezogen wurden, werden auf das Vorhandensein und die Expression des Kunitz Trypsin-Inhibitor-Gens aus Sojabohnen getestet, indem die folgenden Testverfahren angewendet werden.

(1) DNA wird isoliert und mit BamHI geschnitten; die Abbauprodukte werden auf einem 1,5 % Agarosegel elektrophoretisch aufgetrennt. Die DNA-Fragmente werden auf Nitrocellulose übertragen und mit dem Kunitz Trypsin-Inhibitor-Gen aus Sojabohnen hybridisiert, das durch nick-Translation mit $^{32}$P markiert ist [siehe Maniatis et al. (1982) wegen der Techniken]. Das Vorhandensein des Kunitz Trypsin-Inhibitor-Gens wird durch eine Bande ausfindig macht, die etwa 630 Bp entspricht und mit der Probe hybridisiert.

(2) RNA wird durch das Northern Blot Verfahren als eine Bande ausfinding gemacht, die etwa 650 Basen entspricht und mit dem $^{32}$P-Kunitz Trypsin-Inhibitor-Gen aus Sojabohnen hybridisiert, das oben beschrieben ist.

(3) Kunitz Trypsin-Inhibitor-Protein aus Sojabohnen wird mit Hilfe immunologischer Standardtests mit einem polyklonalen Antikörper aus Kaninchen nachgewiesen, der gegen kommerziell (Sigma) erhältlichen Kunitz Trypsin-Inhibitor nach Reinigung durch Reverse Phase-HPLC über eine Vydac Phenylsäule produziert wurde.

(4) Die Aktivität des Kunitz Trypsin-Inhibitors aus Sojabohnen wird bestimmt, indem mit immunologischen Verfahren Material aus Pflanzenextrakten mit Hilfe des polyklonalen Anti-Sojabohnen-Kunitz Trypsin-Inhibitor-Antikörpers aus Kaninchen und Protein A Sepharose gereinigt und anschliessend das isolierte Material auf die Fähigkeit getestet wird, die Proteolyse von $^{14}$C-BSA durch Trypsin zu hemmen, indem der in Beispiel 1 beschriebene Test benutzt wird.

D. Resistenz transformierter Pflanzen gegenüber Schaden durch Lepidopteren-Larven. T1 Samen werden gekeimt und Blattstücke, die von Keimlingen im 4-Blattstadium gewonnen werden, an frischgeschlüpfte Larven von *Ostrinia nubilalis* oder *Heliothis zea* verfüttert. Frischgeschlüpfte Larven werden in einzelne Fütterungstöpfe mit einem 1 cm$^2$ Blattstück gesetzt. 50 Insektion werden pro Gruppe getestet. Nach 5 Tagen werden das Insektengewicht, das Insektenüberleben und die Menge gefressener Blätter bestimmt. Die Resistenz der transformierten Pflanzen wird durch einen statistisch signifikanten (Student's t Test $p < 0,05$) Abfall im Insektenüberleben, im Insektengewicht oder in der Menge der gefressenen Blätter bestimmt, indem Blätter von Pflanzen, die das Kunitz Trypsin-Inhibitor-Gen aus Sojabohnen exprimieren, mit denen von Kontrollpflanzen, die mit dem Vektor ohne Geneinschub transformiert wurden, oder mit denen von nichttransformierten Pflanzen verglichen werden.

Beispiel 37: Baumwolle, die durch Expression des Kunitz Trypsin-Inhibitors aus Sojabohnen resistent gegen Schaden durch Lepidopterenlarven ist

A. Konstruktion des pCIB10-KTI Vektors. Das Kunitz Trypsin-Inhibitor-Gen aus Sojabohnen wird zusammen mit dem 35S CaMV Promotor aus pCIB710-KTI (Beispiel 36A) entfernt, indem geeignete Restriktonsenzyme verwendet werden, und mit pCIB10 verknüpft. Dieser Vektor wird als pCIB10-KTI bezeichnet.

B. Transformation und Regenerierung von Pflanzen. Transformierte Pflanzen werden wie in den Beispielen 7 bis 10 beschrieben erhalten, indem pCIB10-KTI oder pCIB715/710 KTI (Beispiel 36A) in *Agrobacterium* verwendet werden.

C. Test von Pflanzen auf die Expression des Trypsin-Inhibitors. Tests von Transformanten auf die Expression des Kunitz Trypsin-Inhibitors aus Sojabohnen werden wie in Beispiel 36C beschrieben durchgeführt.

D. Resistenz von transformierten Pflanzen gegen Lepidopteren. Blattscheiben von 4 Wochen alten transformierten Pflanzen werden an frischgeschlüpfte *Heliothis virescens, H. zea* oder *Pectinophora gossypiella* verfüttert. Frischgeschlüpfte Larven werden in einzelne Fütterungsbecher mit einem 1 cm$^2$ Blattstück gesetzt. 50 Insekten werden pro Gruppe getestet. Nach 5 Tagen werden das Insektengewicht, das Insektenüberleben und die Menge der gefressenen Blätter bestimmt. Die Resistenz der transformierten Pflanzen wird durch einen statistisch signifikanten (Student's t Test p<0,05) Abfall im Larvengewicht, Larvenüberleben und in der Menge der gefressenen Blätter bestimmt, indem die Blätter von Pflanzen, in denen das Kunitz Trypsin-Inhibitor-Gen exprimiert wird, mit denen von Kontrollpflanzen, die mit dem Vektor ohne Geneinschub transformiert sind, oder mit denen von nichttransformierten Pflanzen verglichen werden.

Beispiel 38: Tomaten, die durch die Expression des Kunitz Trypsin-Inhibitors aus Sojabohnen resistent gegen Schaden durch Lepidopteren-Larven sind

A. Konstruktion des Vektors. pCIB715/710-KTI und pCIB10-KTI werden wie in den Beispielen 36A und 37A beschrieben hergestellt.

B. Transformation und Regenerierung von Pflanzen. Transformierte Pflanzen werden wie in Beispiel 33B beschrieben hergestellt, indem der pCIB10-KTI Vektor oder der pCIB715/710-KTI Vektor verwendet wird.

C. Test von Pflanzen auf die Expression des Trypsin-Inhibitors. Der Test der Transformanten auf die Expression des Kunitz Trypsin-Inhibitors aus Sojabohnen wird wie in Beispiel 36C beschrieben durchgeführt.

D. Resistenz von transformierten Pflanzen gegen Schaden durch Lepidopteren. Blattscheiben von 4 Wochen alten transformierten Pflanzen werden an frischgeschlüpfte *Heliothis zea* oder *Manduca sexta* verfüttert. Frischgeschlüpfte Larven werden in einzelne Fütterungsbecher mit einem 1 cm$^2$ Blattstück gesetzt. 50 Insekten werden pro Gruppe getestet. Nach 5 Tagen wird das Insektengewicht, das Insektenüberleben und die Menge der gefressenen Blätter bestimmt. Die Resistenz der transformierten Pflanzen wird durch einen statistisch signifikanten (Student's t Test p<0,05) Abfall im Larvengewicht, Larvenüberleben und in der Menge der gefressenen Blätter bestimmt, indem die Blätter von Pflanzen, in denen das Kunitz Trypsin-Inhibitor-Gen exprimiert wird, mit denen von Kontrollpflanzen, die mit dem Vektor ohne Geneinschub transformiert sind, oder mit denen von nichttransformierten Pflanzen verglichen werden.

Beispiel 39: Tabak, der durch die Expression des Kunitz Trypsin-Inhibitors aus Sojabohnen resistent gegen Schaden durch Lepidopteren-Larven ist

A. Konstruktion des Vektors. Der pCIB10-KTI Vektor wird wie in Beispiel 37A beschrieben hergestellt.

B. Transformation und Regenerierung von Pflanzen. Transformierte Pflanzen werden wie in Beispiel 6 beschrieben hergestellt, indem der pCIB10-KTI Vektor oder der pCIB715/710-KTI Vektor (Beispiel 36A) in *Agrobacterium* verwendet wird.

C. Test von Pflanzen auf die Expression des Trypsin-Inhibitors. Der Test der Transformanten auf die Expression des Kunitz Trypsin-Inhibitors aus Sojabohnen wird wie in Beispiel 36C beschrieben durchgeführt.

(D) Resistenz von transformierten Pflanzen gegen Schaden durch Lepidopteren. Blattscheiben von 4 Wochen alten transformierten Pflanzen werden an frischgeschlüpfte *Heliothis zea* oder *Manduca sexta* verfüttert. Frischgeschlüpfte Larven werden in einzelne Fütterungsbecher mit einem 1 cm$^2$ Blattstück gesetzt. 50 Insekten werden pro Gruppe getestet. Nach 5 Tagen werden das Insektengewicht, das Insektenüberleben und die Menge der gefressenen Blätter bestimmt. Die Resistenz der transformierten Pflanzen wird durch einen statistisch signifikanten (Student's t Test p < 0,05) Abfall im Larvengewicht, Larvenüberleben und in der Menge der gefressenen Blätter bestimmt, indem die Blätter von Pflanzen, in denen das Kunitz Trypsin-Inhibitor-Gen exprimiert wird, mit denen von Kontrollpflanzen, die mit dem Vektor ohne Geneinschub transformiert sind, oder mit denen von nichttransformierten Pflanzen verglichen werden.

Beispiel 40: Knäuelgras (*Dactylis glomerata*), das durch die Expression des Kunitz Trypsin-Inhibitors aus Sojabohnen resistent gegen Schaden durch Lepidopteren Larven ist

A. Konstruktion des Vektors. pCIB710-KTI und pCIB715/710-KTI werden wie in Beispiel 36A beschrieben hergestellt.

B. Transformation und Regenerierung von Pflanzen. Transformierte Pflanzen werden wie in den Beispielen 24 bis 28 beschrieben erhalten, indem pCIB710-KTI und pCIB715/710-KTI verwendet werden.

C. Test von Pflanzen auf die Expression des Trypsin-Inhibitors. Der Test von Transformanten auf die Expression des Kunitz Trypsin-Inhibitors aus Sojabohnen wird wie in Beispiel 36C beschrieben durchgeführt.

D. Resistenz von transformierten Pflanzen gegen Schaden durch Lepidopteren-Larven. Aus T1 Samen erhaltene Keimlinge werden in feine Erde in 100 mm Petrischalen (10 pro Schale, 5 Schalen) gepflanzt. Wenn die zweiten Blätter am Keimling erscheinen, wird jede Schale mit 20 Larven von *Crambus caliginosellus* im 2. Häutungsstadium infiziert. Nach 7 Tagen werden die Anzahl und das Gewicht der Ueberlebenden zusammen mit dem Gewicht der gewaschenen Graswurzeln bestimmt. Die Resistenz transformierter Pflanzen wird durch einen statistisch signifikanten (Student's t Test $p < 0.05$) Abfall in der larvalen Gewichtszunahme, Abfall in der larvalen Ueberlebensrate oder in einem Abfall im Verlust von Wurzelgewicht bestimmt, relativ zu Insekten-freien Pflanzen, indem die Pflanzen, die das Kunitz Trypsin-Inhibitor-Gen aus Sojabohnen exprimieren, mit Kontrollpflanzen, die mit dem Vektor ohne Geneinschub transformiert sind, oder mit nichttransformierten Pflanzen verglichen werden.

Beispiel 41: Mais, der durch die Expression von $\alpha_1$-Antitrypsin resistent gegen Schaden durch Lepidopterenlarven ist

(A) Konstruktion des pCIB715/710-AATI Vektors. Die DNA-Sequenzen, die $\alpha_1$-Antitrypsin kodieren, werden gemäss dem Verfahren von Rosenberg et al. (1984) erhalten. Für die Insertion in die BamHI-Schnittstelle im pCIB710 Vektor wird das DNA-Fragment mit geeigneten Enzymen geschnitten und die Oligonukleotide, die nötig sind, um kompatible Enden zu schaffen, werden mit pCIB710, das mit BamHI geschnitten wurde, verknüpft, indem das oben beschriebene Verfahren angewendet wird. Der resultierende Vektor wird pCIB710-AATI genannt. Dieser Vektor wird mit EcoRI geschnitten und die resultierende DNA wird mit alkalischer Phosphatase behandelt. Plasmid pCIB715 (Rothstein et al., 1987) wird mit EcoRI geschnitten und mit dem oben beschriebenen pCIB710-AATI verknüpft. Der resultierende Vektor wird pCIB715/710-AATI benannt.

(B) Transformation und Regenerierung von Mais. Maisgewebe wird mit dem pCIB710 Vektor oder bevorzugt mit dem pCIB715/710 Vektor, der den $\alpha_1$-Antitrypsin-Geneinschub enthält, transformiert und Pflanzen werden wie in den Beispielen 16 bis 20 beschrieben regeneriert. Zur Kontrolle werden Pflanzen auf dieselbe Weise hergestellt, jedoch mit dem pCIB710 Vektor oder dem pCIB715 Vektor ohne Geneinschub. Transformiertes Pflanzengewebe wird auf Grund der Empfindlichkeit gegenüber dem entsprechenden Antibiotikum selektioniert. Die Anfangspflanzen werden selbstbestäubt und Samen (T1 Samen) wird erhalten.

(C) Test von Pflanzen auf $\alpha_1$-Antitrypsin-Expression. Pflanzen, die aus T1 Samen gezogen worden sind, werden auf das Vorhandensein und die Expression des $\alpha_1$-Antitrypsin-Gens analysiert, indem die folgenden Testverfahren angewendet werden.

(1) DNA wird isoliert und mit BamHI geschnitten; die Abbauprodukte werden auf einem 1,5 % Agarosegel elektrophoretisch aufgetrennt. Die DNA-Fragmente werden auf Nitrocellulose übertragen und mit dem $\alpha_1$-Antitrypsin-Gen hybridisiert, das durch nick-Translation mit $^{32}$P markiert ist. Das Vorhandensein des $\alpha_1$-Antitrypsin-Gens wird durch eine Bande, die etwa 1200 Bp entspricht und mit der Probe hybridisiert, ausfindig gemacht.
(2) RNA wird durch das Northern Blot Verfahren als eine Bande ausfindig gemacht, die etwa 1200 Basen entspricht und mit dem $^{32}$P-$\alpha_1$-Antitrypsin-Gen hybridisiert das oben beschrieben ist.
(3) $\alpha_1$-Antitrypsin-Protein wird mit Hilfe immunologischer Standardtests durch einen polyklonalen Antikörper aus Kaninchen nachgewiesen, der gegen kommerziell (Sigma) erhältliches $\alpha_1$-Antitrypsin produziert wurde.
(4) Die Aktivität von $\alpha_1$-Antitrypsin wird bestimmt, indem mit immunologischen Verfahren Material aus Pflanzenextrakten mit Hilfe des polyklonalen Anti-$\alpha_1$-Antitrypsins aus Kaninchen und Protein A Sepharose gereinigt und das isolierte Material auf die Fähigkeit getestet wird, die Proteolyse von $^{14}$C-BSA durch Trypsin zu hemmen, indem der oben beschriebene Test benutzt wird.

D. Resistenz transformierter Pflanzen gegenüber Schaden durch Lepidopteren-Larven. T1 Samen werden gekeimt und Blattstücke, die von Keimlingen im 4-Blattstadium gewonnen werden, werden an frischgeschlüpfte Larven von *Ostrinia nubilalis* oder *Heliothis zea* verfüttert. Frischgeschlüpfte Larven werden in einzelne Fütterungstöpfe mit einem 1 $cm^2$ Blattstück gesetzt. 50 Insekten werden pro Gruppe getestet. Nach 5 Tagen werden das Insektengewicht, das Insektenüberleben und die Menge gefressener Blätter bestimmt. Die Resistenz der transformierten Pflanzen wird durch einen statistisch signifikanten (Student's t Test $p < 0,05$) Abfall im Insektenüberleben, Insektengewicht oder Menge der gefressenen Blätter bestimmt, indem Blätter von Pflanzen, die das $\alpha_1$-Antitrypsin-Gen exprimieren, mit denen von Kontrollpflanzen, die mit dem Vektor ohne Geneinschub transformiert wurden, oder mit denen von nichttransformierten Pflanzen, verglichen werden.

Beispiel 42: Baumwolle, die durch Expression von $\alpha_1$-Antitrypsin resistent gegen Schaden durch Lepidopterenlarven ist

A. Konstruktion des pCIB10-AATI Vektors. Das $\alpha_1$-Antitrypsin-Gen wird zusammen mit dem 35S CaMV Promotor aus pCIB710-AATI (Beispiel 41A) entfernt, indem geeignete Restriktionsenzyme verwendet werden, und mit pCIB10 verknüpft. Dieser Vektor wird als pCIB10-AATI bezeichnet.

B. Transformation und Regenerierung von Pflanzen. Transformierte Pflanzen werden wie in den Beispielen 7 bis 10 beschrieben erhalten, indem pCIB10-AATI oder pCIB715/710-AATI (Beispiel 41A) in *Agrobacterium* verwendet wird.

C. Test von Pflanzen auf Expression von $\alpha_1$-Antitrypsin. Tests von Transformanten auf die Expression von $\alpha_1$-Antitrypsin werden wie in Beispiel 41C beschrieben durchgeführt.

D. Resistenz von transformierten Pflanzen gegen Lepidopteren. Blattscheiben von 4 Wochen alten transformierten Pflanzen werden an frischgeschlüpfte *Heliothis virescens, H. zea* oder *Pectinophora gossypiella* verfüttert. Frischgeschlüpfte Larven werden in einzelne Fütterungsbecher mit einem 1 $cm^2$ Blattstück gesetzt. 50 Insekten werden pro Gruppe getestet. Nach 5 Tagen werden das Insektengewicht, das Insektenüberleben und die Menge der gefressenen Blätter bestimmt. Die Resistenz der transformierten Pflanzen wird durch einen statistisch signifikanten (Student's t Test $p<0,05$) Abfall im Larvengewicht, Larvenüberleben und in der Menge der gefressenen Blätter bestimmt, indem die Blätter von Pflanzen, in denen das $\alpha_1$-Antitrypsin-Gen exprimiert wird, mit denen von Kontrollpflanzen, die mit dem Vektor ohne Geneinschub transformiert sind, oder mit denen von nichttransformierten Pflanzen verglichen werden.

Beispiel 43: Tomaten, die durch die Expression von $\alpha_1$-Antitrypsin resistent gegen Schaden durch Lepidopteren-Larven sind

A. Konstruktion des Vektors. pCIB715/710-AATI und pCIB10-AATI werden wie in den Beispielen 41A und 42A beschrieben hergestellt.

B. Transformation und Regenerierung von Pflanzen. Transformierte Pflanzen werden wie in Beispiel 33B beschrieben hergestellt, indem der pCIB10-AATI Vektor und der pCIB715/710-AATI Vektor verwendet werden.

C. Test von Pflanzen auf Expression von $\alpha_1$-Antitrypsin. Der Test der Transformanten auf die Expression von $\alpha_1$-Antitrypsin wird wie in Beispiel 41C beschrieben durchgeführt.

D. Resistenz von transformierten Pflanzen gegen Schaden durch Lepidopteren. Blattscheiben von 4 Wochen alten transformierten Pflanzen werden an frischgeschlüpfte *Heliothis zea* oder *Manduca sexta* verfüttert. Frischgeschlüpfte Larven werden in einzelne Fütterungsbecher mit einem 1 $cm^2$ Blattstück gesetzt. 50 Insekten werden werden pro Gruppe getestet. Nach 5 Tagen werden das Insektengewicht, das Insektenüberleben und die Menge der gefressenen Blätter bestimmt. Die Resistenz der transformierten Pflanzen wird durch einen statistisch signifikanten (Student's t Test p<0,05) Abfall im Larvengewicht, Larvenüberleben und in der Menge der gefressenen Blätter bestimmt, indem die Blätter von Pflanzen, in denen das $\alpha_1$-Antitrypsin-Gen exprimiert wird, mit denen von Kontrollpflanzen, die mit dem Vektor ohne Geneinschub transformiert sind, oder mit denen von nichttransformierten Pflanzen verglichen werden.

Beispiel 44: Tabak, der durch die Expression von $\alpha_1$-Antitrypsin resistent gegen Schaden durch Lepidopteren-Larven ist.

A. Konstruktion des Vektors. pCIB715/710-AATI und pCIB10-AATI werden wie in den Beispielen 41A und 42A beschrieben hergestellt.

B. Transformation und Regenerierung von Pflanzen. Transformierte Pflanzen werden wie in Beispiel 6 beschrieben hergestellt, indem der pCIB10-AATI Vektor oder der pCIB715/710-AATI-Vektor (Beispiel 41A) in *Agrobacterium* verwendet wird.

C. Test von Pflanzen auf Expression von $\alpha_1$-Antitrypsin. Der Test der Transformanten auf die Expression von $\alpha_1$-Antitrypsin wird wie in Beispiel 41C beschrieben durchgeführt.

D. Resistenz von transformierten Pflanzen gegen Schaden durch Lepidopteren. Blattscheiben von 4 Wochen alten transformierten Pflanzen werden an frischgeschlüpfte *Heliothis zea* oder *Manduca sexta* verfüttert. Frischgeschlüpfte Larven werden in einzelne Fütterungsbecher mit einem 1 cm$^2$ Blattstück gesetzt. 50 Insekten werden pro Gruppe getestet. Nach 5 Tagen werden das Insektengewicht, das Insektenüberleben und die Menge der gefressenen Blätter bestimmt. Die Resistenz transformierten Pflanzen wird durch einen statistisch signifikanten (Student's t Test p < 0,05) Abfall im Larvengewicht, Larvenüberleben und in der Menge der gefressenen Blätter bestimmt, indem die Blätter von Pflanzen, in denen das $\alpha_1$-Antitrypsin-Gen exprimiert wird, mit denen von Kontrollpflanzen, die mit dem Vektor ohne Geneinschub transformiert sind, oder mit denen von nichttransformierten Pflanzen verglichen werden.

Beispiel 45: Knäuelgras (*Dactylis glomerata*), das durch die Expression von $\alpha_1$-Antitrypsin resistent gegen Schaden durch Lepidopteren-Larven ist.

A. Konstruktion des Vektors. pCIB710-AATI und pCIB715/710-AATI werden wie in Beispiel 41A hergestellt.

B. Transformation und Regeneration von Pflanzen. Transformierte Pflanzen werden wie in den Beispielen 24 bis 28 beschrieben erhalten, indem pCIB710-AATI und pCIB715/710-AATI verwendet werden.

C. Test von Pflanzen auf Expression von $\alpha_1$-Antitrypsin. Der Test von Transformanten auf die Expression von $\alpha_1$-Antitrypsin wird wie in Beispiel 41C beschrieben durchgeführt.

D. Resistenz von transformierten Pflanzen gegen Schaden durch Lepidopteren-Larven. Aus T1 Samen erhaltene Keimlinge werden in feine Erde in 100 mm Petrischalen (10 Schale, 5 Schalen) gepflanzt. Wenn die zweiten Blätter am Keimling erscheinen, wird jede Schale mit 20 Larven von *Crambus caliginosellus* im 2. Häutungsstadium infiziert. Nach 7 Tagen werden die Anzahl und das Gewicht der Ueberlebenden zusammen mit dem Gewicht der gewaschenen Graswurzeln bestimmt. Die Resistenz transformierter Pflanzen wird durch einen statistisch signifikanten (Student's t Test p < 0.05) Abfall in der larvelen Gewichtszunahme, Abfall in der larvalen Ueberlebensrate oder in einem Abfall im Verlust von Wurzelgewicht bestimmt, relativ zu Insekten-freien Pflanzen, indem die Pflanzen, die das $\alpha_1$-Antitrypsin-Gen exprimieren, mit Kontrollpflanzen, die mit dem Vektor ohne Geneinschub transformiert sind, oder mit nichttransformierten Pflanzen verglichen werden.

Beispiel 46: Mais, der durch die Expression von Eglin resistent gegen Schaden durch Lepidopteren-Larven ist

A. Konstruktion des pCIB715/710-Eglin-Vektors. Die DNA-Sequenzen, die Eglin C und Eglin C-Mutanten kodieren, werden wie in Beispiel 3 D beschrieben erhalten. Zur Insertion in die BamHI-Schnittstelle des pCIB710 Vektors wird das DNA-Fragment mit geeigneten Enzymen geschnitten und die synthetischen Oligonukleotide, die nötig sind, um kompatible Enden zu erzeugen, werden angeknüpft, zusammen mit BamHI geschnittenem pCIB710, indem die oben beschriebene Methode verwendet wird. Der resultierende von der Eglin C (Arg45) Mutante abstammende Vektor wird als pCIB710-Eglin bezeichnet. Dieser Vektor wird mit EcoRI geschnitten und die resultierende DNA mit alkalischer Phosphatase behandelt. Das Plasmid pCIB715 (Rothstein et al., 1987) wird mit EcoRI geschnitten und mit dem CIB710-Eglin von oben verknüpft. Der resultierende Vektor wird pCIB715/710-Eglin genannt.

B. Transformation und Regenerierung von Mais. Maisgewebe wird mit dem pCIB710 Vektor oder bevorzugt mit dem pCIB715/710 Vektor, der den Eglin C (Arg45)-Geneinschub enthält, transformiert und Pflanzen werden wie in den Beispielen 16 bis 20 beschrieben regeneriert. Zur Kontrolle werden Pflanzen auf dieselbe Weise hergestellt, jedoch mit dem pCIB710 Vektor oder dem pCIB715 Vektor ohne Geneinschub. Transformiertes Pflanzengewebe wird auf Grund der Empfindlichkeit gegenüber dem entsprechenden Antibiotikum selektioniert. Die Anfangspflanzen werden selbstbestäubt und Samen (T1 Samen) wird erhalten.

C. Test von Pflanzen auf die Eglin-Expression. Pflanzen, die aus T1 Samen gezogen wurden, werden auf das Vorhandensein und die Expression des Eglin C (Arg45)-Gens getestet, indem die folgenden Testverfahren angewendet werden.

(1) DNA wird isoliert und mit BamHI geschnitten; die Abbauprodukte werden auf einem 1,5 % Agarosegel elektrophoretisch aufgetrennt. Die DNA-Fragmente werden auf Nitrocellulose übertragen und mit dem Eglin C (Arg45)-Gen hybridisiert, das durch nick-Translation mit $^{32}$P markiert ist. Das Vorhandensein des Eglin-Gens wird durch eine Bande ausfindig gemacht, die etwa 230 Bp entspricht und mit der Probe hybridisiert.
(2) RNA wird durch das Northern Blot Verfahren als eine Bande ausfindig gemacht, die etwa 230 Basen entspricht und mit dem $^{32}$P-Eglin C (Arg45)-Gen hybridisiert, das oben beschrieben ist.
(3) Mutiertes Eglin C (Arg45) Protein wird mit Hilfe immunologischer Standardtests mit einem polyklonalen Antikörper aus Kaninchen nachgewiesen, der gegen Eglin C (Arg45) produziert wurde.
(4) Die Eglin-Aktivität wird bestimmt, indem mit immunologischen Verfahren Material aus Pflanzenextrakten mit Hilfe des polyklonalen Anti-Eglin C (Arg45)-Antikörpers aus Kaninchen und Protein A Sepharose gereinigt und anschliessend das isolierte Material auf die Fähigkeit getestet wird, die Proteolyse von $^{14}$C-BSA durch Trypsin zu hemmen, indem der oben beschriebene Test benutzt wird.

D. Resistenz transformierter Pflanzen gegenüber Schaden durch Lepidopteren-Larven. T1 Samen werden gekeimt und Blattstücke, die von Keimlingen im 4-Blattstadium gewonnen werden, werden an frischgeschlüpfte Larven von *Ostrinia nubilalis* oder *Heliothis zea* verfüttert. Frischgeschlüpfte Larven werden in einzelne Fütterungstöpfe mit einem 1 cm$^2$ Blattstück gesetzt. 50 Insekten werden pro Gruppe getestet. Nach 5 Tagen werden das Insektengewicht, das Insektenüberleben und die Menge gefressener Blätter bestimmt. Die Resistenz der transformierten Pflanzen wird durch einen statistisch signifikanten (Student's t Test $p<0,05$) Abfall im Insektenüberleben, im Insektengewicht oder in der Menge der gefressenen Blätter bestimmt, indem Blätter von Pflanzen, die das Eglin C-(Arg45)-Gen exprimieren, mit denen von Kontrollpflanzen, die mit dem Vektor ohne Geneinschub transformiert wurden, oder mit denen von nichttransformierten Pflanzen verglichen werden.

Beispiel 47: Baumwolle, die durch Expression von Eglin resistent gegen Schaden durch Lepidopterenlarven ist

A. Konstruktion des pCIB10-Eglin Vektors. Das Eglin C (Arg45)-Gen wird zusammen mit dem 35S CaMV Promotor aus pCIB710-Eglin (Beispiel 46A) entfernt, indem geeignete Restriktonsenzyme verwendet werden, und mit pCIB10 verknüpft. Dieser Vektor wird als pCIB10-Eglin bezeichnet.

B. Transformation und Regenerierung von Pflanzen. Transformierte Pflanzen werden wie in den Beispielen 7 bis 10 beschrieben erhalten, indem pCIB10-Eglin oder pCIB715/710 Eglin (Beispiel 46A) in *Agrobacterium* verwendet werden.

C. Test von Pflanzen auf die Eglin-Expression. Tests von Transformanten auf die Eglin-Expression werden wie in Beispiel 46C beschrieben durchgeführt.

D. Resistenz von transformierten Pflanzen gegen Lepidopteren. Blattscheiben von 4 Wochen alten transformierten Pflanzen werden an frischgeschlüpfte *Heliothis virescens, H. zea* oder *Pectinophora gossypiella* verfüttert. Frischgeschlüpfte Larven werden in einzelne Fütterungsbecher mit einem 1 cm$^2$ Blattstück gesetzt. 50 Insekten werden pro Gruppe getestet. Nach 5 Tagen werden das Insektengewicht, das Insektenüberleben und die Menge der gefressenen Blätter bestimmt. Die Resistenz der transformierten Pflanzen wird durch einen statistisch signifikanten (Student's t Test $p < 0,05$) Abfall im Larvengewicht, Larvenüberleben und in der Menge der gefressenen Blätter bestimmt, indem die Blätter von Pflanzen, in denen das Eglin C (Arg45)-Gen exprimiert wird, mit denen von Kontrollpflanzen, die mit dem Vektor ohne Geneinschub transformiert sind, oder mit denen von nichttransformierten Pflanzen verglichen werden.

Beispiel 48: Tomaten, die durch die Eglin-Expression resistent gegen Schaden durch Lepidopteren-Larven sind

A. Konstruktion des Vektors. pCIB715/710-Eglin und pCIB10-Eglin werden wie in den Beispielen 46A und 47A beschrieben hergestellt.

B. Transformation und Regenerierung von Pflanzen. Transformierte Pflanzen werden wie in Beispiel 33B beschrieben hergestellt, indem der pCIB10-Eglin Vektor oder der pCIB715/710-Eglin Vektor verwendet wird.

C. Test von Pflanzen auf die Eglin Expression. Der Test der Transformanten auf die Eglin-Expression wird wie in Beispiel 46C beschrieben durchgeführt.

D. Resistenz von transformierten Pflanzen gegen Schaden durch Lepidopteren. Blattscheiben von 4 Wochen alten transformierten Pflanzen werden an frischgeschlüpfte *Heliothis zea* oder *Manduca sexta* vefüttert. Frischgeschlüpfte Larven werden in einzelne Fütterungsbecher mit einem 1 cm$^2$ Blattstück gesetzt. 50 Insekten werden pro Gruppe getestet. Nach 5 Tagen wird das Insektengewicht, das Insektenüberleben und die Menge der gefressenen Blätter bestimmt. Die Resistenz der transformierten Pflanzen wird durch einen statistisch signifikanten (Student's t Test $p < 0,05$) Abfall im Larvengewicht, Larvenüberleben und in der Menge der gefressenen Blätter bestimmt, indem die Blätter von Pflanzen, in denen das Eglin C (Arg45)-Gen exprimiert wird, mit denen von Kontrollpflanzen, die mit dem Vektor ohne Geneinschub transformiert sind, oder mit denen von nichttransformierten Pflanzen verglichen werden.

Beispiel 49: Tabak, der durch die Eglin-Expression resistent gegen Schaden durch Lepidopteren-Larven ist

A. Konstruktion des Vektors. pCIB715/710-Eglin und pCIB10-Eglin werden wie in den Beispielen 46A und 47A beschrieben hergestellt.

B. Transformation und Regenerierung von Pflanzen. Transformierte Pflanzen werden wie in Beispiel 6 beschrieben hergestellt, indem der pCIB10-Eglin Vektor oder der pCIB715/710-Eglin Vektor (Beispiel 46A) in *Agrobacterium* verwendet wird.

C. Test von Pflanzen auf die Eglin-Expression. Der Test der Transformanten auf die Expression wird wie in Beispiel 46C beschrieben durchgeführt.

D. Resistenz von transformierten Pflanzen gegen Schaden durch Lepidopteren. Blattscheiben von 4 Wochen alten transformierten Pflanzen werden an frischgeschlüpfte *Heliothis zea* oder *Manduca sexta* vefüttert. Frischgeschlüpfte Larven werden in einzelne Fütterungsbecher mit einem 1 cm$^2$ Blattstück gesetzt. 50 Insekten werden pro Gruppe getestet. Nach 5 Tagen werden das Insektengewicht, das Insektenüberleben und die Menge der gefressenen Blätter bestimmt. Die Resistenz der transformierten Pflanzen wird durch einen statistisch signifikanten (Student's t Test ; $< 0,05$) Abfall im Larvengewicht, Larvenüberleben und in der Menge der gefressenen Blätter bestimmt, indem die Blätter von Pflanzen, in denen das Eglin C (Arg45)-Gen exprimiert wird, mit denen von Kontrollpflanzen, die mit dem Vektor ohne Geneinschub transformiert sind, oder mit denen von nichttransformierten Pflanzen verglichen werden.

Beispiel 50: Knäuelgras (*Dactylis glomerata*), das durch die Expression von Eglin resistent gegen Schaden durch Lepidopteren-Larven ist.

A. Konstruktion des Vektors. pCIB710-Eglin und pCIB715/710-Eglin werden wie in Beispiel 46A beschrieben hergestellt.

B. Transformation und Regenerierung von Pflanzen. Transformierte Pflanzen werden wie in den Beispielen 24 bis 28 beschrieben erhalten, indem pCIB710-Elgin und pCIB715/710-Eglin verwendet werden.

C. Test von Pflanzen auf die Expression von Eglin. Der Test von Transformanten auf die Expression von Eglin wird wie in Beispiel 46C beschrieben durchgeführt.

D. Resistenz von transformierten Pflanzen gegen Schaden durch Lepidopteren-Larven. Aus T1 Samen erhaltene Keimlinge werden in feine Erde in 100 mm Petrischalen (10 pro Schale, 5 Schalen) gepflanzt. Wenn die zweiten Blätter am Keimling erscheinen, wird jede Schale mit 20 Larven von *Crambus caliginosellus* im 2. Häutungsstadium infiziert. Nach 7 Tagen werden die Anzahl und das Gewicht der Ueberlebenden zusammen mit dem Gewicht der gewaschenen Graswurzeln bestimmt. Die Resistenz transformierter Pflanzen wird durch einen statistisch signifikanten (Student's t Test $p < 0.05$) Abfall in der larvalen Gewichtszunahme, Abfall in der larvalen Ueberlebensrate oder in einem Abfall im Verlust von Wurzelgewicht bestimmt, relativ zu Insekten-Freien Pflanzen, indem die Pflanzen, die das Eglin C (Arg45)-Gen exprimieren, mit Kontrollpflanzen, die mit dem Vektor ohne Geneinschub transformiert sind, oder mit nichttransformierten Pflanzen verglichen werden.

## Tabelle A

### PFLANZENKLASSIFIKATION NACH DER VERWENDUNG

**GETREIDE**

<u>Monokotyledonen</u>

| | |
|---|---|
| *Avena nuda (chinensis)* | Rauhhafer |
| *A. sativa* | Hafer |
| *Eleusine coracan* | Fingerhirse |
| *Eragrostis tef* | Zwerghirse |
| *Hordeum distichum* | Gerste |
| *H. vulgare* | |
| *Oryza sativa* | Reis |
| *Panicum italicium* | |
| *P. miliaceum* | Rispenhirse |
| *Pennisetum glaucum* | Rohrkolbenhirse |
| *P. spicatum (americanum)* | Perlhirse |
| *Secale cereale* | Roggen |
| *Sorghum vulgare* | Mohrenhirse |
| *X Triticosecale* | Triticale |
| *Triticum aestivum* | Weizen |
| *T. dicoccum* | Emmerweizen |
| *T. durum* | Hartweizen |
| *T. monococcum* | Einkornweizen |
| *Zea mays* | Mais |

<u>Dikotyledonen</u>

| | |
|---|---|
| *Amaranthus paniculatus* | Rispenfuchsschwanz |
| *Fagopyrum esculentum* | Buchweizen |
| *F. tataricum* | |

**PROTEIN-LIEFERNDE PFLANZEN**
<u>Dikotyledonen</u>

| | |
|---|---|
| *Arachis hypogea* | Erdnuss |
| *Cajanus indicus* | Erbsenbohne |
| *Cicer arietinum* | Kichererbse |
| *Dolichos lablab* | Helmbohne |
| *Glycine gracilis* | |
| *G. max* | Sojabohne |
| *G. ussuriensis* | |
| *Lathyrus sativus* | Saatplatterbse |
| *Lens culinaris* | Linse |
| *Mucuna pruriens* | |
| *Phaseolus acutifolius* | Teparybohne |
| *P. aureus* | |
| *P. lunatus* | Limabohne |
| *P. coccineus* | Feuerbohne |
| *(multiflorus)* | |
| *P. mungo* | Mungobohne |

| | |
|---|---|
| *P. vulgaris* | Stangenbohne |
| *Vicia faba* | Saubohne |
| *Vigna angularis* | |
| *V. sesquipedalis* | |
| *V. sinensis* | Kuhbohne |

**OBST-LIEFERNDE PFLANZEN**

<u>Dikotyledonen</u>

| | |
|---|---|
| *Amygdalus communis* | Mandelbaum |
| *Ananas comosus* | Ananas |
| *Artocarpus communis* | Brotfruchtbaum |
| *Carica papaya* | Melonenbaum |
| *Citrullus vulgaris* | Wassermelone |
| *Citrus grandis* | Pampelmuse |
| *C. medica* | Zitrone |
| *C. nobilis* | Tangerine |
| *C. reticulata* | Mandarine |
| *C. sinensis* | Orange |
| *Cydonia oblonga* | Quitte |
| *Diospyros kaki* | Kakipflaume |
| *Ficus carica* | Feige |
| *Fragaria chiloensis* | Erdbeere |
| *F. virginiana* | |
| *Litchi chinensis* | Litchi |
| *Malus asiatica* | |
| *M. pumila* | Apfel |
| *Mangifera indica* | Mangobaum |
| *Morus rubra* | Maulbeerbaum |
| *Musa cavendishii* | Banane |
| *M. paradisiaca* | |
| *Passiflora edulis* | Passionsblume |
| *P. ligularis* | |
| *Persea americana* | Avocadobirne |
| *Phoenix dactylifera* | Dattelpalme |
| *Prunus armeniaca* | Aprikose |
| *P. avium* | Süsskirsche |
| *P. cerasifera* | Kirschpflaume |
| *(divaricata)* | |
| *P. cerasus* | Sauerkirsche |
| *P. domestica* | Zwetschge |
| *P. maheleb* | |
| *P. persica* | Pfirsich |
| *P. pseudocerasus* | |
| *P. salicinia* | |
| *P. serotina* | |
| *Psidium guajava* | Guajave |
| *Punica granatum* | Granatapfelbaum |
| *Pyrus communis* | Birne |
| *P. ussuriensis* | |
| *Ribes grossularia* | Stachelbeere |
| *R. nigrum* | Schwarze Johannisbeere |
| *R. rubrum* | Rote Johannisbeere |
| *Rubus idaeus* | |
| *R. strigosus* | Himbeere |
| *Tamarindus indica* | |
| *Vaccinium angustifolium* | |

| | |
|---|---|
| *V. ashei* | |
| *V. corymbosum* | Kulturheidelbeere |
| *V. myrtillus* | Heidelbeere |
| *V. oxycoccos* | Moosbeeren |
| *Viburnum trilobum* | |
| *Vitis labrusca* | Weinrebe |
| *V. vinifera* | |

## GEMUESE- UND KNOLLEN-LIEFERNDE PFLANZEN
### Monokotyledonen

| | |
|---|---|
| *Allium ascalonicum* | Schalotte |
| *A. cepa* | Küchenzwiebel |
| *A. chinense* | |
| *A. fistulosum* | Winterzwiebel |
| *A. porrum* | Porree |
| *A. sativum* | Knoblauch |
| *A. schoenoprasum* | Schnittlauch |
| *Asparagus officinalis* | Spargel |
| *Zea mays* | Mais |

### Dikotyledonen

| | |
|---|---|
| *Amoracia lapathifolia* | |
| *Apium graveolens* | Sellerie |
| *Arabidopsis thaliana* | |
| *Beta vulgaris* | Rübe |
| *Brassica alboglabra* | |
| *B. campestris* | |
| *B. carinata* | |
| *B. cernua* | Chinesischer Senf |
| *B. chinensis* | Chinakohl |
| *B. hirta* | |
| *B. juncea* | Rumänischer Sarepta-senf |
| *B. kaber* | |
| *B. napobrassica* | Steckrübe |
| *B. napus* | Raps |
| *B. nigra* | Schwarzer Senf |
| *B. oleracea* | Kohl |
| *B. pekinensis* | Pekingkohl |
| *B. rapa* | Weisse Rübe |
| *Cajanus cajan (indicus)* | |
| *Canavalia ensiformis* | Jackbohne |
| *Canna edulis* | Blumenrohr |
| *Capsicum annuum* | Gemüsepaprika |
| *C. chinense* | Pfeffer |
| *C. frutescens* | Cayennepfeffer |
| *C. pendulum* | |
| *C. pubescens* | |
| *Cichorium endivia* | Endivie |
| *C. intybus* | Wurzelzichorie |
| *Colocasia antiquorum* | Taro |
| *Crambe maritima* | Meerkohl |
| *Cucumis melo* | Melone |
| *C. sativus* | Gurke |
| *Cucurbita ficifolia* | |
| *C. foetidissima* | |

| | |
|---|---|
| *C. maxima* | Riesenkürbis |
| *C. moschata* | Moschuskürbis |
| *C. pepo* | Gartenkürbis |
| *Cynara scolymus* | Artischocke |
| *Daucus carota* | Möhre |
| *Dioscorea alata* | Wasseryam |
| *D. batatas* | Brotwurzel |
| *D. cayennensis* | Gelber Yam |
| *Eruca sativa* Mill. | Oelranke |
| *Ipomea batatas* | Süsskartoffel |
| *Lactuca sativa* | Salat |
| *Lepidium sativum* | Gartenkresse |
| *Lycopersicon cerasiforme* | Kirschtomate |
| *L. esculentum* | Tomate |
| *Mahihot utilissima* | Maniok |
| *Nasturtium officinale* | Brunnenkresse |
| *Pastinaca sativa* | Pastinak |
| *Petroselinum crispum* (*sativum*) | Petersilie |
| *Physalis peruviana* | Kapstachelbeere |
| *Pisum sativum* | Gartenerbse |
| *Raphanus sativus* | Rettich |
| *Rheum officinale* | |
| *R. rhaponticum* | Rhabarber |
| *Scorzonera hispanica* | Schwarzwurzel |
| *Sechium edule* | Chayote |
| *Solanum andigenum* | |
| *S. melongena* | Aubergine |
| *S. muricatum* | |
| *S. phureja* | |
| *S. tuberosum* | Kartoffel |
| *Spinacia oleracea* | Spinat |

**NUESSE-LIEFERNDE PFLANZEN**
<u>Dikotyledonen</u>

| | |
|---|---|
| *Anacardium occidentale* | Cashewnuss |
| *Arachis hypogaea* | Erdnuss |
| *Carya illinoinensis* | Pekannussbaum |
| *C. ovata* | |
| *Castanea sativa* | Esskastanie |
| *Cocos nucifera* | Kokospalme |
| *Corylus americana* | |
| *C. avellana* | Haselnuss |
| *Juglans nigra* | Schwarznuss |
| *J. regia* | Walnuss |
| *J. sinensis* | |
| *Litchi chinensis* | |
| *Macadamia integrifolia* | |
| *Pistacia vera* | Pistazie |
| *Prunus amygdalus* | |

**OELPFLANZEN (SPEISE- ODER PFLANZENOELE)**
<u>Monokotyledonen</u>

| | |
|---|---|
| *Zea mays* | Mais |

Dikotyledonen

| | |
|---|---|
| *Aleurites cordata* | Tungölbaum |
| *A. moluccana (triloba)* | |
| *Arachis hypogea* | |
| *Brassica campestris* | |
| *B. napus* | |
| *Cannabis sativa* | Hanf |
| *Carthamus tinctorius* | Färberdistel |
| *Cocos nucifera* | |
| *Elaeis guineensis* | Olpalme |
| *Glycine gracilus* | |
| *G. max* | |
| *G. ussuriensis* | |
| *Gossypium hirsutum* | Baumwolle |
| *Helianthus annus* | Sonnenblume |
| *Linum usitatissimum* | Flachs |
| *Olea europaea* | Oelbaum |
| *Papaver somniferum* | Mohn |
| *Ricinus communis* | Rizinus |
| *Sesamum indicum* | Sesam |

**ZUCKERPFLANZEN**

Monokotyledonen

| | |
|---|---|
| *Saccharum officinarum* | Zuckerrohr |
| *(officinarum* x | |
| *spontaneum)* | |
| *S. robustum* | |
| *S. sinense* | |
| *S. spontaneum* | |
| *Sorghum dochna* | Zuckerhirse |

Dikotyledonen

| | |
|---|---|
| *Acer saccharum* | Zuckerahorn |
| *Beta vulgaris* | Zuckerrübe |

**FUTTER- UND RASENGRAESER**

Monokotyledonen

| | |
|---|---|
| *Agropyron cristatum* | |
| *A. desertorum* | |
| *A. elongatum* | |
| *A. intermedium* | |
| *A. smithii* | |
| *A. spicatum* | |
| *A. trachycaulum* | |
| *A. trichophorum* | |
| *Alopecurus pratensis* | Wiesenfuchsschwanz |
| *Andropogon gerardi* | |
| *Arrhenatherum elatius* | Glatthafer |
| *Bothriochloa barbinodis* | |
| *B. ischaemum* | |
| *B. saccharoides* | |
| *Bouteloua curipendula* | |
| *B. eriopoda* | |
| *B. gracilis* | |
| *Bromus erectus* | |
| *B. inermis* | |

| | |
|---|---|
| *B. riparius* | |
| *Cenchrus ciliaris* | |
| *Chloris gayana* | Rhodesgras |
| *Cymbopogon nardus* | |
| *Cynodon dactylon* | Bermudagras |
| *Dactylis glomerata* | Knäuelgras |
| *Dichanthium annulatum* | |
| *D. aristatum* | |
| *D. sericeum* | |
| *Digitaria decumbens* | Pangolagras |
| *D. smutsii* | |
| *Elymus angustus* | |
| *E. junceus* | |
| *Eragrostis curvula* | |
| *Festuca arundinacea* | |
| *F. ovina* | |
| *F. pratensis* | Wiesenschwingel |
| *F. rubra* | Rotschwingel |
| *Lolium multiflorum* | Welsches Weidelgras |
| *L. perenne* | Deutsches Weidelgras |
| *Panicum maximum* | Guineagras |
| *P. purpurascens* | |
| *P. virgatum* | |
| *Paspalum dilatatum* | Dallisgras |
| *P. notatum* | Bahiagras |
| *Pennisetum clandestinum* | Kikuyugras |
| *P. purpureum* | Elefantengras |
| *Phalaris arundinacea* | Rohrglanzgras |
| *Phleum bertolinii* | |
| *P. pratense* | Wiesenlieschgras |
| *Poa fendleriana* | |
| *P. nemoralis* | |
| *P. pratensis* | Wiesenrispengras |
| *Setaria sphacelata* | Timothygras |
| *Sorghastrum nutans* | |
| *Sorghum halepense* | Johnsongras |
| *S. sudanense* | Sudangras |
| *Sorghum vulgare* | |

**FUTTERLEGUMINOSEN**

Dikotyledonen

| | |
|---|---|
| *Coronilla varia* | |
| *Crotalaria juncea* | Sunnhanf |
| *Lespedeza stipulacea* | Koreanischer Klee |
| *L. striata* | Japanischer Klee |
| *L. sericea* | |
| *Lotus corniculatus* | Hornklee |
| *L. uliginosus* | |
| *Lupinus albus* | Weisse Lupine |
| *L. angustifolius* | Blaue Lupine |
| *L. luteus* | Gelbe Lupine |
| *L. mutabilis* | |
| *Medicago arabica* | Klettenklee |
| *M. arborea* | |
| *M. falcata* | Sichelluzerne |
| *M. hispida* | |

| | |
|---|---|
| *M. sativa* | Luzerne |
| *M. tribuloides* | |
| *Melilotus albus* | Weisser Steinklee |
| *M. officinalis* | Gelber Steinklee |
| *Onobrychis viciifolia* | Esparsette |
| *Ornithopus sativus* | Serradella |
| *Pueraria thunbergiana* | Kudzu |
| *Trifolium alexandrinum* | Alexandrinerklee |
| *T. augustifolium* | |
| *T. diffusum* | |
| *T. hybridum* | Schwedenklee |
| *T. incarnatum* | Blutroter Inkarnatklee |
| *T. ingrescens* | |
| *T. pratense* | Rotklee |
| *T. repens* | Weissklee |
| *T. resupinatum* | Persischer Klee |
| *T. subterraneum* | Erdfrüchtiger Klee |
| *Trigonella foenum-graecum* | Bockshornklee |
| *Vicia sativa* | Futterwicke |
| *V. villosa* | Zottelwicke |
| *V. atropurpurea* | |
| *V. angustifolia* | |
| *V. dasycarpa* | |
| *V. ervilia* | Linsenwicke |
| *V. pannonica* | Ungarische Wicke |
| *V. calcarata* | |

**FASERN- UND HOLZ-LIEFERNDE PFLANZEN**
Monokotyledonen

| | |
|---|---|
| *Bambusa vulgaris* | Bambus |

Dikotyledonen

| | |
|---|---|
| *Agave sisalana* | |
| *Boehmeria nivea* | Ramiepflanze |
| *Cannabis indica* | Rauschhanf |
| *C. sativa* | |
| *Ceiba pentandra* | Kapokbaum |
| *Corchorus mucronata (striata)* | |
| *Gossypium arboreum* | |
| *G. barbadense* | |
| *G. herbaceum* | |
| *G. hirsutum* | |
| *G. nanking* | |
| *Linum angustifolium* | |
| *L. usitatissimum* | |
| *Musa textiles* | Manilahanf |

**DROGEN-ENTHALTENDE PFLANZEN**
Dikotyledonen

| | |
|---|---|
| *Angelica archangelica* | Engelwurz |
| *Chrysanthemum cinerariifolium* | Dalmatinische Insektenblume |
| *Camellia sinensis* | Teestrauch |
| *C. coccineum* | |

| | |
|---|---|
| *Coffea arabica* | Bergkaffe |
| *C. canephora* | Kongokaffee |
| *Cola acuminata* | Kolabaum |
| *Nicotiana rustica* | Bauerntabak |
| *N. tabacum* | Tabak |
| *Papaver dubium* | |
| *P. somniferum* | Schlafmohn |
| *Theobroma cacao* | Kakao |

**GEWUERZ- UND DUFTPFLANZEN**
Monokotyledonen

| | |
|---|---|
| *Vanilla fragrans* | Vanille |

Dikotyledonen

| | |
|---|---|
| *Artemisa dracunculus* | Estragon |
| *Cinnamomum zeylanicum* | Ceylonzimtbaum |
| *Hibiscus esculentus* | Okra |
| *Salvia officinalis* | Salbei |
| *Thymus vulgaris* | Gartenthymian |
| *Pimpinella anisum* | Anis |
| *Mentha arvensis* | |
| *M. piperita* | Pfefferminze |
| *M. viridis* | |
| *Coriandrum sativum* | Koriander |

## Tabelle B

### Repräsentative transgene Pflanzen, Proteinase Inhibitoren und Zielschädlinge

| Pflanze | Proteinase Inhibitor | Repräsentative Zielschädlinge |
|---|---|---|
| Mais (Monok.) | Cystatin | *Coleoptera:*<br>*Diabrotica*<br>*Melanotus, Agriotes, Limonius, Dalopius, Eleodes*<br>*Chaetocnema*<br>*Macrodactylus*<br>*Sphenophorus*<br>*Sitophilus*<br>*Oulema*<br>*Rhyzopertha, Prostephanus* |
| | Kunitz Trypsin-Inhibitor aus Sojabohnen, $\alpha_1$-Antitrypsin, Eglin C (Arg45) | *Lepidoptera:*<br>*Heliothis*<br>*Ostrinia, Diatraea, Elasmopalpus, Papaipema*<br>*Agrotis, Loxagrotis, Euxoa, Peridroma saucia,*<br>*Chorizagrotis*<br>*Spodoptera, Pseudaletia*<br>*Chilo, Busseola, Sesamia, Eldana*<br>*Sitotroga cerealella*<br>*Plodia interpunctella*<br>*Coleoptera:*<br>*Phyllophaga, Cyclocephala* |

EP 0 348 348 B1

Leupeptin, Antipain

*Coleoptera* und *Lepidoptera* wie oben und
*Thysanoptera:*
*Frankliniella, Anaphothrips, Hercothrips*
*Homoptera:*
*Dalbulus*
*Cicadulina*
*Rhopalosiphum, Melanaphis*
*Anuraphis*
*Prosapia*
*Diptera:*
*Delia platura*
*Euxesta*
*Orthoptera:*
*Melanoplus, Schistocerca, Sphenarium*
*Aneolamia*
*Isoptera:*
*Microtermes, Macrotermes, Allodontermes, Odontotermes*
*Heteroptera:*
*Nezara, Acrosternum, Euschistus*
*Blissus*
*Acarina:*
*Tetranychus, Paratetranychus, Oligonychus*

Rasengras          Cystatin
(Monok.)

*Coleoptera:*
*Popillia*

Kunitz Trypsin Inhibitor
aus Sojabohnen, $\alpha_1$-Antitrypsin,
Eglin C (Arg 45)

*Coleoptera:*
*Phyllophaga, Cyclocephala*
*Lepidoptera*
*Crambus*
*Agrotis, Peridroma saucia, Chorizagrotis*

EP 0 348 348 B1

Leupeptin, Antipain

*Coleoptera* und *Lepidoptera* wie oben

Reis
(Monok.)

Cystatin

*Coleoptera:*
*Sitophilus, Lissorhoptrus*
*Rhyzopertha*

Kunitz Trypsin-Inhibitor aus
Sojabohnen, $\alpha_1$-Antitrypsin,
Eglin C (Arg45)

*Lepidoptera:*
*Chilo, Maliarpha, Scirpophaga, Duataea, Elasmopalpus,*
*Sesamia, Rupela*
*Mythimna*
*Sitroga*

Leupeptin, Antipain

*Coleoptera* und *Lepidoptera* wie oben und
*Homoptera:*
*Nilaparvata, Sogatella, Laodelphax*
*Sogatodes, Nephotettix, Reciian, Cofana, Empoasca*
*Diptera:*
*Diopsis*
*Atherigona, Hydrellia*
*Orseolia*
*Chironomus*
*Thysanoptera:*
*Stenothrips*

Hirse
(Monok.)

Cystatin

*Coleoptera:*
*Diabrotica*
*Melanotus, Agriotes, Dalopius, Eleodes*

Kunitz Trypsin-Inhibitor aus
Sojabohnen, $\alpha_1$-Antitrypsin,
Eglin C (Arg45)

*Lepidoptera:*
*Chilo*
*Sesamia, Diatraea, Busseola*
*Spodoptera*
*Agrotis, Peridroma saucia, Chorizagrotis*
*Heliothis*
*Nola*

EP 0 348 348 B1

| | | |
|---|---|---|
| | Leupeptin, Antipain | *Coleoptera* und *Lepidoptera* wie oben und *Homoptera:* *Poophilus* *Schizaphis, Rhopalosiphum, Melanaphis, Sipha* *Heteroptera:* *Blissus* *Acarina:* *Oligonychus* *Diptera:* *Atherigona* *Contarinia* |
| Baumwolle (Dik.) | Cystatin | *Coleoptera:* *Anthonomus* |
| | Kunitz Trypsin-Inhibitor aus Sojabohnen, $\alpha_1$-Antitrypsin, Eglin C (Arg45) | *Lepidoptera:* *Heliothis, Pectinophora* *Spodoptera* *Acontia* *Trichoplusia* |
| | Leupeptin, Antipain | *Coleoptera* und *Lepidoptera* wie oben und *Acarina:* *Oligonychus* *Heteroptera:* *Euschistus* |
| Sojabohne (Dik.) | Cystatin | *Coleoptera:* *Zabrotes* |
| | Kunitz Trypsin-Inhibitor aus Sojabohnen, $\alpha_1$-Antitrypsin, Eglin C (Arg45) | *Lepidoptera:* *Anticarsia* *Pseudoplusia* *Heliothis* *Spodoptera* |

| | | |
|---|---|---|
| | Leupeptin, Antipain | *Coleoptera* und *Lepidoptera* wie oben |
| Tomate (Dik.) | Cystatin | *Coleoptera:* <br> *Leptinotarsa* |
| | $\alpha_1$-Antitrypsin, Kunitz Trypsin-Inhibitor aus Sojabohnen, Eglin C (Arg45) | *Lepidoptera:* <br> *Manduca* <br> *Heliothis* |
| | Leupeptin, Antipain | *Coleoptera* und *Lepidoptera* wie oben und <br> *Homoptera:* <br> *Paratrioza* <br> *Empoasca, Ophiola, Scleroracus, Macrosteles, Circulifer, Aceratagallia, Agallia* <br> *Myzus* |
| Kartoffel (Dik.) | Cystatin | *Coleoptera:* <br> *Leptinotarsa, Lema* |
| | Leupeptin, Antipain | *Coleoptera* und *Lepidoptera* wie oben und <br> *Homoptera:* <br> *Paratrioza* <br> *Empoasca, Ophiola, Scleroracus, Macrosteles, Circulifer, Aceratagallia, Agallia* <br> *Myzus, Macrosiphum, Aphis* |
| Tabak (Dik.) | Leupeptin, Antipain | *Lepidoptera:* <br> *Manduca* <br> *Heliothis* <br> *Homoptera:* <br> *Aceratagallia, Agallia* <br> *Aphis, Myzus* |

EP 0 348 348 B1

REFERENZEN

An, G., Watson, B.D., Stachel, S., Gordon, M.P., Nester, E.W., Embo J. **4**, 277, 1985

Anastasi, A., Brown, M.A., Kembhavi, A.A., Nicklin, M.H.J., Sayers, C.A., Sunter, D.C., Barrett, A.J., Biochem. J. **211**, 129, 1983

Barrett, A.J., Salvesen, G. (Hrsg.), Proteinase inhibitors, Research monographs in cell and tissue physiology **12**, Elsevier, Amsterdam, 1986

Beasley, C.A., Ting, J.P., Amer. J. Bot. **60**, 130, 1973

Bevan, M., Barnes, W.M., Chilton, M.-D., Nucl. Acids Res. **11**, 369, 1983

Bevan, M., Nucl. Acids Res. **12**, 8711, 1984

de Block, M., Herrera-Estrella, L., van Montagu, M., Schnell, J., Zambryski, P., EMBO J. **3**, 1681, 1984

Christie, B.R. (Hrsg.), CRC Series in Agriculture, CRC Handbook of Plant Science in Agriculture **I**, CRC Press, Boca Raton, Florida, 1987

Chu, C., Wang, C., Sun, C., Hsü, C., Yin, K. Chu, C., Bi, Fi., Scientia Sinica **18**, 659, 1975

Covey, S.N., Lomonossoff, G.P., Hull, R., Nucl. Acids Res. **9**, 6735, 1981

Ditta, G., Stansfield, S., Corbin, D., Helinski, D.R., Proc. Natl. Acad. Sci. USA **77**, 7347, 1980

Fischhoff, D.A., Bowdish, K.S., Perlak, F.J., Marrone, P.G., McCormick, S.M., Niedermeyer, J.G., Dean, D.A., Kusano-Kretzmer, K., Mayer, E.J., Rochester, D.E., Rogers, S.G., Fraley, R.T., Biotechnology **5**, 807, 1987

de Framond, A.J., Barton, K.A., Chilton, M.-D., Biotechnology **1**, 262, 1983

Gamborg, O.L., Miller, R.A., Ojima, K., Exptl. Cell Res. **50**, 151, 1968

Gatehouse, A.M.R., Boulter, D., J. Sci. Food Agric. **34**, 345, 1983

Graves, A.C.F., Goldman, S.L., Plant Mol. Biol. **7**, 43, 1986

Gray, D.J., Conger, B.V., Plant Cell Tissue Organ Culture **4**, 123, 1985

Grimsley, N., Hohn, T., Davies, J.W., Hohn, B., Nature **325**, 177, 1987

Grimsley, N.H., Ramos, C., Hein, T., Hohn, B., Biotechnology **6**, 185, 1988

Gritz, L., Davies, J., Gene **25**, 179, 1983

Guiseley, K.B., Renn, D.W., Agarose, Marine Colloids Division, FMC Corporation, Rockland, Maine, 1975

Hanning, G.E., Conger, B.V., Theor. Appl. Genet. **63**, 155, 1982

Hilder, V.A., Gatehouse, A.M.R., Sheerman, S.E., Barker, R.F., Boulter, D., Nature **330**, 160, 1987

Hoekema, A., Hirsch, P.R., Hooykaws, P.J.J., Schilperoort, R.A., Nature **303**, 179, 1983

Hoffman, L.M., Sengupta-Gopalan, C., Paaren, H.E., Plant Mol. Biol. **3**, 111, 1984

Hohn, T., Richards, K., Lebeurier, G., In: Gene cloning in organisms other than E.coli, Current Topics in Microbiology and Immunology **96**, 193, 1982

Holsters, M., de Waele, D., Depicker, A., Messens, E., van Montagu, M., Schell, J., Mol. Gen. Genet. **163**, 181, 1978

Hood, E.E., Helmer, G.L., Fraley, R.T., Chilton, M.-D., J. Bacteriol. **168**, 1291, 1986

Hooykaas-Van Slogteren, G.M.S., Hooykaas, P.J.J., Schilperoort, R.A., Nature **311**, 763, 1984

Kao, K.N., Michayluk, M.R., Planta **126**, 105, 1975

Klein, T.M., Gradziel, T., Fromm, M.E., Sanford, J.C., Biotechnology **6**, 559, 1988

Maniatis, T., Fritsch, E.F., Sambrook, J., Molecular cloning, a laboratory manual, Cold Spring Harbor Laboratory, Cold Spring Harbor, 1982

McCormick, S., Niedermeyer, J., Fry, J., Barnason, A., Horsch, R., Fraley, R., Plant Cell Reports **5**, 81, 1986

Murashige, T., Skoog, F., Physiol. Plant. **15**, 473, 1962

Murdock, L.L., Brookhart, G., Dunn, P.E., Foard, D.E., Kelley, S., Kitch, L., Shade, R.E., Shukle, R.H., Wolfson, J.L., Comp. Biochem. Physiol. **87B**, 783, 1987

Nagy, J.I., Maliga, P., Z. Pflanzenphysiol. **78**, 453, 1976

Negrutiu, I., Shillito, R., Potrykus, I., Biasini, G., Sala, F., Plant Mol. Biol. **8**, 363, 1987

Nelson, R.S., McCormick, S.M., Delannay, X., Dubé, P., Layton, J., Anderson, E.J., Kaniewska, M., Proksch, R.K., Horsch, R.B., Rogers, S.G., Fraley, R.T., Beachy, R.N., Biotechnology **6**, 403, 1988

Odell, J.T., Nagy, F., Chua, N.-H., Nature **313**, 810, 1985

Oka, A., Sugisaki, H., Takanami, M., J. Mol. Biol. **147**, 217, 1981

Ooms, G., Hooykaas, P.J.J., Moolenaar, G., Schilperoort, R.A., Gene **14**, 33, 1981

Ooms, G., Hooykaas, P.J.J., van Veen, R.J.M., van Beelen, P., Regensburg-Tuïnk, T.J.G., Schilperoort, R.A., Plasmid **7**, 15, 1982

de la Peña, A., Lörz, H., Schell, J., Nature **325**, 274, 1987

Potrykus, J., Saul., M.W., Petruska, J., Paszkowski, J., Shillito, R.D., Mol. Gen. Gen. Genet. **199**, 183, 1985

Reich, T.J., Iyer, V.N., Haffner, M., Holbrook, L.A., Miki, B.L., Can. J. Bot. **64**, 1259, 1986a

Reich, T.J., Iyer, V.N., Miki, B.L., Biotechnology **4**, 1001, 1986b

Rothstein, S.J., Lahners, K.N., Lotstein, R.J., Carozzi, N.B., Jayne, S.M., Rice, D.A., Gene **53**, 153, 1987

Rosenberg, S., Barr, P.J., Najarian, R.C., Hallewell, R.A., Nature **312**, 77, 1984

Sanger, F., Nicklen, S., Coulson, A.R., Proc. Natl. Acad. Sci. USA **74**, 5463, 1977

Sanger, F., Science **214**, 1205, 1981

Schenk, R.U., Hildebrandt, A.C., Can. J. Bot., **50**, 199, 1972

Schwabe, C., Anastasi, A., Crow, H., McDonald, J.K., Barrett, A.J., Biochem. J. **217**, 813, 1984

Shillito, R.D., Paszkowski, J., Potrykus, I., Plant Cell Reports **2**, 244, 1983

Shillito, R.D., Saul M.W., Paszkowski, J., Müller, M., Potrykus, I., Biotechnology **3**, 1099, 1985

Simon, R., Priefer, U., Pühler, A., in: Pühler, A. (Hrsg.), Molecular Genetics of the Bacteria-Plant Interaction, Springer Verlag, Berlin, 98, 1983a

Simon et al., Biotechnology **1**, 784, 1983b

Southern, E.M., J. Mol. Biol. **98**, 503, 1975

Stockhaus, J., Eckes, P., Blau, A., Schell, J., Willmitzer, L., Nucl. Acids Res. **15**, 3479, 1987

Turk, V., Brzin, J., Longer, M., Ritonja, A., Eropkin, M., Borchart, U., Machleidt, W., Hoppe-Seyler's Z. Physiol. Chem **364**, 1487, 1983

Wolfson, J.L., Murdock, L.L., Entomol. Exp. Appl. **44**, 235, 1987

Wood, W.I., Gitschier, J., Lasky, L.A., Lawn, R.M., Proc. Natl. Acad. Sci. USA **82**, 1585, 1985

Yadav, N.S., Vanderleyden, J., Bennett, D.R. Barnes, W.M., Chilton, M.D., Proc. Natl. Acad. Sci. USA **79**, 6322, 1982

Young, R.A., Davis, R.W., Proc. Natl. Acad. Sci. USA **80**, 1194, 1983

Zoller, M.J., Smith, M., in: Wu, R., Grossmann, L., Moldave, K., Methods in Enzymology **100**, 468, 1983

Hinterlegungen:

(1) *E. coli* HB101/pML147 wurde am 28. Januar 1988 unter der Hinterlegungsnummer DMS 4380 bei der Deutschen Sammlung von Mikroorganismen und Zellkulturen in Braunschweig, Bundesrepublik Deutschland hinterlegt. Die folgenden Mikroorganismen wurden bei der American Type Culture Collection (ATCC) in Rockville, Maryland, USA hinterlegt:

(2) Plasmid pLW111 (ATCC 40235) am 14. Mai 1986.

(3) *Zea mays* Zellkultur (ATCC 40326) am 20. Mai 1987.

(4) Plasmid p368 (ATCC 67700) am 19. Mai 1988.

**Patentansprüche**

1. Ein Verfahren zur Bekämpfung eines Pflanzenschädlings, der eine bestimmte Zielpflanze angreift, welche entweder eine Monokotyledone oder eine Dikotyledone ist, basierend auf dem Prinzip, den Schädling in oder auf einer Pflanze einer pestizid wirksamen Menge eines nicht-pflanzlichen Proteinase-Inhibitors auszusetzen, welcher kein Eglin B oder C darstellt und in der Pflanze biologisch als Ergebnis der Expression eines fremden Gens, welches den Proteinase-Inhibitor kodiert, oder als Ergebnis der Expression eines oder mehrerer fremder Gene, die einen oder mehrere Vorläufer des Proteinase-Inhibitors kodieren, synthetisiert wird.

2. Ein Verfahren gemäss Anspruch 1, dadurch gekennzeichnet, dass der Proteinase-Inhibitor von einem Tier, einem Bakterium oder einem Pilz abstammt, oder dass das Inhibitorgen wesentliche Sequenz-Homologie mit einem Proteinase-Inhibitorgen hat, das aus einem der vorgenannten Organismen abstammt.

3. Ein Verfahren gemäss Anspruch 1, dadurch gekennzeichnet, dass der Schädling ein Insekt, eine Milbe, ein Pilz oder ein Bakterium ist.

4. Ein Verfahren gemäss Anspruch 1, dadurch gekennzeichnet, dass die Monokotyledone typmässig ausgewählt ist aus der Gruppe bestehend aus Getreide, Gemüse- und Knollen-liefernden Pflanzen sowie Futter- und Rasengräsern.

5. Ein Verfahren gemäss Anspruch 4, dadurch gekennzeichnet, dass die Monokotyledone ausgewählt ist aus der Gruppe bestehend aus *Dactylis* (Knäuelgras) und *Zea mays* (Mais).

**6.** Ein Verfahren gemäss Anspruch 1, dadurch gekennzeichnet, dass die Dikotyledone typmässig ausgewählt ist aus der Gruppe bestehend aus Obst-liefernden Pflanzen, Gemüse- und Knollen-liefernden Pflanzen, Oelpflanzen, Drogen-Enthaltenden Pflanzen sowie Fasern-und Holzliefernden Pflanzen.

**7.** Ein Verfahren gemäss Anspruch 6, dadurch gekennzeichnet, dass die Dikotyledone ausgewählt ist aus der Gruppe von Gattungen bestehend aus *Lycopersicon* (Tomaten), *Solanum* (Kartoffeln), *Nicotiana* (Tabak) und *Gossypium* (Baumwolle).

**8.** Ein Verfahren gemäss Anspruch 1, dadurch gekennzeichnet, dass der Proteinase-Inhibitor ein Inhibitor einer Serin-Proteinase ist

**9.** Ein Verfahren gemäss Anspruch 8, dadurch gekennzeichnet, dass der Inhibitor ausgewählt ist aus der Gruppe bestehend aus $\alpha_1$-Antitrypsin, Aprotinin, und Hühner-Trypsin-Inhibitor.

**10.** Ein Verfahren gemäss Anspruch 1, dadurch gekennzeichnet, dass der Inhibitor ein Inhibitor einer Thiolproteinase ist.

**11.** Ein Verfahren gemäss Anspruch 10, dadurch gekennzeichnet, dass der Inhibitor ausgewählt ist aus der Gruppe bestehend aus Cystatin, Antipain, Leupeptin, E64 und dessen Abkömmlingen.

**12.** Ein Verfahren gemäss Anspruch 10, dadurch gekennzeichnet, dass der Inhibitor Antipain oder Leupeptin ist.

**13.** Ein Verfahren gemäss Anspruch 10, dadurch gekennzeichnet, dass der Inhibitor Hühnereiweiss-Cystatin ist

**14.** Ein Verfahren gemäss Anspruch 1, dadurch gekennzeichnet, dass der Inhibitor ein Inhibitor einer Metallproteinase ist.

**15.** Ein Verfahren gemäss Anspruch 1, dadurch gekennzeichnet, dass der Inhibitor ein Inhibitor einer sauren Proteinase ist.

**16.** Ein Verfahren gemäss Anspruch 15, dadurch gekennzeichnet, dass der Inhibitor Pepstatin ist sowie ein Inhibitor, der eine wesentliche strukturelle oder funktionelle Ähnlichkeit mit Pepstatin hat.

**17.** Ein Verfahren gemäss Anspruch 1, welches dadurch gekennzeichnet ist, dass

(a) es sich bei der Pflanze um eine Monokotyledone handelt und
(b) besagter Proteinase-Inhibitor ausgewählt ist aus einer Gruppe bestehend aus Inhibitoren der Serin-Proteinasen, Thiolproteinasen, Metallproteinasen und sauren Proteinasen.

**18.** Ein Verfahren gemäss Anspruch 17, dadurch gekennzeichnet, dass der besagte Proteinase-Inhibitor ein Inhibitor einer Serin-Proteinase ist

**19.** Ein Verfahren gemäss Anspruch 18, dadurch gekennzeichnet, dass der Inhibitor $\alpha_1$-Antitrypsin ist.

**20.** Ein Verfahren gemäss Anspruch 17, dadurch gekennzeichnet, dass besagter Proteinase-Inhibitor ein Inhibitor einer Thiolproteinase ist.

**21.** Ein Verfahren gemäss Anspruch 20, dadurch gekennzeichnet, dass der Inhibitor ausgewählt ist aus der Gruppe bestehend aus Cystatin, Antipain, Leupeptin, E64 und dessen Abkömmlingen.

**22.** Ein Verfahren gemäss Anspruch 21, dadurch gekennzeichnet, dass der Inhibitor Antipain oder Leupeptin ist.

**23.** Ein Verfahren gemäss Anspruch 21, dadurch gekennzeichnet, dass der Inhibitor Hühnereiweiss-Cystatin ist

**24.** Ein Verfahren gemäss Anspruch 17, dadurch gekennzeichnet, dass die Monokotyledone Mais ist.

**25.** Ein Verfahren gemäss Anspruch 17, dadurch gekennzeichnet, dass der Schädling ein Vertreter der Ordnungen *Coleoptera* oder *Lepidoptera* ist.

**26.** Ein Verfahren gemäss Anspruch 25, dadurch gekennzeichnet, dass der Schädling *Diabrotica* ist.

**27.** Ein Verfahren gemäss Anspruch 17, dadurch gekennzeichnet, dass der Proteinase-Inhibitor in den Wurzeln, Stengeln, Blättern, Samen oder Pollen der Pflanze exprimiert wird.

**28.** Ein Verfahren gemäss Anspruch 1, welches dadurch gekennzeichnet ist, dass

(a) es sich bei der Pflanze um eine Dikotyledone handelt und
(b) besagter Proteinase-Inhibitor ausgewählt ist aus einer Gruppe bestehend aus Inhibitoren der Serin-Proteinasen, Thiolproteinasen, Metallproteinasen und sauren Proteinasen.

**29.** Ein Verfahren gemäss Anspruch 28, dadurch gekennzeichnet, dass der besagte Proteinase-Inhibitor ein Inhibitor einer Serin-Proteinase ist

**30.** Ein Verfahren gemäss Anspruch 29, dadurch gekennzeichnet, dass der Inhibitor $\alpha_1$-Antitrypsin ist.

**31.** Ein Verfahren gemäss Anspruch 28, dadurch gekennzeichnet, dass besagter Proteinase-Inhibitor ein Inhibitor einer Thiolproteinase ist.

**32.** Ein Verfahren gemäss Anspruch 31, dadurch gekennzeichnet, dass der Inhibitor ausgewählt ist aus der Gruppe bestehend aus Cystatin, Antipain, Leupeptin, E64 und dessen Abkömmlingen.

**33.** Ein Verfahren gemäss Anspruch 32, dadurch gekennzeichnet, dass der Inhibitor Antipain oder Leupeptin ist.

**34.** Ein Verfahren gemäss Anspruch 32, dadurch gekennzeichnet, dass der Inhibitor Hühnereiweiss-Cystatin ist

**35.** Ein Verfahren gemäss Anspruch 28, dadurch gekennzeichnet, dass die Dikotyledone Kartoffel, Tomate, Tabak oder Baumwolle ist.

**36.** Ein Verfahren gemäss Anspruch 28, dadurch gekennzeichnet, dass der Schädling ein Vertreter der Ordnungen *Coleoptera* oder *Lepidoptera* ist.

**37.** Ein Verfahren gemäss Anspruch 36, dadurch gekennzeichnet, dass der Schädling aus der Gruppe von Gattungen ausgewählt ist bestehend aus *Diabrotica*, *Leptinotarsa* und *Anthonomus*.

**38.** Ein Verfahren gemäss Anspruch 37, dadurch gekennzeichnet, dass der Schädling ein Kartoffelkäfer ist.

**39.** Ein Verfahren gemäss Anspruch 28, dadurch gekennzeichnet, dass der Proteinase-Inhibitor in den Wurzeln, Stengeln, Blättern, Samen oder Pollen der Pflanze exprimiert wird.

**40.** Ein Verfahren gemäss Anspruch 1 zur Bekämpfung eines Pflanzenschädlings, der eine bestimmte Zielpflanze angreift, basierend auf dem Prinzip, den Schädling in oder auf der Pflanze einer pestizid wirksamen Menge eines nicht pflanzlichen Proteinase-Inhibitors auszusetzen, welcher kein Eglin B oder C darstellt und welcher in der Pflanze biologisch als Ergebnis der Expression eines fremden Gens, welches den Proteinase-Inhibitor kodiert, oder als Ergebnis der Expression eines oder mehrerer Gene, die einen oder mehrere Vorläufer des Proteinase-Inhibitors kodieren, synthetisiert wird, wobei das Verfahren dadurch gekennzeichnet ist, dass das Polypeptid ausgewählt ist aus der Gruppe von Proteinase-Inhibitoren der Thiolproteinasen, Metallproteinasen, sauren Proteinasen und Serin-Proteinasen.

**41.** Ein Verfahren gemäss Anspruch 40, dadurch gekennzeichnet, dass die Pflanze eine Dikotyledone ist.

**42.** Ein Verfahren gemäss Anspruch 41, dadurch gekennzeichnet, dass die Dikotyledone aus der Gruppe von Gattungen ausgewählt ist, bestehend aus *Lycopersicon* (Tomaten), *Solanum* (Kartoffeln), *Gossypium* (Baumwolle) und *Nicotiana* (Tabak).

**43.** Ein Verfahren gemäss Anspruch 40, dadurch gekennzeichnet, dass der Proteinase-Inhibitor ein Serin-Proteinase-Inhibitor ist.

**44.** Eine transgene Pflanze, welche entweder eine Monokotyledone oder eine Dikotyledone ist, dadurch gekennzeichnet, dass sie ein fremdes Gen oder eine fremde Gengruppe enthält, welche wenigstens einen kodierenden Abschnitt umfassen, der einen nicht-pflanzlichen Proteinase-Inhibitor oder einen oder mehrere Vorläufer eines Proteinase-Inhibitors expremiert, wobei der Proteinase-Inhibitor kein Eglin B oder C darstellt und aus der Gruppe bestehend aus Inhibitoren der Serin-Proteinasen, Thiolproteinasen, Metallproteinasen und sauren Proteinasen ausgewählt ist

**45.** Eine Pflanze gemäss Anspruch 44, dadurch gekennzeichnet, dass die Monokotyledone typmässig ausgewählt ist aus der Gruppe bestehend aus Getreide, Gemüse- und Knollen-liefernden Pflanzen sowie Futter- und Rasengräsern.

**46.** Eine Pflanze gemäss Anspruch 45, dadurch gekennzeichnet, dass die Monokotyle ausgewählt ist aus der Gruppe bestehend aus *Zea mays* (Mais) und *Dactylis* (Knäuelgras).

**47.** Eine Pflanze gemäss Anspruch 46, dadurch gekennzeichnet, dass die Dikotyledone typmässig ausgewählt ist aus der Gruppe bestehend aus Obst-liefernden Pflanzen, Gemüse- und Knollen-liefernden Pflanzen, Oelpflanzen, Drogen-Enthaltenden Pflanzen sowie Fasern- und Holzliefernden Pflanzen.

**48.** Eine Pflanze gemäss Anspruch 47, dadurch gekennzeichnet, dass die Dikotyle ausgewählt ist aus der Gruppe von Gattungen bestehend aus *Lycopersicon* (Tomaten), *Solanum* (Kartoffeln), *Nicotiana* (Tabak) und *Gossypium* (Baumwolle).

**49.** Eine Pflanze gemäss Anspruch 44, dadurch gekennzeichnet, dass der Inhibitor von einem Tier, einem Bakterium oder einem Pilz abstammt, oder dass das Inhibitorgen wesentliche Sequenz-Homologie mit einem Proteinase-Inhibitorgen hat, das aus einem der vorgenannten Organismen abstammt.

**50.** Eine Pflanze gemäss Anspruch 44, dadurch gekennzeichnet, dass der Proteinase-Inhibitor ein Inhibitor einer Serin-Proteinase ist

**51.** Eine Pflanze gemäss Anspruch 50, dadurch gekennzeichnet, dass der Inhibitor $\alpha_1$-Antitrypsin ist.

**52.** Eine Pflanze gemäss Anspruch 44, dadurch gekennzeichnet, dass der Inhibitor ein Inhibitor einer Thiolproteinase ist

**53.** Eine Pflanze gemäss Anspruch 52, dadurch gekennzeichnet, dass der Inhibitor ausgewählt ist aus der Gruppe bestehend aus Cystatin, Antipain, Leupeptin, E64 und dessen Abkömmlingen sowie Inhibitoren, die eine wesentliche strukturelle oder funktionelle Aehnlichkeit mit den genannten haben.

**54.** Eine Pflanze gemäss Anspruch 53, dadurch gekennzeichnet, dass der Inhibitor Antipain oder Leupeptin ist.

**55.** Eine Pflanze gemäss Anspruch 53, dadurch gekennzeichnet, dass der Inhibitor Hühnereiweiss-Cystatin ist

**56.** Eine Pflanze gemäss Anspruch 44, dadurch gekennzeichnet, dass der Inhibitor ein Inhibitor einer Metallproteinase ist.

**57.** Eine Pflanze gemäss Anspruch 44, dadurch gekennzeichnet, dass der Inhibitor ein Inhibitor einer sauren Proteinase ist

**58.** Eine Pflanze gemäss Anspruch 57, dadurch gekennzeichnet, dass der Inhibitor Pepstatin ist.

**59.** Eine transgene Pflanze gemäss Anspruch 44, dadurch gekennzeichnet, dass sie eine Monokotyledone ist.

**60.** Eine Pflanze gemäss Anspruch 59, dadurch gekennzeichnet, dass die Monokotyledone Mais ist

**61.** Eine Pflanze gemäss Anspruch 59, dadurch gekennzeichnet, dass sie Schädlinge der Gattungen *Diabrotica* bekämpft.

**62.** Eine Pflanze gemäss Anspruch 59, dadurch gekennzeichnet, dass der Proteinase-Inhibitor in den Wurzeln, Stengeln, Blättern, Samen oder Pollen der Pflanze exprimiert wird.

**63.** Eine Pflanze gemäss Anspruch 62, dadurch gekennzeichnet, dass der Proteinase-Inhibitor in den Wurzeln der Pflanze exprimiert wird.

**64.** Eine transgene Pflanze gemäss Anspruch 44, dadurch gekennzeichnet, dass sie eine Dikotyledone ist

**65.** Eine Pflanze gemäss Anspruch 64, dadurch gekennzeichnet, dass die Dikotyledone Tomate, Kartoffel, Tabak oder Baumwolle ist.

**66.** Eine Pflanze gemäss Anspruch 64, dadurch gekennzeichnet, dass sie Kartoffelkäfer bekämpft.

**67.** Eine Pflanze gemäss Anspruch 64, dadurch gekennzeichnet, dass der Proteinase-Inhibitor in den Wurzeln, Stengeln, Blättern, Samen oder Pollen der Pflanze exprimiert wird.

**68.** Ein Vektor, der eine DNA-Sequenz umfasst, die einen nicht pflanzlichen Proteinase-Inhibitor kodiert, welcher kein Eglin B oder C darstellt, dadurch gekennzeichnet, dass der Vektor zur Transformation von Pflanzenzellen oder von *Agrobacterium* und zur Expression des Proteinase-Inhibitors in Pflanzen verwendet werden kann

**69.** Ein Vektor gemäss Anspruch 68, dadurch gekennzeichnet, dass er ein vom Ti-Plasmid abgeleiteter Vektor ist.

**70.** Ein Vektor gemäss Anspruch 68, dadurch gekennzeichnet, dass er von den Plasmiden pCIB10 oder pCIB715 abgeleitet ist.

**Claims**

**1.** A method of controlling a plant pest which attacks a specific target plant, which is either a monocot or a dicot, based on the principle of exposing the pest, in or on a plant, to a pesticidally active amount of a non-plant proteinase inhibitor, which is other than Eglin B or C and which is synthesized in the plant biologically as the result of expressing a foreign gene which encodes the proteinase inhibitor, or as the result of expressing one or more foreign genes which encode one or more precursors of the proteinase inhibitor.

**2.** A method according to claim 1, wherein the proteinase inhibitor is derived from an animal, a bacterium or a fungus, or where the sequence of the inhibitor gene is essentially homologous to the sequence of a proteinase inhibitor gene derived from one of the abovementioned organisms.

**3.** A method according to claim 1, wherein the pest is an insect, a mite, a fungus or a bacterium.

**4.** A method according to claim 1, wherein the type of the monocot is selected from the group consisting of cereals, vegetable plants, tuber plants, fodder grasses and turf grasses.

**5.** A method according to claim 4, wherein the monocot is selected from the group consisting of Dactylis (orchard grass) and Zea mays (maize).

**6.** A method according to claim 1, wherein the type of the dicot is selected from the group consisting of fruiting plants, vegetable plants, tuber plants, oil plants, medicinal plants, fibre plants and timber plants.

**7.** A method according to claim 6, wherein the dicot is selected from the group of genera consisting of Lycopersicon (tomatoes), Solanum (potatoes), Nicotiana (tobacco) and Gossypium (cotton).

**8.** A method according to claim 1, wherein the proteinase inhibitor is a serine proteinase inhibitor.

**9.** A method according to claim 8, wherein the inhibitor is selected from the group consisting of $a_1$-antitrypsin, aprotinin, and chicken trypsin inhibitor.

**10.** A method according to claim 1, wherein the inhibitor is a thiol proteinase inhibitor.

**11.** A method according to claim 10, wherein the inhibitor is selected from the group consisting of cystatin, antipain, leupeptin, E64 and its derivatives.

**12.** A method according to claim 10, wherein an inhibitor is antipain or leupeptin.

**13.** A method according to claim 10, wherein the inhibitor is chicken albumen cystatin.

**14.** A method according to claim 1, wherein the inhibitor is a metal proteinase inhibitor.

**15.** A method according to claim 1, wherein the inhibitor is an inhibitor of an acid proteinase.

**16.** A method according to claim 15, wherein the inhibitor is pepstatin and an inhibitor which is essentially structurally or functionally similar to pepstatin.

**17.** A method according to claim 1, wherein

(a) the plant is a monocot and
(b) said proteinase inhibitor is selected from a group consisting of serine proteinase inhibitors, thiol proteinase inhibitors, metal proteinase inhibitors and inhibitors of acid proteinases.

**18.** A method according to claim 17, wherein said proteinase inhibitor is a serine proteinase inhibitor.

**19.** A method according to claim 18, wherein the inhibitor is $a_1$-antitrypsin.

**20.** A method according to claim 17, wherein said proteinase inhibitor is a thiol proteinase inhibitor.

**21.** A method according to claim 20, wherein the inhibitor is selected from the group consisting of cystatin, antipain, leupeptin, E64 and its derivatives.

**22.** A method according to claim 21, wherein the inhibitor is antipain or leupeptin.

**23.** A method according to claim 21, wherein the inhibitor is chicken albumen cystatin.

**24.** A method according to claim 17, wherein the monocot is maize.

**25.** A method according to claim 17, wherein the pest is a representative of the orders Coleoptera or Lepidoptera.

**26.** A method according to claim 25, wherein the pest is Diabrotica.

**27.** A method according to claim 17, wherein the proteinase inhibitor is expressed in the roots, the stalks, the leaves, the seeds or the pollen of the plant.

**28.** A method according to claim 1, wherein

(a) the plant is a dicot and
(b) said proteinase inhibitor is selected from a group consisting of serine proteinase inhibitors, thiol proteinase inhibitors, metal proteinase inhibitors and inhibitors of acid proteinases.

**29.** A method according to claim 28, wherein said proteinase inhibitor is a serine proteinase inhibitor.

**30.** A method according to claim 29, wherein the inhibitor is $a_1$-antitrypsin.

**31.** A method according to claim 28, wherein said proteinase inhibitor is a thiol proteinase inhibitor.

**32.** A method according to claim 31, wherein the inhibitor is selected from the group consisting of cystatin, antipain, leupeptin, E64 and its derivatives.

**33.** A method according to claim 32, wherein the inhibitor is antipain or leupeptin.

**34.** A method according to claim 32, wherein the inhibitor is chicken albumen cystatin.

**35.** A method according to claim 28, wherein the dicot is potato, tomato, tobacco or cotton.

**36.** A method according to claim 28, wherein the pest is a representative of the orders Coleoptera or Lepidoptera.

**37.** A method according to claim 36, wherein the pest is selected from the group of genera consisting of Diabrotica, Leptinotarsa and Anthonomus.

**38.** A method according to claim 37, wherein the pest is a Colorado beetle.

**39.** A method according to claim 28, wherein the proteinase inhibitor is expressed in the roots, the stalks, the leaves, the seeds or the pollen of the plant.

**40.** A method according to claim 1 for controlling a plant pest which attacks a specific target plant, based on the principle of exposing the pest, in or on a plant, to a pesticidally active amount of a non-plant proteinase inhibitor, which is other than eglin B or C and which is synthesized in the plant biologically as the result of expressing a foreign gene which encodes the proteinase inhibitor, or as the result of expressing one or more genes which encode one or more precursors of the proteinase inhibitor, in which the polypeptide is selected from the group of proteinase inhibitors of the thiol proteinases, metal proteinases, acid proteinases and serine proteinases.

**41.** A method according to claim 40, wherein the plant is a dicot.

**42.** A method according to claim 41, wherein the dicot is selected from the group of genera consisting of Lycopersicon (tomatoes), Solanum (potatoes), Gossypium (cotton) and Nicotiana (tobacco).

**43.** A method according to claim 40, wherein the proteinase inhibitor is a serine proteinase inhibitor.

**44.** A transgenic plant which is either a monocot or a dicot, which plant comprises a foreign gene or a group of foreign genes, which encompass at least one coding region expressing a non-plant proteinase inhibitor or one or more precursors of a proteinase inhibitor, where the proteinase inhibitor is other than eglin B or C and is selected from the group consisting of serine proteinase inhibitors, thiol proteinase inhibitors, metal proteinase inhibitors and inhibitors of acid proteinases.

**45.** A plant according to claim 44, wherein the type of monocot is selected from the group consisting of cereals, vegetable plants, tuber plants, fodder grasses and turf grasses.

**46.** A plant according to claim 45, wherein the monocot is selected from the group consisting of Zea mays (maize) and Dactylis (orchard grass).

**47.** A plant according to claim 46, wherein the type of the dicot is selected from the group consisting of fruiting plants, vegetable plants, tuber plants, oil plants, medicinal plants, fibre plants and timber plants.

**48.** A plant according to claim 47, wherein the dicot is selected from the group of genera consisting of Lycopersicon (tomatoes), Solanum (potatoes), Nicotiana (tobacco) and Gossypium (cotton).

**49.** A plant according to claim 44, wherein the inhibitor is derived from an animal, a bacterium or a fungus, or where the sequence of the inhibitor gene is essentially homologous to the sequence of a proteinase inhibitor gene derived from one of the abovementioned organisms.

**50.** A plant according to claim 44, wherein the proteinase inhibitor is a serine proteinase inhibitor.

**51.** A plant according to claim 50, wherein the inhibitor is $a_1$-antitrypsin.

**52.** A plant according to claim 44, wherein the inhibitor is a thiol proteinase inhibitor.

**53.** A plant according to claim 52, wherein the inhibitor is selected from the group consisting of cystatin, antipain, leupeptin, E64 and its derivatives and inhibitors which are essentially structurally or functionally similar to those mentioned.

**54.** A plant according to claim 53, wherein the inhibitor is antipain or leupeptin.

**55.** A plant according to claim 53, wherein the inhibitor is chicken albumen cystatin.

**56.** A plant according to claim 44, wherein the inhibitor is a metal proteinase inhibitor.

**57.** A plant according to claim 44, wherein the inhibitor is an inhibitor of an acid proteinase.

**58.** A plant according to claim 57, wherein the inhibitor is pepstatin.

**59.** A transgenic plant according to claim 44, which is a monocot.

**60.** A plant according to claim 59, which monocot is maize.

**61.** A plant according to claim 59, which controls pests of the genus Diabrotica.

**62.** A plant according to claim 59, wherein the proteinase inhibitor is expressed in the roots, the stalks, the leaves, the seeds or the pollen of the plant.

**63.** A plant according to claim 62, wherein the proteinase inhibitor is expressed in the roots of the plant.

**64.** A transgenic plant according to claim 44, which is a dicot.

**65.** A plant according to claim 64, which dicot is tomato, potato, tobacco or cotton.

**66.** A plant according to claim 64, which controls Colorado beetles.

**67.** A plant according to claim 64, wherein the proteinase inhibitor is expressed in the roots, the stalks, the leaves, the seeds or the pollen of the plant.

**68.** A vector encompassing a DNA sequence which encodes a non-plant proteinase inhibitor which is other than eglin B or C, which vector can be used for the transformation of plant cells or of Agrobacterium and for expressing the proteinase inhibitor in plants.

**69.** A vector according to claim 68, which is derived from the Ti-plasmid.

**70.** A vector according to claim 68, which is derived from plasmid pCIB10 or pCIB715.

**Revendications**

**1.** Procédé de lutte contre un parasite des plantes, qui attaque une plante cible déterminée, laquelle est une monocotylédone ou une dicotylédone, sur la base du principe consistant à exposer le parasite dans ou sur une plante à une quantité à action pesticide d'un inhibiteur de la protéinase non végétal, qui ne représente pas d'égline B ou C, et qui est synthétisé dans la plante de façon biologique comme résultat de l'expression d'un gène étranger qui code pour l'inhibiteur de protéinase, ou comme résultat de l'expression d'un ou plusieurs gènes étrangers qui codent pour un ou plusieurs précurseurs de l'inhibiteur de la protéinase.

**2.** Procédé selon la revendication 1, caractérisé en ce que l'inhibiteur de la protéinase provient d'un animal, d'une bactérie ou d'un champignon, ou en ce que le gène de l'inhibiteur présente une homologie de séquence essentielle avec un gène d'inhibiteur de la protéinase qui provient de l'un des organismes mentionné ci-dessus.

**3.** Procédé selon la revendication 1, caractérisé en ce que le parasite est un insecte, un acarien, un champignon ou une bactérie.

**4.** Procédé selon la revendication 1, caractérisé en ce que la monocotylédone est choisie selon son type dans le groupe constitué par les céréales, les plantes donneuses de légumes et de tubercules ainsi que les légumineuses fourragères et herbeuses.

5. Procédé selon la revendication 4, caractérisé en ce que la monocotylédone est choisie dans le groupe constitué par *Dactylis* (patte de lièvre) et *Zea mays* (maïs).

6. Procédé selon la revendication 1, caractérisé en ce que la dicotylédone est choisie selon son type dans le groupe constitué par les plantes donneuses de fruits, les plantes donneuses de légumes et de tubercules, les plantes oléagineuses, les plantes contenant des drogues ainsi que les plantes donneuses de fibres et ligneuses.

7. Procédé selon la revendication 6, caractérisé en ce que la dicotylédone est choisie dans le groupe des genres constitué par *Lycopersicon* (tomates), *Solanum* (pommes de terre), *Nicotiana* (tabac) et *Gossypium* (coton).

8. Procédé selon la revendication 1, caractérisé en ce que l'inhibiteur de la protéinase est un inhibiteur d'une protéinase de sérine.

9. Procédé selon la revendication 8, caractérisé en ce que l'inhibiteur est choisi dans le groupe constitué par l'$\alpha_1$-antitrypsine, l'aprotinine, et l'inhibiteur de la trypsine d'oeuf.

10. Procédé selon la revendication 1, caractérisé en ce que l'inhibiteur est un inhibiteur d'une thiol protéinase.

11. Procédé selon la revendication 10, caractérisé en ce que l'inhibiteur est choisi dans le groupe constitué par la cystatine, l'antipaïne, la leupeptine, l'E64 et ses dérivés.

12. Procédé selon la revendication 10, caractérisé en ce que l'inhibiteur est l'antipaïne ou la leupeptine.

13. Procédé selon la revendication 10, caractérisé en ce que l'inhibiteur est la cystatine d'albumine d'oeuf.

14. Procédé selon la revendication 1, caractérisé en ce que l'inhibiteur est un inhibiteur d'une protéinase métallique.

15. Procédé selon la revendication 1, caractérisé en ce que l'inhibiteur est un inhibiteur d'une protéinase acide.

16. Procédé selon la revendication 15, caractérisé en ce que l'inhibiteur est la pepstatine ainsi qu'un inhibiteur qui présente une analogie structurelle ou fonctionnelle essentielle avec la pepstatine.

17. Procédé selon la revendication 1, qui se caractérise en ce que

      (a) il s'agit pour la plante d'une monocotylédone et
      (b) l'inhibiteur de la protéinase en question est choisi dans un groupe constitué par les inhibiteurs des protéinases sériques, des thiol protéinases, des protéinases métalliques et des protéinases acides.

18. Procédé selon la revendication 17, caractérisé en ce que l'inhibiteur de la protéinase en question est un inhibiteur d'une protéinase de sérine.

19. Procédé selon la revendication 18, caractérisé en ce que l'inhibiteur est l'$\alpha_1$-antitrypsine.

20. Procédé selon la revendication 17, caractérisé en ce que l'inhibiteur de la protéinase en question est un inhibiteur d'une thiol protéinase.

21. Procédé selon la revendication 20, caractérisé en ce que l'inhibiteur est choisi dans le groupe constitué par la cystatine, l'antipaïne, la leupeptine, l'E64 et ses dérivés.

22. Procédé selon la revendication 21, caractérisé en ce que l'inhibiteur est l'antipaïne ou la leupeptine.

23. Procédé selon la revendication 21, caractérisé en ce que l'inhibiteur est la cystatine d'albumine d'oeuf.

24. Procédé selon la revendication 17, caractérisé en ce que la monocotylédone est le maïs.

25. Procédé selon la revendication 17, caractérisé en ce que le parasite est un représentant des ordres des coléoptères ou des lépidoptères.

**26.** Procédé selon la revendication 25, caractérisé en ce que le parasite est *Diabrotica*.

**27.** Procédé selon la revendication 17, caractérisé en ce que l'inhibiteur de la protéinase est exprimé dans les racines, les tiges, les feuilles, les graines ou le pollen de la plante.

**28.** Procédé selon la revendication 1, caractérisé en ce que

(a) il s'agit, pour la plante, d'une dicotylédone et
(b) l'inhibiteur de la protéinase en question est choisi dans le groupe constitué par les inhibiteurs des protéinases sériques, des thiol protéinases, des protéinases métalliques et des protéinases acides.

**29.** Procédé selon la revendication 28, caractérisé en ce que l'inhibiteur de la protéinase en question est un inhibiteur d'une protéinase de sérine.

**30.** Procédé selon la revendication 29, caractérisé en ce que l'inhibiteur est l'$\alpha_1$-antitrypsine.

**31.** Procédé selon la revendication 28, caractérisé en ce que l'inhibiteur de la protéinase en question est un inhibiteur d'une thiol protéinase.

**32.** Procédé selon la revendication 31, caractérisé en ce que l'inhibiteur est choisi dans le groupe constitué par la cystatine, l'antipaïne, la leupeptine, l'E64 et ses dérives.

**33.** Procédé selon la revendication 32, caractérisé en ce que l'inhibiteur est l'antipaïne ou la leupeptine.

**34.** Procédé selon la revendication 32, caractérisé en ce que l'inhibiteur est la cystatine d'albumine d'oeuf.

**35.** Procédé selon la revendication 28, caractérisé en ce que la dicotylédone est la pomme de terre, la tomate, le tabac ou le coton.

**36.** Procédé selon la revendication 28, caractérisé en ce que le parasite est un représentant des ordres des coléoptères ou des lépidoptères.

**37.** Procédé selon la revendication 36, caractérisé en ce que le parasite est choisi dans le groupe des genres constitué par *Diabrotica*, *Leptinotarsa* et *Anthonomus*.

**38.** Procédé selon la revendication 37, caractérisé en ce que le parasite est un doryphore.

**39.** Procédé selon la revendication 28, caractérisé en ce que l'inhibiteur de protéinase est exprimé dans les racines, les tiges, les feuilles, les graines ou le pollen de la plante.

**40.** Procédé selon la revendication 1 de lutte contre un parasite des plantes, qui attaque une plante cible déterminée, sur la base du principe consistant à exposer le parasite dans ou sur la plante à une quantité à action pesticide d'un inhibiteur de la protéinase non végétal, qui ne représente pas une égline B ou C, et qui est synthétisé dans la plante biologiquement comme résultat de l'expression d'un gène étranger qui code pour l'inhibiteur de la protéinase, ou comme résultat de l'expression d'un ou plusieurs gènes qui code pour un ou plusieurs précurseurs de l'inhibiteur de la protéinase, le procédé se caractérisant en ce que le polypeptide est choisi dans le groupe des inhibiteurs de la protéinase des thiol protéinases, des protéinases métalliques, des protéinases acides et des protéinases de sérine.

**41.** Procédé selon la revendication 40, caractérisé en ce que la plante est une dicotylédone.

**42.** Procédé selon la revendication 41, caractérisé en ce que la dicotylédone est choisie dans le groupe des genres constitué par *Lycopersicon* (tomates), *Solanum* (pommes de terre), *Gossypium* (coton) et *Nicotiana* (tabac).

**43.** Procédé selon la revendication 40, caractérisé en ce que l'inhibiteur de la protéinase est un inhibiteur d'une protéinase de sérine.

**EP 0 348 348 B1**

44. Plante transgénique qui est une monocotylédone ou une dicotylédone, caractérisée en ce qu'elle contient un gêne étranger ou un groupe de gènes étrangers comprenant au moins une section codante qui exprime un inhibiteur de la protéinase non végétal ou un ou plusieurs précurseurs d'un inhibiteur de la protéinase, l'inhibiteur de la protéinase ne représentant pas l'égline B ou C et étant choisi dans le groupe constitué par les inhibiteurs des protéinases de sérine, des thiol protéinases, des protéinases métalliques et des protéinases acides.

45. Plante selon la revendication 44, caractérisée en ce que la monocotylédone est choisie selon son type dans le groupe constitué par les céréales, les plantes donnant des légumes et des tubercules, ainsi que les légumineuses fourragères et herbeuses.

46. Plante selon la revendication 45, caractérisée en ce que la monocotylédone est choisie dans le groupe constitué par *Zea mays* (maïs) et *Dactylis* (patte de lièvre).

47. Plante selon la revendication 46, caractérisée en ce que la dicotylédone est choisie selon son type dans le groupe constitué par les plantes donneuses de fruits, les plantes donneuses de légumes et de tubercules, les plantes oléagineuses, les plantes contenant des drogues ainsi que les plantes donneuses de fibres et ligneuses.

48. Plante selon la revendication 47, caractérisée en ce que la dicotylédone est choisie dans le groupe des genres constitué par *Lycopersicon* (tomates), *Solanum* (pommes de terre), *Gossypium* (coton) et *Nicotiana* (tabac).

49. Plante selon la revendication 44, caractérisée en ce que l'inhibiteur dérive d'un animal, d'une bactérie ou d'un champignon, ou en ce que le gène inhibiteur présente une homologie de séquence essentielle avec un gène inhibiteur de la protéinase qui provient de l'un des organismes mentionnés plus haut.

50. Plante selon la revendication 44, caractérisée en ce que l'inhibiteur de la protéinase est un inhibiteur d'une protéinase de sérine.

51. Plante selon la revendication 50, caractérisée en ce que l'inhibiteur est l'$\alpha_1$-antitrypsine.

52. Plante selon la revendication 44, caractérisée en ce que l'inhibiteur est un inhibiteur d'une thiol protéinase.

53. Plante selon la revendication 52, caractérisée en ce que l'inhibiteur est choisi dans le groupe constitué par la cystatine, l'antipaïne, la leupeptine, l'E64 et ses dérivés, ainsi que les inhibiteurs qui ont une analogie structurelle ou fonctionnelle essentielle avec les inhibiteurs mentionnés.

54. Plante selon la revendication 53, caractérisée en ce que l'inhibiteur est l'antipaïne ou la leupeptine.

55. Plante selon la revendication 53, caractérisée en ce que l'inhibiteur est la cystatine d'albumine d'oeuf.

56. Plante selon la revendication 44, caractérisée en ce que l'inhibiteur est un inhibiteur d'une protéinase métallique.

57. Plante selon la revendication 44, caractérisée en ce que l'inhibiteur est un inhibiteur d'une protéinase acide.

58. Plante selon la revendication 57, caractérisée en ce que l'inhibiteur est la pepstatine.

59. Plante transgénique selon la revendication 44, caractérisée en ce qu'elle est une monocotylédone.

60. Plante selon la revendication 59, caractérisée en ce que la monocotylédone est le maïs.

61. Plante selon la revendication 59, caractérisée en ce qu'elle lutte contre les parasites des genres *Diabrotica*.

62. Plante selon la revendication 59, caractérisée en ce que l'inhibiteur de la protéinase est exprimé dans les racines, les tiges, les feuilles, les graines ou le pollen de la plante.

63. Plante selon la revendication 62, caractérisée en ce que l'inhibiteur de la protéinase est exprimé dans les racines de la plante.

64. Plante transgénique selon la revendication 44, caractérisée en ce qu'elle est une dicotylédone.

70

**65.** Plante selon la revendication 64, caractérisée en ce que la dicotylédone est la tomate, la pomme de terre, le tabac ou le coton.

**66.** Plante selon la revendication 64, caractérisée en ce qu'elle lutte contre le doryphore.

**67.** Plante selon la revendication 64, caractérisée en ce que l'inhibiteur de la protéinase est exprimé dans les racines, les tiges, les feuilles, les graines ou le pollen de la plante.

**68.** Vecteur, qui comprend une séquence d'ADN qui code pour un inhibiteur de la protéinase non végétal qui ne représente pas d'égline B ou C, caractérisé en ce que le vecteur peut être utilisé pour la transformation de cellules végétales ou d'*Agrobacterium* et pour l'expression de l'inhibiteur de la protéinase dans des plantes.

**69.** Vecteur selon la revendication 68, caractérisé en ce qu'il s'agit d'un vecteur dérivé d'un plasmide de Ti.

**70.** Vecteur selon la revendication 68, caractérisé en ce qu'il est dérivé des plasmides pCIB10 ou pCIB715.

*Fig. 1*

*Fig. 2*

Fig. 3

Fig. 4

## Fig. 5

*Fig. 6*

## Fig. 1

pLW 111
↓ Bgl III
↓ ISOLIERUNG EINES
↓ 1.15kBp FRAGMENTES

pUC 19
↓ Bam HI

VERKNÜPFEN

(BamHI/Bgl II)
FUSION

(BamHI/Bgl II)
FUSION

pUC 19/35S

IN VITRO
MUTAGENESE

BamHI
GGATCC
▽

CaMV 35S
PROMOTER

CaMV 35S
TERM

XbaI
SalI
PstI
SphI
HindIII

pCIB 710

SmaI
Kpn I
SstI
EcoRI

## _Fig. 8_

# Fig. 9

## CYSTATIN SEQUENZEN

|  |  | 10 | 30 | 50 | 70 | 90 |
|---|---|---|---|---|---|---|

**Aminosäure**

SerGluAspArgSerArgLeuLeuGlyAlaProValProValAspGluAsnAspGluGlyLeuGlnArgAlaLeuGlnPheAlaMetAlaGl
|————————————————————A————————————————————||————————————————B————————|

**mRNA**

5'GATCCATGAGCGAGGACCGCAGCCGCCTGCTGGGCGCCCCGGTGCCGGTGGACGAGAACGACGAGGGCCTGCAGCGCGCCCTGCAGTTCGCCATGGCCGA
--------+---------+---------+---------+---------+---------+---------+---------+---------+---------+

**1**

**compl. DNA**

3'CTAGGTACTCGCTCCTGGCGTCGGCGGACGACCCGCGGGGCCACGGCCACCTGCTCTTGCTGCTCCCGGACGTCGCGCGGGACGTCAAGCGGTACCGGCT
|————————————————————F————————————————||————————————————G————————|

|  |  | 110 | 130 | 150 | 170 | 190 |
|---|---|---|---|---|---|---|

**AA**

uTyrAsnArgAlaSerAsnAspLysTyrSerSerArgValValArgValIleSerAlaLysArgGlnLeuValSerGlyIleLysTyrIleLeuGlnVal
——————————————————————————||————————————————C————————————————

**mRNA**

GTACAACCGCGCCAGCAACGACAAGTACAGCAGCCGCGTGGTGCGCGTGATCAGCGCCAAGCGCCAGCTGGTGAGCGGCATCAAGTACATCCTGCAGGTG
--------+---------+---------+---------+---------+---------+---------+---------+---------+---------+

**101**

**cDNA**

CATGTTGGCGCGGTCGTTGCTGTTCATGTCGTCGGCGCACCACGCGCACTAGTCGCGGTTCGCGGTCGACCACTCGCCGTAGTTCATGTAGGACGTCCAC
————————————————|————————————H————————————||————————————I————————|

|  |  | 210 | 230 | 250 | 270 | 290 |
|---|---|---|---|---|---|---|

**AA**

GluIleGlyArgThrThrCysProLysSerSerGlyAspLeuGlnSerCysGluPheHisAspGluProGluMetAlaLysTyrThrThrCysThrPheV
————————||————————————D————————————————||—————————

**mRNA**

GAGATCGGCCGCACCACCTGCCCGAAGAGCAGCGGCGACCTGCAGAGCTGCGAGTTCCACGACGAGCCGGAGATGGCCAAGTACACCACCTGCACCTTCG
--------+---------+---------+---------+---------+---------+---------+---------+---------+---------+

**201**

**cDNA**

CTCTAGCCGGCGTGGTGGACGGGCTTCTCGTCGCCGCTGGACGTCTCGACGCTCAAGGTGCTGCTCGGCCTCTACCGGTTCATGTGGTGGACGTGGAAGC
————————————————||————————————J————————————||——————

|  |  | 310 | 330 | 350 |
|---|---|---|---|

**AA**

alValTyrSerIleProTrpLeuAsnGlnIleLysLeuLeuGluSerLysCysGlnEnd
————————————————E————————————————|

**mRNA**

TGGTGTACAGCATCCCGTGGCTGAACCAGATCAAGCTGCTGGAGAGCAAGTGCCAGTAGGATC
--------+---------+---------+---------+---------+---------+----   **363**

**301**

**cDNA**

ACCACATGTCGTAGGGCACCGACTTGGTCTAGTTCGACGACCTCTCGTTCACGGTCATCCTAG
————————————————K————————————————|

EP 0 348 348 B1

80

# Fig. 10

## CYSTATIN GEN-FRAGMENTE FÜR DIE SYNTHESE

### mRNA Strang

A  1- 70  GATCCATGAG CGAGGACCGC AGCCGCCTGC TGGGCGCCCC GGTGCCGGTG GACGAGAACG ACGAGGGCCT

B  71-140  GCAGCGCGCC CTGCAGTTCG CCATGGCCGA GTACAACCGC GCCAGCAACG ACAAGTACAG CAGCCGCGTG

C  141-210  GTGCGCGTGA TCAGCGCCAA GCGCCAGCTG GTGAGCGGCA TCAAGTACAT CCTGCAGGTGGAGATCGGCC

D  211-288  GCACCACCTG CCCGAAGAGC AGCGGCGACC TGCAGAGCTG CGAGTTCCAC GACGAGCCGG AGATGGCCAA GTACACCA

E  289-359  CC TGCACCTTCG TGGTGTACAG CATCCCGTGG CTGAACCAGA TCAAGCTGCT GGAGAGCAAG

TGCCAGTAG

### Komplementärer DNA Strang

F  5- 50  CACCGG CACCGGGGCG CCCAGCAGGC GGCTGCGGTC CTCGCTCATG

G  51-120  CGTTGC TGGCGCGGTT GTACTCGGCC ATGGCGAACT GCAGGGCGCG CTGCAGGCCC TCGTCGTTCT CGTC

H  121-170  CAGCTG GCGCTTGGCG CTGATCACGC GCACCACGCG GCTGCTGTAC TTGT

I  171-240  GGTCGC CGCTGCTCTT CGGGCAGGTG GTGCGGCCGA TCTCCACCTG CAGGATGTAC TTGATGCCGC TCAC

J  241-294  TGCAGGTGGT GTACTTGGCC ATCTCCGGCT CGTCGTGGAA CTCGCAGCTC TGCA

K  295-362  GATCCTACT GGCACTTGCT CTCCAGCAGC TTGATCTGGT TCAGCCACGG GATGCTGTAC ACCACGAAGG

EP 0 348 348 B1

## _Fig. 11:_ SYNTHETISCHE FRAGMENTE ZUR KONSTRUKTION DES KUNITZ TRYPSIN-INHIBITOR-GENS AUS SOJABOHNEN

## mRNA Strang

5M   GA TCCATGAAGA GCACCATCTT CTTTGCTCTC TTTCTCTTTT GTGCCTTC

3M   GCGTTT GGTGGTGTCT AAGAACAAAC CGTTAGTGGT TCAGTTTCAA AAACTTGATA

AGGAGTCACT CTAG

## cDNA Strang

3C   GATC CTAGAGTGAC TCCTTATCAA GTTTTTGAAA CTGAACCACT AACGGTTTCT

TCTTAGACAC CACCAA

5C   GTGAAG GCACAAAAGA GAAAGAGAGC AAAGAAGATG GTGCTCTTCA TGGATC

82